# EUROPEAN PATENT APPLICATION

(11) **EP 4 112 739 A1**
(43) Date of publication of application: **04.01.2023**
(21) Application number: 22170219.4
(22) Date of filing: 17.08.2017
(51) Int. Cl.: C12Q 1/68, C12Q 1/6804

(54) **METHOD OF DETECTION OF DNA END(S) AND ITS USE**

(62) Divisional of application: 17771598.4
(71) Applicant: INTODNA SPÓLKA AKCYJNA, 30-348 Kraków (PL)
(72) Inventor: KORDON-KISZALA, Magdalena, 31-518 Kraków (PL); ZAREBSKI, Miroslaw, 30-389 Kraków (PL); DOBRUCKI, Jerzy, 31-326 Kraków (PL); SOLARCZYK, Kamil, 43-200 Pszczyna (PL)
(74) Representative: Boult Wade Tennant LLP

(57) **Abstract**

The invention concerns the method of detection of DNA end(s) in a biological material, comprising the following steps I - III and at least one of sub-steps a-h of each of steps I - III: *I. PREPARATION OF THE* MATERIAL comprising a. fixation and/or permeabilization and/or lysis and/or isolation and/or fractionation and/or immobilization of the biological material, b. increasing accessibility of DNA end(s), c. blocking nonspecific binding site(s) for molecules type 2-6, in the biological material; *II. PROCESSING OF DNA END(S)* comprising d. *):* modification of DNA end(s) by chemical or physical processing followed by binding molecules type 1 to the DNA end(s) by catalytic or noncatalytic means; blocking nonspecific binding site(s) for molecules type 2-6 in the biological material; *III. RECOGNITION AND DETECTION OF THE MODIFIED DNA END(S):* incubation of the biological material from step II with at least two molecules type 2 and 3 which bind to the molecules type 1 in a manner that allows steps leading to rolling circle amplification (RCA) reactions, g. detection of DNA end(s) by: i. optionally contacting suitable molecules type 4 and/or 5 with molecules type 2 and 3, wherein the molecules type 4 and/or 5 are conjugated with the oligonucleotides type 1, ii. adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2, iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions, h. detection of molecules type 6; wherein when more than one sub-step a - c of step I is performed then they may occur in any order. The invention concerns also use of rolling circle replication for marking the presence and position of single DNA end(s) and use above-mentioned method for detection of DNA end(s) in a biological material.

## Description

### FIELD OF THE INVENTION

The presented invention falls within the molecular diagnostics and biomedical field. It is related to the method of detection and/or quantitative and/or qualitative analysis of DNA damage. More precisely, the present invention enables direct, highly sensitive, specific and efficient recognition and/or detection of different types of free DNA ends formed in result of induction of single- or double-strand DNA breaks in the cell nucleus, or in isolated biological materials.

### BACKGROUND

For a life to continue from generation to generation, genomes of organisms must be passed on without any significant changes. The storage of genetic information in a form of a unique sequence of nucleobases that form deoxyribonucleic acid (DNA), and the ability to transfer it through generations, are principal features of all living organisms. Cells are the basic structural and functional units of living organisms. The sequence of bases must, therefore, be preserved during the life of a single cell. Moreover, DNA of a parent cell should be copied faithfully, and the two copies that are passed on to the daughter cells ought to be identical. However, DNA is not infallibly stable, and it is susceptible to spontaneous chemical changes occurring under physiological conditions (Lindahl T. Instability and decay of the primary structure of DNA. Nature. 1993 Apr 22;362(6422):709-15). A DNA change, such as a DNA damage, may be induced by various endogenous and exogenous factors. The endogenous factors comprise damaging byproducts of cellular metabolism and respiration (such as reactive oxygen species). Exogenous factors include various damaging chemicals (for instance, methylating, oxidising, or crosslinking agents), UV and higher energy photons (X-rays, gamma rays), and heavy ions and particles (for instance, beta or alpha particles) (Friedberg Errol C., Graham C. Walker, Wolfram Siede, Richard D. Wood. DNA Repair and Mutagenesis. American Society for Microbiology Press, 2005).

Since DNA is inherently chemically unstable, and is constantly damaged by various factors, preserving the genomic integrity requires specialised surveillance and repair mechanisms. Such various protective mechanisms have evolved alongside DNA as the main and unique molecule encoding genetic information on Earth. Despite the fact that DNA is subjected to detrimental influence of its complex environment which induces a high rate of genomic degradation and changes in the base sequence, the numbers of mutations accumulated within a single cell division cycle are low (Araten DJ, Golde DW, Zhang RH, Thaler HT, Gargiulo L, Notaro R, Luzzatto L. A quantitative measurement of the human somatic mutation rate. Cancer Res. 2005 Sep 15;65(18):8111-7. Erratum in: Cancer Res. 2005 Nov 15;65(22):10635). The mutation rate is kept low by efficient action of specialised damage detection and repair mechanisms. Only genomic degradation which escapes the correct action of the repair mechanisms may lead to somatic mutations. An accumulation of unrepaired DNA damage compromises genome integrity and can lead to various cellular malfunctions, neoplastic transformation, or death. Non-lethal changes in the genome may be passed on to subsequent generations, accumulate, and eventually result in serious malfunctions of the progeny. Neoplastic transformation may eventually have serious consequences on the level of the whole organism, since a transformed cell may give rise to a clone of such cells and a growth of a malignant tumour.

In some cases, death of just a few cells, which results from DNA damage, may have serious consequences for the entire organism, for instance, a loss of irreplaceable neurons in the brain. The accumulation of DNA damage is also directly associated with various phenomena described in the literature as DNA replication stress, senescence of cells, and cell ageing (Técher H, Koundrioukoff S, Nicolas A, Debatisse M. The impact of replication stress on replication dynamics and DNA damage in vertebrate cells. Nat Rev Genet. 2017 Jul 17. doi: 10.1038/nrg.2017.46. [Epub ahead of print] Review; White RR, Vijg J. Do DNA Double-Strand Breaks Drive Aging? Mol Cell. 2016 Sep 1;63(5):729-38. doi: 10.1016/j.molcel.2016.08.004. Review). The integrity of sperm DNA is well known to have profound influence on a pregnancy outcome (Evenson DP, Darzynkiewicz Z, Melamed MR: Relation of mamalian sperm chromatin heterogeneity to fertility. Science. 1980, 240: 1131-1133; Evenson DP. The Sperm Chromatin Structure Assay (SCSA(®)) and other sperm DNA fragmentation tests for evaluation of sperm nuclear DNA integrity as related to fertility. Anim Reprod Sci. 2016 Jun;169:56-75. doi: 10.1016/j.anireprosci.2016.01.017. Epub 2016 Feb 17; Rex AS, Aagaard J, Fedder J. DNA fragmentation in spermatozoa: a historical review. Andrology. 2017 Jul;5(4):622-630. doi: 10.1111/andr.12381).

Many damaging factors induce DNA breaks directly, while others cause DNA lesions that may eventually lead to a discontinuity of a DNA molecule, that is, a single- and double-strand break. Single-strand breaks or nicks result from disruption of the phosphodiester bond between two adjacent nucleotides in one strand of DNA. Double-strand DNA breaks are a discontinuity of both complementary strands of DNA, and occur when the phosphodiester bonds linking nucleotides in opposing or closely located positions on the DNA strands are broken. Double-strand DNA breaks (DSBs) are the most dangerous DNA lesions, whereas, single-strand breaks (SSBs) bring about less deleterious effects, however, their number is much higher than the number of double-strand lesions. Double-strand breaks in normal cells often result from more than one single-strand break that occur close to each other in both complementary strands. When a double-strand break is induced, the DNA ends may become physically separated.

Several types of DNA brakes are known (Clark Robert S. B., Minzhi Chen, Patrick M. Kochanek, Simon C. Watkins, Kun Lin Jin, Romesh Draviam, Paula D. Nathaniel, Rodnina Pinto, Donald W. Marion, and Steven H. Graham. Journal of Neurotrauma. July 2004, 18(7): 675-689):
- Single-strand nicks (where 3'-OH DNA strand ends are present)
- Single-strand gaps (where 3'-OH DNA strand ends are present)
- Single-strand breaks of single phosphodiester bonds (where 3'-OH DNA strand ends are present)
- Double-strand blunt-end breaks (where 3'-OH DNA strand ends are present)
- Double-strand 3'-protruding breaks (where overhanging 3'-OH DNA strand ends are present)
- Double-strand 5'-protruding breaks (where 3'-OH DNA strand ends are present)
- DNA breaks with "damaged" ends of the DNA strands (where there are no 3'-OH DNA strand ends)

DNA strand breaks occur during many normal, endogenous cellular processes, such as DNA replication, transcription, relaxation of supercoils, gene rearrangement, DNA damage repair, etc. Such strand breaks are usually immediately ligated by specialized enzymes.

The occurrence of an unrepaired DSB generates unprotected reactive DNA ends which may initiate or/and participate in recombination with DNA regions of partial homology located elsewhere in the genome. This may produce various rearrangements in the genome, such as deletions, translocations, fusion and breakage of chromosomes, cyclisation of chromosomes, appearance of mini chromosomes, etc.

DNA strand breaks may be associated with various types of disorders, or may be a manifestation of an existing, underlying disorder that could result from mutations and malfunctions of the molecular repair machinery. An accumulation of DNA strand breaks may be associated with the development of a variety of diseases including cancers, such as colon, breast, skin or prostate, neurodegenerative disorders, including Alzheimer's, Huntington's and Parkinson's diseases, and diseases such as Fragile X syndromes, Friedrich's ataxia, spinocerebellar ataxias, diabetes mellitus type 2, Creutzfeldt-Jakob disease, and myotonic dystrophy, and a number of other disorders.

Knowledge about the presence, number and location of single- and double-strand DNA breaks in the cell nucleus, and the ability to distinguish a specific type of a lesion is required in order to assess the quality of DNA of cells and tissues of the organism, and to study and understand the mechanisms of DNA damage induction and its repair. Measurements of the number and determinations of the subnuclear localisation of DNA breaks are required in order to assess genotoxic potential of various endo- and exogenous factors. Moreover, the analyses of the quality of biological material reflected in the degree of DNA fragmentation can serve as a marker in analyses of the environmental conditions under which the organisms live. A determination of DNA breaks may serve as a parameter to assess the quality of reproductive cells of human and other organisms.

DNA breaks in cellular DNA can be detected by some existing methods known in the art, such as:
- Direct detection and *in situ* identification of DNA ends exposed when the break is induced (for example, polymerase I-mediated nick translation assay, Klenow fragment of polymerase I-mediated nick-end labelling assay, or terminal deoxynucleotidyl transferase (TdT)-mediated dUTP nick-end labelling assay, known as TUNEL assay)
- Detection of repair factors recruited to a DNA lesion (for example 53BP1 for DSB or XRCC1 for SSB)
- Detection of epigenetic chromatin modifications specific for DNA breaks (currently only yH2A.X - phosphorylation of histone H2A.X on Serine139)
- Detection of DNA ends in extracted material by biochemical methods (for example, Comet assay, or alkaline elution assay, or filter elution assay (Kohn Kurt W., Leonard C. Erickson, Regina A. G. Ewig, and Charles A. Friedman. Fractionation of DNA from mammalian cells by alkaline elution. Biochemistry, 1976, 15 (21), pp 4629-4637; DNA Damage Detection In Situ, Ex Vivo, and In Vivo (2011). Methods and Protocols. Springer. Editor: Vladimir V Didenko)
- Detection of an overall degree of DNA fragmentation measured by the degree of the susceptibility of DNA to acid-induced DNA denaturation *in situ* (for example SCSA test)

While indirect approaches (yH2A.X, XRCC1, 53BP1) allow for detection of a low number of DNA breaks, currently, there are no methods of detecting individual DNA breaks directly, in live or fixed cells.

Methods based on direct labelling and detection of DNA ends are all characterised by low sensitivity, i.e., they are unable to detect individual DNA breaks in intact cells or treated biological materials. TUNEL assay, well known to the persons skilled in the art, is able to readily detect large numbers (likely hundreds and more) of DNA breaks during apoptosis, however, it is not capable of detecting individual DNA double-strand breaks, or even groups consisting of low numbers (10 or so) of these breaks.

Most cells in a human body exposed to typical every-day endogenous and exogenous factors inducing strand breaks exhibit a low number of double-strand breaks (approximately 0 - 10 at any given time, as detected by immunolabeling of yH2A.X (Rybak P, Hoang A, Bujnowicz L, et al. Low level phosphorylation of histone H2AX on serine 139 (yH2AX) is not associated with DNA double-strand breaks. Oncotarget.2016;7(31):49574-49587)), and a somewhat larger number of single-strand breaks. Only in the cells producing antibodies, the DNA breaks do not constitute damage, but are a component of a natural process of gene rearrangements. The number of DNA breaks is very high in cells undergoing a process of programmed cell death (apoptosis). Double-strand breaks are repaired in the processes lasting usually many hours, while single-strand breaks are repaired rapidly, often within minutes. Thus, the number of DNA breaks of both types present at any given time in a human somatic cell usually does not exceed 30 (detected by immunolabeling of accumulations of yH2A.X and XRCC1 repair factors (Berniak K., Rybak P., Bernas T., Zar 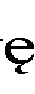 bski M., Biela E., Zhao H., Darzynkiewicz Z., Dobrucki J.W.: Relationship between DNA damage response, initiated by camptothecin or oxidative stress, and DNA replication, analyzed by quantitative 3D image analysis. Cytometry. A, 2013; 83: 913-24; Rybak P, Hoang A, Bujnowicz L, et al. Low level phosphorylation of histone H2AX on serine 139 (yH2AX) is not associated with DNA double-strand breaks. Oncotarget.2016;7(31):49574-49587; Solarczyk K.J., Kordon M., Berniak K., Dobrucki J.W.: Two stages of XRCC1 recruitment and two classes of XRCC1 foci formed in response to low level DNA damage induced by visible light, or stress triggered by heat shock. DNA Repair (Amst)., 2016; 37: 12-21). The typical low number of DNA breaks encountered in somatic cells under physiological conditions cannot be detected and quantified by TUNEL, or any other established direct detection approach. The existing methods are all based on traditional immunofluorescent labelling (including TUNEL), therefore, their specificity is limited and heavily dependent of the specificity of primary and secondary antibodies, while the sensitivity of these methods is limited by the presence of considerable non-specific background signals (the intensity of specific signals do not sufficiently exceed the intensity of nonspecific ones).

An indirect detection of DNA breaks by means of detecting the recruited cellular DNA damage response factors (like 53BP1 or XRCC1) has the following limitations: (i) the number of recruited protein molecules may be too low to be detected by the generally used methods like immunofluorescence, (ii) immunofluorescence is often not fully specific due to limited specificity of the antibody, (iii) sensitivity of detection is limited by the presence of a signal background which arises from nonspecific binding of the secondary antibody to various cell components, (iv) recruitment of repair factors may be activated in the absence of DNA breaks, or may be inhibited or absent under the conditions of saturation of repair capacity, or when the repair systems themselves are damaged or impaired, thus false positive or false negative results may occur. Detection of fluorescently tagged repair proteins (for instance EGFP-53BP1) marking DNA breaks can be performed in live cells, however, this is usually done in heavily manipulated, transfected cells, under the conditions of overexpression of the fusion protein, in in vitro cultures. Measurements of the number of DNA breaks by means of detecting accumulation of fluorescently tagged proteins at the site of damage in cells freshly derived from a body of a patient are not possible.

A detection of double-strand breaks based on immunodetection of phosphorylation of histone H2A.X (yH2A.X) is an exception among other methods, since it is highly sensitive due to natural biological signal amplification. Namely, one DSB induces phosphorylation of up to one million of histone residues in the vicinity of the break. As of today, the ability to exploit this amplification is compromised, however, by the aforementioned shortcomings of the method of immunofluorescence, including nonspecific binding of the primary and secondary antibody to various cellular targets, and a resulting nonspecific signal. Although phosphorylation of histone H2A.X on Serine139 has been commonly used as a gold standard for the detection of DSBs, it has recently become known that this method has limited specificity with respect to DSBs in DNA. It has been demonstrated by several research groups that histone phosphorylation of histone H2A.X can be induced by lesions other than double-strand breaks (Cleaver JE1. yH2Ax: biomarker of damage or functional participant in DNA repair "all that glitters is not gold! ".Photochem Photobiol. 2011 Nov-Dec;87(6):1230-9; Rybak P, Hoang A, Bujnowicz L, et al. Low level phosphorylation of histone H2AX on serine 139 (γH2AX) is not associated with DNA double-strand breaks. Oncotarget.2016;7(31):49574-49587). These and other published data testify to the fact that this method exhibits only limited specificity towards DSBs. Finally, when breaks are located in close vicinity, they cannot be detected as individual entities, since immunofluorescent signals representing the large numbers of phosphorylated H2A.X molecules appear in microscopy images as large spots (a few hundred nanometers up to one micrometer diameter).

There are also several biochemical methods used in analysis of DNA damage known in the state of the art. However, these known techniques have some limitations. For example, Comet assay is used to detect large numbers of DNA breaks in individual cells. It is useful for the estimation of the general level of DNA damage, and the distribution of damage within a population of cells, however, it does not have sufficient sensitivity to detect individual DNA breaks. It does not deliver any information regarding the type of DNA damage, and the localization of DNA breaks in relation to the nuclear structure, since it requires cell lysis.

There are several versions of a widely used alkaline elution assay of assessing DNA damage, however, it is known that this method can introduce DNA breaks in the process (Tice RR, Agurell E, Anderson D, Burlinson B, Hartmann A, Kobayashi H, Miyamae Y, Rojas E, Ryu JC, Sasaki YF. Single cell gel/comet assay: guidelines for in vitro and in vivo genetic toxicology testing. Environ Mol Mutagen. 2000;35(3):206-21).

Based on the existing knowledge of state of the art, it is reasonable to expect that sensitive and specific detection of individual DNA breaks will employ detection of molecules attached specifically to DNA ends, as it is done in TUNEL assay. As the number of such attached molecules may be low (only one or several), it is logical to expect that a very sensitive method of detection of such molecules must be employed. A number of very sensitive techniques capable of specific detection of various molecules, or complexes of molecules, or their interactions, are known in the art, including various types of mass spectrometry, mass cytometry (mass spectrometry combined with flow cytometry), energy dispersive X-ray microanalysis (EDXMA, EDXA), or fluorescence-based methods, including immunofluorescence, Forster Resonance Energy Transfer (FRET), Bimolecular Fluorescence Complementation Assay (BiFc), proximity ligation assay (PLA), single molecule fluorescence detection, spectroscopy and imaging. None of these approaches (including immunofluorescence, as explained above) is directly applicable to detection of individual molecules, including proteins or nucleotide analogs attached to DNA ends in chromatin in cells, or in isolated chromatin.

The abovementioned methods of detection of DNA breaks, and the ones that are potentially applicable to detection of DSBs or SSBs, are also broadly described in the patent and non-patent literature.

For example, the invention described in WO9708345 publication pertains to the field of DNA detection for basic research, medical diagnostic testing, and forensic testing. The method described in the evoked publication constitutes the basis of TUNEL assay that has been mentioned in previous paragraphs. According to this invention, the DNA strands are first incubated with a halogenated deoxynucleotide triphosphate, such as brominated deoxyuridine triphosphate (BrdUTP), and an enzyme which can catalyze the addition of the halogenated deoxynucleotide to the 3'-OH ends of the DNA strand, such as terminal deoxynucleotidyl transferase (TdT). The resulting modified DNA strands are then incubated with a labeled antibody, such as a fluoresceinated monoclonal antibody, which binds specifically to the halogenated deoxynucleotide. The label is then detected by flow cytometry, microscopy, or multiparameter laser scanning microscopy. The method has utility in detecting apotosis, DNA synthesis and/or repair, and as a general method for DNA end labeling, however is not capable of detecting individual or low numbers of DNA ends. According to what was described in previous paragraphs, this invention, when used for detection of DNA breaks in cells, is dedicated to detection of hundreds of double-strand breaks that occur during cell apoptosis. As demonstrated in scientific literature, its sensitivity is too low to allow for detection of individual DNA lesions. The principal reason of this low sensitvity is the fact that this assay is based on standard methods of immunofluorescent staining resulting in high level of nonspecific signal, as it has been explained earlier in the text.

There are some disclosures in patents and patent applications of use of the modified proximity ligation assay (PLA) for DNA damage detection. Briefly, the PLA technique utilizes one pair of oligonucleotide labeled antibodies, or other molecules binding in close proximity (10-40 nm apart) to different epitopes of two analytes that form a complex or to the same analyte. The assay is mostly recommended to be used, in general, for localized detection, visualization and quantification of protein-protein complexes, or sensitive detection of proteins. This technique, combining two well-known methods, relies on the principle of the so-called "proximity probing", wherein the analyte is detected by the binding of two probes, which, when brought into proximity by binding to the analyte (hence "proximity" probes), allow a signal to be generated (see, for example, US Patent Publication Nos. US8013134 B2, US7306904 B2, US patent Application Publication No US2008/0293051, or WO16106298A1). This approach employs rolling circle amplification (RCA) reactions (see, for example, European Patent Publication No. EP1997909, US Patent Publication Nos. US7,88,849 or US7,790,338) which multiply the signal derived from the two probes. Said combination of proximity probing and RCA is also known in the art. For example, international application WO 2012/160083 A1 discloses a method for detecting interactions between or with any two of at least three target substrates, or any two of at least three features of a target substrate, or a combination of interactions and features of target substrates, by a multiplexed proximity ligation assay, said method comprising: a) for each of the at least three target substrates or features, providing a proximity probe comprising a binding moiety with affinity for the feature or binding site on said substrate, and a proximity probe oligonucleotide coupled to the binding moiety; wherein each of the proximity probe oligonucleotide carries a unique tag sequence; b) mixing the proximity probes with a sample, under a condition to allow binding of each proximity probe to its respective binding site or feature on each of said substrates through the binding moiety, c) simultaneous with, or following step b), forming circularized DNA molecules where any two proximity probes bind sufficiently close to each other on the substrate, wherein each of the circularized DNA molecules comprise complementary sequences to the unique tag sequences from the two proximity probes oligonucleotides; d) amplifying the circularized DNA; and e) characterizing the amplified DNA. For more variant solutions concerning PLA technique, see also Patent Application Nos. US2011223585A, US2014194311A, US2004248103A, or US2003008313A.

However, there are no embodiments of using said solution, namely PLA technique, for direct detection of DNA ends that occur as a result of any types of DNA breaks. Moreover, the simple application of PLA technique in DNA damage detection would require probes which target specific proteins which exclusively occur at the sites of only one type of DNA breaks and, as mentioned above, since the same protein can have multiple functions, the possibility of the presence of proteins at sites other than the DNA breaks is highly likely.

Publications *"*A novel single-cell method provides direct evidence of persistent DNA damage in senescent cells and aged mammalian tissues", Aging Cell (2017) 16, pp 422-427 and Alessandro Galbiati, Christian Beauséjour and Fabrizio d'Adda di Fagagna discloses a method, named *'DNA damage in situ ligation followed by proximity ligation assay'* (DI-PLA) concerns detection and imaging of DSBs in cells. DI-PLA is based on the capture of free DNA ends in fixed cells in situ, by ligation to biotinylated double-stranded DNA oligonucleotides, which are next recognized by antibiotin antibodies. Detection is enhanced by using PLA technique for the combination of the abovementioned antibodies with the antibodies recognizing a partner protein involved in the processes of DNA damage response (yH2AX or 53BP1) at the site of the DSB. Validatation of said DI-PLA method was performed by demonstrating its ability to detect DSBs induced by various genotoxic insults in cultured cells and tissues. However, this method can be used only for double-strand DNA breaks which were marked by histone H2AX phosphorylation, or to which a repair factor 53BP1 was recruited, therefore, its use is limited. First of all, this is not a technique providing an opportunity of direct detection of DNA breaks, since it requires simultaneous detection of histone modifications or proteins involved in DNA damage repair. Secondly, it cannot be used in detection of DNA breaks that have not been recognized by the cellular protein repair machinery, or that are subjected to malfunctioning repair machinery.

In a publication of Hanif Rassoolzadeh, Christos Coucoravas & Marianne Farnebo, "The proximity ligation assay reveals that at DNA double-strand breaks WRAP53β associates with yH2AX and controls interactions between RNF8 and MDC1", Nucleus, 6:5, 417-424, 2015, the PLA assay was used to show that at the DNA double-strand breaks WRAP53b accumulates in close proximity to yH2A.X. More importantly, this document highlights PLA as a more sensitive method for the analysis of recruitment of repair factors and complex formation at DNA breaks that are difficult to detect using conventional immunofluorescence. Nevertheless, only double-strand breaks can be detected by this modified PLA method. Moreover, they only can be detected if two particular repair factors (RNF8 and MRC1) are present at the site of the DNA damage. Furthermore, this modification is based on interaction between proteins naturally ocurring in the cell, which may lead to the false positive results due to the possibility of the presence of those proteins and their complexes at different sites than the sites of DNA damage. Thus, the method presented in this publication is not specific enough, and its application is limited to only one particular type of DNA double-strand breaks, provided that they were recognized by the repair machinery.

The invention described in WO12080515A1 provides a method for determining the level of DNA damage in cells of a subject exposed to ionizing radiation comprising (a) contacting a sample comprising cells obtained from said subject with at least two antibodies, aptamers, or anticalins which bind to epitopes on the same or different protein(s) which is/are present in DNA repair foci; (b) contacting said sample with at least the first and second proximity probe, each comprising a binding moiety with affinity for said antibodies, aptamers, or anticalins, and a nucleic acid acting as a reactive functionality, coupled thereto; (c) allowing the binding moiety of said proximity probes to bind to said antibodies, aptamers, or anticalins, and allowing the nucleic acids of said proximity probes to interact with each other if the proximity probes are in close proximity to each other; and (d) detecting the degree of interaction between the nucleic acids, thereby determining the level of DNA damage. Nevertheless, this method was designed to monitor radiation exposure by detecting radiation-induced DNA double-strand breaks. No other types of breaks can be detected. Above all, however, this is another example of an indirect method of detection of DNA breaks. Similarly to what has been described above, it is based on detection of protein(s) involved in the processes of DNA damage repair rather than direct detection of DNA ends that result from DNA breaks.

The search of the existing methods demonstrates the need for a highly sensitive, specific and direct method of identifying and detecting any types of double- or single-strand DNA breaks in fixed or live cells, or tissues, as well as in extracted DNA, or isolated chromatin, or any type of biological material.

In the light of available approaches, there is a requirement for more sensitive, specific, selective and universal methods applicable in DNA damage *in situ* or *in vitro* analysis that would be convenient and simple at the same time. Sensitive and selective detection of DNA damage is very important in drug design, in diagnostics and/or monitoring the state of the patient, and the progress of disease as well. Moreover, it may also provide better insight into the processes related to carcinogenesis, degenerative and autoimmune diseases, or ageing - the major contemporary health problems

The present invention overcomes the disadvantages of the methods known in the art and constitutes a significant advance in the realm of the methods used in studies of DNA breaks and hence in diagnostics related to detection of DNA damage.

### SUMMARY OF THE INVENTION

Present invention relates to the method of detection of DNA end(s) in a biological material, comprising the following steps I - III and at least one of sub-steps a-h of each of steps I - III:

### I. PREPARATION OF THE MATERIAL

a. fixation and/or permeabilization and/or lysis and/or isolation and/or fractionation and/or immobilization of the biological material,
b. increasing accessibility of DNA end(s),
c. blocking nonspecific binding site(s) for molecules type 2-6, in the biological material,

### II. PROCESSING OF DNA END(S)

d. modification of DNA end(s) by chemical or physical processing followed by binding molecules type 1 to the DNA end(s) by catalytic or noncatalytic means,
e. blocking nonspecific binding site(s) for molecules type 2-6 in the biological material,

### III. RECOGNITION AND DETECTION OF THE MODIFIED DNA END(S)

f. incubation of the biological material from step II with at least two molecules type 2 and 3 which bind to the molecules type 1 in a manner that allows steps leading to rolling circle amplification (RCA) reactions,
g. detection of DNA end(s) by:
   i. optionally contacting suitable molecules type 4 and/or 5 with molecules type 2 and 3, wherein the molecules type 4 and/or 5 are conjugated with the oligonucleotides type 1,
   ii. adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2,
   iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
h. detection of molecules type 6,
wherein when more than one sub-step a - c of step I is performed then they may occur in any order.

According to the present invention, DNA end(s) are result(s), preferably of any DNA break(s) including single strand break or double strand break. Preferably, DNA break(s) are selected from the group comprising single-strand nick, single-strand gap, single-strand break of single phosphodiester bond, double-strand blunt-end break, double-strand 3'-protruding break, double-strand 5'-protruding break, types of DNA strand breaks including these where 3'-OH or 5'-phosphate DNA ends are not present.

Preferably detection of DNA end(s) is performed *in situ.*

Preferably the biological material is live.

Preferably the biological material is fixed.

More preferably biological material is selected from the group comprising animal, plant, protozoan, bacterial cells, viruses, tissues and fragments and/or components thereof. More preferably animal is human.

Preferably the biological material is cell or tissue or fragments thereof surrounded by a membrane. In this embodiment, preferable sub-step c is performed in step I.

Preferably before step I sub-step a), an additional step of increasing accessibility of DNA end(s) is performed.

Preferably the biological material is present in a solution or on a porous surface or solid support. More preferably solid supports or types of a porous surface are selected from the group comprising glass, plastic, water gels, aero gels metals and ceramics.

Preferably molecule type 1 is selected from the group comprising halogenated nucleotide or nucleoside molecules such as BrdU, IdU, CldU, or DNA precursor analogs such as EdU (5-Ethynyl-2'-deoxyuridine), F-ara-EdU, 5-Ethynyl-2'-deoxycytidine, or biotinylated nucleotide molecules, ADP-ribose molecules, or protein molecules, nucleotide, or nucleoside molecules labeled with labels selected from a group comprising fluorescent molecules, or chemiluminescent molecules, or radioisotopes, or enzyme substrates, or biotin molecules, molecule type 1 is also selected from the group comprising any other molecules capable of binding to DNA or RNA end(s) and serving as a target for binding of molecules type 2, or type 3, or any other substrates for enzymes, or any of their analogs, or oligomers, or polymers, or combinations thereof.

Preferably molecule type 2 and molecule type 3 independently are selected from the group comprising antibody or fragments thereof, streptavidin, avidin, biotin molecules or analogs thereof, ligands, proteins, peptides, nucleic acids, antigens, reactive molecules like azides, oligomers, polymers, and any analogs of the abovementioned or combinations thereof.

Preferably molecule 4 and molecule 5 independently are selected from the group comprising antibodies covalently linked to nucleic acids (oligonucleotides type 1) of a sequence which allows interaction (hybridisation of selected regions) with other oligonucleotides (oligonucleotides type 2), their ligation, and subsequent RCA reaction.

Preferably molecule 4 and molecule 5 independently are selected from the group comprising antibody streptavidin, avidin, biotin, streptavidin analog, biotin analog, ligands, proteins, peptides, nucleic acids, antibody fragments, antigens, reactive molecules like azides, oligomers, polymers, analogs of the abovementioned or combinations thereof.

Preferably molecule type 6 is a labeled oligonucleotide (oligonucleotides type 3) capable of recognizing and binding to products of RCA reaction formed using a selected sequence of ligated oligonucleotide type 2 as a template.

Preferably molecule type 7 is a molecule capable of recognizing and binding to products of RCA reaction formed using a selected sequence of ligated oligonucleotide type 2 as a template, and capable of serving as a binding target for other molecules that carry or can generate a molecular signature.

Preferably amplification of sub-step g) in step III is improved by addition of the molecules type 7 which bind to molecules type 6.

Preferably molecule type 7 is labeled with labels selected from a group comprising fluorescent chemiluminescent molecules, radioisotopes, enzyme substrates, biotin, nanoparticles.

Preferably catalytic mean comprises non-enzymatic or enzymatic mean. More preferable the enzymatic mean is selected from the group comprising DNA polimerase I, TdT, Klenow fragment, Phu polymerase, Taq polymerase, T4 DNA Polymerase, T7 DNA Polymerase, T4 Polynucleotide Kinase, RNA polymerases. More preferably non-enzymatic mean is selected from the group comprising chemical factors with catalytic properties. More preferably noncatalytic mean comprises physical or biochemical factors.

Preferably detection of sub-step h) is performed by techniques selected from the group comprising microscopy, methods of automated analysis of high cell numbers, spectroscopy, including fluorescence microscopy (wide field, confocal, multifocal, super-resolution, microscopy with catapulting, laser scanning, high throughput, high content), fluorimetry, transmitted light optical microscopy for absorption detection, flow cytometry, cell sorting (FACS), mass spectrometry.

Preferably molecules types 2 and 3 are not conjugated with oligonucleotides type 1. According to this embodiment, preferable contacting suitable molecules type 4 and type 5 with molecule type 2 and 3 is performed in step III sub-step g (i).

Preferably increasing accessibility of DNA end(s) does not induce DNA damage.

Preferably detection of single DNA end(s) is obtained.

Preferably marking the presence and position of single DNA end(s) is acquired.

Preferably, the detection of DNA end(s) in a biological material being cells or tissues, comprises the following steps:

### I. PREPARATION OF THE MA TERIAL

a. fixation and/or permeabilization and/or isolation and/or immobilization of the biological material,
b. increasing accessibility of DNA end(s),

### II. PROCESSING OF DNA END(S)

d. modification of DNA end(s) by chemical processing followed by binding molecules type 1 being nucleotides or analogs thereof, to the DNA end(s) by catalytic means,
e. blocking nonspecific binding site(s) for molecules type 2-6 in the biological material,

### III. RECOGNITION AND DETECTION OF THE MODIFIED DNA END(S)

f. incubation of the biological material from step II with at least two molecules type 2 and 3 being primary antibodies, which bind to the different types of binding sites of molecules type 1 in a manner that allows steps leading to rolling circle amplification (RCA) reactions,
g. detection of DNA end(s) by:
   ii. contacting suitable molecules type 4 and 5 being secondary antibodies, with molecules type 2 and 3, wherein the molecules type 4 and 5 are conjugated with the oligonucleotides type 1,
   iii. adding oligonucleotides type 2 and enzyme ligase to allow hybridization of said added oligonucleotides type 2 to the oligonucleotides type 1 already linked to molecules type 4 and 5 and subsequently performing DNA ligation of oligonucleotides type 2,
   iv. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
h. detection of molecules type 6.

Preferably the detection of DNA end(s) in a biological material being cells or tissues, comprises the following steps:

### I. PREPARATION OF THE MA TERIAL

a. fixation and/or permeabilization and/or isolation and/or immobilization of the biological material,
b. increasing accessibility of DNA end(s),
c. blocking nonspecific binding site(s) for molecules type 2-6 in the biological material,

### II. PROCESSING OF DNA END(S)

d. modification of DNA end(s) by chemical processing followed by binding molecules type 1 being unmodified nucleotides or biotinylated nucleotides, to the DNA end(s) by catalytic means,
e. blocking nonspecific binding site(s) for molecules type 2-6 in the biological material,

### III. RECOGNITION AND DETECTION OF THE MODIFIED DNA END(S)

f. incubation of the biological material from step II with at least two molecules type 2 and 3 being primary antibodies which bind to the different types of binding sites of molecules type 1 in a manner that allows steps leading to rolling circle amplification (RCA) reactions,
g. detection of DNA end(s) by:
   i. contacting suitable molecules type 4 and 5 being secondary antibodies, with molecules type 2 and 3, wherein molecules type 4 and 5 are conjugated with the oligonucleotides type 1,
   ii. adding oligonucleotides type 2 and enzyme ligase to allow hybridization of said added oligonucleotides type 2 to the oligonucleotides type 1 already linked to molecules type 4 and 5 and subsequently performing DNA ligation of oligonucleotides type 2
   iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
h. detection of molecules type 6.

Preferably the detection of DNA end(s) in a biological material being cells or tissues, comprising the following steps:

### I. PREPARATION OF THE MA TERIAL

a. fixation and/or permeabilization and/or isolation and/or immobilization of the biological material,
b. increasing accessibility of DNA end(s),
c. blocking nonspecific binding site(s) for molecules type 2-4 and 6 in the biological material,

### II. PROCESSING OF DNA END(S)

d. modification of DNA end(s) by chemical processing followed by binding molecules type 1 being unmodified nucleotides or biotinylated nucleotides, to the DNA end(s) by catalytic means,
e. blocking nonspecific binding site(s) for molecules type 2-4 and 6 in the biological material,

### III. RECOGNITION AND DETECTION OF THE MODIFIED DNA END(S)

f. incubation of the biological material from step II with at least two molecules: molecule type 2 being primary antibody and molecule type 3 being streptavidin conjugated with oligonucleotides type 1, which bind to the molecules type 1 in a manner that allows steps leading to rolling circle amplification (RCA) reactions,
g. detection of DNA end(s) by:
   i. optionally contacting suitable molecule type 4 being secondary antibody with molecule type 2, wherein the molecule type 4 is conjugated with the oligonucleotides type 1,
   ii. adding oligonucleotides type 2 and enzyme ligase to allow hybridization of said added oligonucleotides type 2 to the oligonucleotides type 1 already linked to molecules type 2 and 3, or to 3 and 4 if oligonucleotide type 1 is not conjugated with the molecule 2, and subsequently performing DNA ligation of oligonucleotides type 2,
   iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
h. detection of molecules type 6.

Present invention relates also to the use of rolling circle replication for marking the presence and position of single DNA end(s).

Present invention relates also to use of the following steps:
- contacting suitable molecules type 4 and/or 5 with molecules type 2 and 3, wherein the molecules type 4 and/or 5 are conjugated with the oligonucleotides type 1,
- adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2,
- performing amplification by adding enzyme polymerase and a solution of nucleotides to allow rolling circle amplification (RCA) reactions, and molecules type 6 to allow subsequent hybridization of molecules type 6 to thus obtained product of RCA reactions,
for detection of DNA end(s) in properly prepared biological material.

Preferably molecules types 2 and 3 are not conjugated with oligonucleotides type 1. In this embodiment, preferably contacting suitable molecules type 4 and type 5 with molecule type 2 and 3 is performed.

Preferably the biological material is prepared by the following steps:
- optionally, fixation and/or permeabilization and/or isolation and/or immobilization of the biological material,
- increasing accessibility of DNA end(s),
- optionally blocking nonspecific binding site(s) for molecules type 2-6 in the biological material.

Invention relates also to use of a method comprising the following steps I - III and at least one of sub-steps a-h of each of steps I - III:

### I. PREPARATION OF THE MA TERIAL

a. fixation and/or permeabilization and/or lysis and/or isolation and/or fractionation and/or immobilization of the biological material,
b.increasing accessibility of DNA end(s),
c. blocking nonspecific binding site(s) for molecules type 2-6, in the biological material,

### II. PROCESSING OF DNA END(S)

d. modification of DNA end(s) by chemical or physical processing followed by binding molecules type 1 to the DNA end(s) by catalytic or noncatalytic means,
e. blocking nonspecific binding site(s) for molecules type 2-6 in the biological material,

### III. RECOGNITION AND DETECTION OF THE MODIFIED DNA END(S)

f. incubation of the biological material from step II with at least two molecules type 2 and 3 which bind to the molecules type 1 in a manner that allows steps leading to rolling circle amplification (RCA) reactions,
g. detection of DNA end(s) by:
   i. optionally contacting suitable molecules type 4 and/or 5 with molecules type 2 and 3, wherein the molecules type 4 and/or 5 are conjugated with the oligonucleotides type 1,
   ii. adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2,
   iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
h. detection of molecules type 6,
wherein when more than one sub-step a - c from step I is performed then they may occur in any order,
for detection of DNA end(s) in a biological material.

Preferably DNA end(s) are result(s) of any DNA break(s) including single strand break or double strand break. More preferably DNA break(s) are selected from the group comprising single-strand nick, single-strand gap, single-strand break of single phosphodiester bond, double-strand blunt-end break, double-strand 3'-protruding break, double-strand 5'-protruding break, types of DNA strand breaks including these where 3'-OH or 5'-phosphate DNA ends are not present.

Preferably detection of DNA end(s) is performed *in situ.*

Preferably the biological material is live.

Preferably the biological material is fixed.

Preferably the biological material is selected from the group comprising animal, plant, protozoan, bacterial cells, viruses, tissues and fragments and/or components thereof. More preferably animal is human.

Preferably the biological material is cell or tissue or fragments thereof surrounded by membrane. In this embodiment, preferably sub-step c is performed in step I.

Preferably before step I sub-step a), an additional step of increasing accessibility of DNA end(s) is performed.

Preferably the biological material is present in a solution or on a porous surface or solid support. More preferably the solid supports or types of porous surface are selected from the group comprising glass, plastic, water gels, aero gels metals and ceramics.

Preferably molecule type 1 is selected from the group comprising halogenated nucleotide or nucleoside molecules such as BrdU, IdU, CldU, or DNA precursor analogs such as EdU (5-Ethynyl-2'-deoxyuridine), F-ara-EdU, 5-Ethynyl-2'-deoxycytidine, or biotinylated nucleotide molecules, ADP-ribose molecules, or protein molecules, nucleotide, or nucleoside molecules labeled with labels selected from a group comprising fluorescent molecules, or chemiluminescent molecules, or radioisotopes, or enzyme substrates, or biotin molecules, molecule type 1 is also selected from the group comprising any other molecules capable of binding to DNA or RNA end(s) and serving as a target for binding of molecules type 2, or type 3, or any other substrates for enzymes, or any of their analogs, or oligomers, or polymers, or combinations thereof.

Preferably molecule type 2 and molecule type 3 independently are selected from the group comprising antibody or fragments thereof, streptavidin, avidin, biotin molecules or analogs thereof, ligands, proteins, peptides, nucleic acids, antigens, reactive molecules like azides, oligomers, polymers, and any analogs of the abovementioned or combinations thereof.

Preferably molecule 4 and molecule 5 independently are selected from the group comprising antibodies covalently linked to nucleic acids (oligonucleotides type 1) of a sequence which allows interaction (hybridisation of selected regions) with other oligonucleotides (oligonucleotides type 2), their ligation, and subsequent RCA reaction.

Preferably molecule 4 and molecule 5 independently are selected from the group comprising antibody streptavidin, avidin, biotin, streptavidin analog, biotin analog, ligands, proteins, peptides, nucleic acids, antibody fragments, antigens, reactive molecules like azides, oligomers, polymers, analogs of the abovementioned or combinations thereof.

Preferably molecule type 6 is a labeled oligonucleotide (oligonucleotides type 3) capable of recognizing and binding to products of RCA reaction formed using a selected sequence of ligated oligonucleotide type 2 as a template.

Preferably molecule type 7 is a molecule capable of recognizing and binding to products of RCA reaction formed using a selected sequence of ligated oligonucleotide type 2 as a template, and capable of serving as a binding target for other molecules that carry or can generate a molecular signature.

Preferably amplification of sub-step g) in step III is improved by addition of the molecules type 7 which bind to molecules type 6.

Preferably molecule type 7 is labeled with labels selected from a group comprising fluorescent chemiluminescent molecules, radioisotopes, enzyme substrates, biotin, nanoparticles.

Preferably catalytic mean comprises non-enzymatic or enzymatic mean. More preferably the enzymatic mean is selected from the group comprising DNA polimerase I, TdT, Klenow fragment, Phu polymerase, Taq polymerase, T4 DNA Polymerase, T7 DNA Polymerase, T4 Polynucleotide Kinase, RNA polymerases.

More preferably the non-enzymatic mean is selected from the group comprising chemical factors with catalytic properties. More preferably noncatalytic mean comprises physical or biochemical factors.

Preferably the detection of sub-step h) is performed by techniques selected from the group comprising microscopy, methods of automated analysis of high cell numbers, spectroscopy, including fluorescence microscopy (wide field, confocal, multifocal, super-resolution, microscopy with catapulting, laser scanning, high throughput, high content), fluorimetry, transmitted light optical microscopy for absorption detection, flow cytometry, cell sorting (FACS), mass spectrometry.

Preferably molecules types 2 and 3 are not conjugated with oligonucleotides type 1. In this embodiment, preferable contacting suitable molecules type 4 and type 5 with molecule type 2 and 3 is performed in step III sub-step g (i).

Preferably increasing accessibility of DNA end(s) does not induce DNA damage.

Preferably detection of single DNA end(s) is obtained.

Preferably marking the presence and position of single DNA end(s) is acquired.

### REPRESENTATIVE EMBODIMENTS

Since the method according to the invention is described by Claims supported by present description, the variety of embodiments on the basis of Claims may be emphasized.

In one embodiment of the present invention, all sub-steps of the method are used in the order a to h, one step followed by another (a followed by b, b followed by **c**, and so on).

In other embodiments, only one from the sub-steps **a-c** is used, followed by sub-steps d, e, f, g and h in disclosed order. Nevertheless, in another preferred embodiment, at least two from the sub-steps **a-c** are used in the method.

According to the invention, certain embodiments of the method include sub-step d or d and e, taking into consideration variation concerning foregoing sub-steps **a-c**.

In the same manner, the variety of embodiments may be specified in relation to the sub-steps **f-h**.

In preferred embodiment, all sub-steps f, g and h are used in the method. All secondary sub-steps **i-iii** may be used for sub-step g in certain embodiments; in other embodiments, however, secondary sub-step i may be omitted.

The preparation of the material in step I for processing the biological material in further sub-steps may be performed, for example, before increasing accessibility of DNA end(s) and/or blocking nonspecific binding site(s) for molecules type 2-6, according to one or more processes selected from the group fixation, permeabilization, lysis, isolation, fractionation, immobilization.

In different embodiments, a person skilled in the art would predict and employ suitable methods from the above, for example based on the type of biological material used.

For example, in one embodiment, if biological material is a cell or tissue, fixation and/or permeabilization and/or isolation and/or immobilization of the biological material is/are performed.

Increasing accessibility of DNA end(s) in step I sub-step **b** may be carried out in live cells or tissues by incubation of said biological material with Tris, or any other agent causing hypoosmotic stress.

Increasing accessibility of DNA end(s) in step I sub-step **b** may be carried out by incubation of the biological material with EDTA, or any other agent causing chromatin loosening and/or expansion of a cell nucleus.

The variations of molecules type 1-7 and nucleotides 1-3 may be used in the method of detection of DNA end(s) according to the invention. Persons skilled in the art would know which combination and order of the sub-steps **a-h** and secondary sub-steps **i-iii** should be used to which application.

For the purpose of the present invention, molecules type 2 and 3 are always two different types of molecules that differ in the aspect of affinity and/or recognize and bind to different binding sites in molecules type 1.

In one specific embodiment, sub-step c in step I was omitted and the processing of DNA end(s) was performed using BrdU as a molecule type 1 and a catalytic mean is enzyme TdT. In this case, the molecules type 2 and 3 are antibodies, specifically, mouse monoclonal anti-BrdU antibody and rabbit polyclonal anti-BrdU antibody, respectively.

In another specific embodiment, the processing of DNA end(s) was performed using as a molecule(s) type 1 biotin-conjugated nucleotide(s), and a catalytic mean is enzyme DNA polymerase I. In this case, the molecules type 2 and 3 are antibodies, specifically, mouse monoclonal anti-biotin antibody and rabbit polyclonal anti-biotin antibody, respectively.

In another specific embodiment, the processing of DNA end(s) was performed using as a molecule(s) type 1 biotin-conjugated nucleotide(s), and a catalytic mean is enzyme DNA polymerase I, but in this case the molecules type 2 is an antibody, for example, a mouse monoclonal anti-biotin antibody and the molecule type 3 is a streptavidin molecule, both are conjugated with oligonucleotides type 1. The secondary sub-step i should be omitted.

In yet another embodiment, the processing of DNA end(s) was performed using as a molecule type 1 also biotin-conjugated nucleotide, but a catalytic mean is enzyme Klenow fragment.

In yet another embodiment, the processing of DNA end(s) was performed using first an exonuclease activity of DNA polymerase I (without the presence of nucleotides) to trim and modify the types of DNA ends in the sample and secondly, using biotin-conjugated nucleotides combined with unmodified nucleotides as a molecule(s) type 1 and enzyme Klenow fragment as a catalytic mean.

In yet another embodiment, the processing of DNA end(s) was performed using as a molecule type 1 ADP-ribose monomer and a catalytic mean is enzyme poly(ADP-ribose) polymerase, and in the step II, the processing of DNA end(s) is anticipated with the degradation of endogenous poly(ADP)-ribose polymers using poly(ADP-ribose) glycohydrolase and digestion of proteins using, for example, proteinase K.

In yet another embodiment, after sub-steps **a-d**, isolation of biological material is performed. In another embodiment, the molecules type 2 and 3 are primary antibodies from different hosts that are characterized by different levels of affinity and bind to different antigens present in molecules type 1. In this embodiment the suitable molecules type 4 and 5 are secondary antibodies that specifically target primary antibodies, which are molecules type 2 and 3, which are bound to antigens present in molecules type 1. More specifically for this embodiment the molecules type 2 and 3 are mouse and rabbit primary antibodies, and the molecules type 4 and 5 are anti-mouse and anti-rabbit secondary antibodies, respectively.

In yet another embodiment, the molecule type 2 is a primary antibody that binds to an antigen or antigens present in molecules type 1, where molecules type 1 comprise biotinylated nucleotides, and the molecule type 3 is a streptavidin molecule conjugated with an oligonucleotide type 1. Then in the step III sub-step g, secondary sub-step **i** comprises contacting the molecule type 2 with a suitable secondary antibody targeting the primary antibody from a certain host, which means that the molecule type 4 is conjugated with an oligonucleotide type 1.

In yet another embodiment, molecule type 2 is a primary antibody that binds to an antigen or antigens present in molecules type 1, where molecules type 1 comprise biotinylated nucleotides, and is conjugated with an oligonucleotide type 1, and molecule type 3 is a streptavidin molecule conjugated with an oligonucleotide type 1. In this embodiment, the secondary sub-step **i** in the step **g** is omitted.

In another embodiment the molecules type 2 and 3 are primary antibodies from different hosts that are characterized by different levels of affinity and that bind to different epitopes present in molecules type 1 and are conjugated with oligonucleotides type 1. In this embodiment the secondary sub-step **i** in the sub-step **g** is omitted.

In yet another embodiment the molecule type 2 is a primary antibody that binds to an antigen or antigens present in molecules type 1, where molecules type 1 comprise biotinylated nucleotides, and molecule type 3 is a streptavidin molecule. In this embodiment, in the sub-step **g** in secondary step **i** the molecule type 2 is contacted with a suitable secondary antibody targeting the primary antibody from a certain host, which means that the molecule type 4 is conjugated with an oligonucleotide type 1, whereas the molecule type 3 is contacted with a suitable primary antibody that recognizes and binds to antigens unique for streptavidin and constitutes the molecule type 5 conjugated with an oligonucleotide type 1.

In other embodiments if the molecules type 1 comprise any biotinylated molecules the step c cannot be omitted.

In another specific embodiment molecules type 1 are the proteins being the enzymatic means (for example DNA polymerase I, Klenow fragment, TdT or PARP1) involved in DNA end(s) processing that are bound and crosslinked at a site of a free DNA end(s). The molecules 2 and 3 are primary antibodies from different hosts that are characterized by different levels of affinity and that recognize and bind to different antigens present in molecules type 1, in other words that target different epitopes present on the same molecule of an enzyme. In this specific embodiment, the processing of DNA end(s) is followed by crosslinking reaction followed by permeabilization reaction.

In another embodiment, the method may be applied to cell nuclei isolated from cells in culture or tissues, where the nuclei may be extracted using one of the commercially available kits of reagents, or any other methods of isolation, such as a sucrose method. After the isolation, the nuclei have to be immobilized, for example, they may adhere to poly-D-lysine surface in 1x HBSS (137 mM Na⁺, 1 mM Mg²⁺). The sub-step **b** has to be omitted in this embodiment, although Tris buffer that may be used for the increase of accessibility of DNA ends in chromatin is one of the components used for isolation of the material.

In another embodiment, the nuclei may be isolated from cells and adhered after the sub-step **d** but before the sub-step **e.** The sub-step **b** has to be omitted in this embodiment and the cells should not be subjected to cross-linking prior to isolation.

In another embodiment, if the method is applied to isolated DNA or extracted chromatin from biological material comprising cells in culture or tissues, meaning the sub-step **a** comprises lysis and/or isolation, and/or fractionation, and/or immobilisation of the biological material, the material is immobilised, for example, on glass or it can be immobilised on glass covered with biotin, blocked with streptavidin if the biotinylated nucleotides are incorporated using nick translation assay prior to the immobilisation and further steps of modification of DNA ends. Alternatively, when modifying DNA ends, a mix of enzymes and modified and unmodified nucleotides can be used, for example, TdT with Polymerase I with mixture of an unmodified nucleotide, biotinylated nucleotides and BrdU. These embodiments exclude the possibility of further detection of biotinylated nucleotides; if the modification of DNA ends is performed in a solution, prior to the immobilisation of the material, DNA is precipitated using isopropanol and washed with 70% ethanol; sub-step **b** is omitted.

In other embodiments, DNA or chromatin are isolated from adherent cells or cells in suspension, or a tissue and immobilized after the sub-step **d** but before the sub-step **e** (the cells should not be subjected to any cross-linking reactions prior to isolation).

In yet another embodiment, DNA or chromatin are isolated from adherent cells, or cells in suspension, or a tissue and immobilized after the sub-steps **a-g.**

The above-mentioned embodiments reflect also to use of rolling circle replication for marking the presence and position of single DNA end(s) and use the method for detection of DNA end(s) in a biological material.

### DEFINITIONS

When describing the methods, techniques, and agents used in the methods e.g. compounds, compositions, solutions, buffers, of the invention, the following terms have the following meanings unless otherwise indicated. Additionally, as used herein, the singular forms "a," "an," and "the" include the corresponding plural forms unless the context of use clearly indicates otherwise. The terms "comprising", "including," and "having" are intended to be inclusive and mean that there may be additional elements other than the listed elements. All numbers expressing quantities of ingredients, properties such as treating conditions, and so forth used herein are to be understood as being modified in all instances by the terms "about" or "approximately", which for the purposes of this invention are equivalents and can be used interchangeably, unless otherwise indicated. At least, and not as an attempt to limit the application of the doctrine of equivalents to the scope of the claims, each number should be construed in light of the reported significant digits and by applying ordinary rounding techniques.

Since the present invention concerns the method of detection of DNA end(s), herein the "DNA end" is defined as a site in a DNA molecule where the nucleotide is connected with only one other nucleotide, or where deoxyribose is connected with only one phosphate group, or where a phosphate group is connected to one instead of two deoxyribose molecules. In general, such ends may be generated by (bio)chemical (e.g. endonuclease, topoisomerase) or physical (e.g. ionizing radiation) factors. DNA ends (other than in telomeres or transients created by topoisomerase) interfere with functions of DNA thus causing adverse effects for cell functions.

The term "DNA damage" is understood as any change in the base composition, base structure or primary or secondary structure of DNA and includes, but is not limited to, base loss, base oxidation, breakage of phosphodiester bond, which makes the DNA molecule different from the original one found in a normal, healthy cell of a living organism prior to the action of a damaging agent. Typical damaging agents are, for instance, ionizing radiation, UV, oxidising agents, methylating agents, and others well known in the art.

"DNA break" is defined in the present description as any breakage of a covalent bond, for instance, breakage of a phosphodiester bond or breakage of a deoxyribose ring, resulting in discontinuity of the DNA polymer. DNA break typically leads to DNA damage and for purposes of the present invention are understood as leading to formation of DNA ends. Representative types of DNA break observed in the DNA molecule include, but are not limited to single-strand nick, single-strand gap, single-strand break of single phosphodiester bond, double-strand blunt-end break, double-strand 3'-protruding break, double-strand 5'-protruding break, etc., and other types DNA strand breaks, including these where 3'-OH or 5'-phosphate DNA ends are not present.

"DNA strand(s)", "DNA molecule", "DNA polymer", DNA, are used herein as equivalents and are used interchangeably.

Since the method according to the invention concerns detection of DNA end(s) in a biological material, "biological material", for the purposes of the present invention, includes, but is not limited to, live or fixed material selected from the group comprising, e.g., animal including human, plant, protozoan, or bacterial cells, viruses, tissues and fragments thereof, and components of said materials. All or some of said materials may be excised or grown under *in vitro* conditions, obtained by extraction, fractionation, chromatography or other method of isolation.

"Preparation of the biological material" according to the invention includes treatment methods selected from the group comprising fixation, permeabilization, lysis, isolation, fractionation of the biological material, increasing accessibility of DNA end(s), blocking nonspecific binding sites.

The term "fixation of biological" material means chemical and/or physical procedures resulting in changes of the biological material intended to cause preservation of its structure and/or chemical components. Examples include, but are not limited to, crosslinking of proteins and DNA by formaldehyde or glutaraldehyde, preservation of biological material by exposure to ethyl alcohol, acetone or other chemicals (thus called fixatives).

The term "permeabilization of biological material" means chemical and/or physical procedures resulting in compromising the integrity of biological membranes (plasma and intracellular) and/or cell walls in order to allow access of molecules that have a limited capacity to enter cells or tissues.

The term "lysis" means destruction of the integrity of biological material in order to extract soluble components for further analysis. Lysis may be performed by chemical or physical means, including but not limited to homogenization, osmotic shock, or lysing buffers containing detergents and other chemicals.

Isolation step may be needed according to the invention to achieve a properly prepared biological material. The term "isolation", for the purposes of the present invention, means any processes performed on the biological material which leads to separation of specific component from the material. Specific components include, but are not limited to, cells from tissues, organelles from cells, fragments of organelles, chromatin from the nuclei.

The term "fractionation of biological material" means separating one or more desired components from this biological material. Fractionation may be applied to a crude (unprocessed) biological material as well as processed, e.g. as in a material acquired in a process of lysis.

"Increasing accessibility of DNA ends" is a process of removing steric hindrance and providing sufficient space to enable access to DNA ends of molecules (for instance proteins) that otherwise have limited capacity to come in contact with these ends.

The term "blocking", in relation to the nonspecific binding site, in the biological material means a procedure leading to prevention of binding of critical molecules used in the assay, for instance, antibodies, to molecular targets other than the ones for which the selected molecules exhibit affinity useful in the assay. A typical example of blocking is a procedure used in antibody detection (immunodetection) of a molecule of interest in biological material. The antibody is directed against a well-defined antigen but can also bind to various cell components via weak chemical interactions. This nonspecific binding is minimized by prior addition of blocking agents (such as albumin) that occupy such binding sites.

For the purposes of the present invention to better understand the present solution, terms "molecule type 1-7" were incorporated.

"Molecule type 1" is defined as any molecules capable of binding to DNA ends and constituting but not limited to (a) halogenated nucleotide or nucleoside molecules such as BrdU, IdU, CldU, or DNA precursor analogs such as EdU (5-Ethynyl-2'-deoxyuridine), F-ara-EdU, 5-Ethynyl-2'-deoxycytidine, or biotinylated nucleotide molecules, (b) nucleotide, or nucleoside molecules labeled with labels selected from a group comprising fluorescent molecules, or chemiluminescent molecules, or radioisotopes, or enzyme substrates, or biotin molecules, (c) ADP-ribose molecules, or protein, or any other molecules capable of binding to DNA or RNA end(s) and serving as a target for binding of molecules type 2, or type 3, or any other substrates for enzymes, or any of their analogs, or oligomers, or polymers, or combinations thereof.

"Molecule type 2" and "molecule type 3" are defined as molecules including, but not limited to, antibody or fragments thereof, streptavidin, avidin, biotin molecules, or analogs thereof, or ligands, proteins, peptides, nucleic acids, antigens, reactive molecules like azides, oligomers, polymers, and any analogs of the abovementioned, or combinations thereof that have a capacity to interact and/or bind to molecules type 1.

"Molecule type 4" and "molecule type 5" are defined as molecules including, but not limited to, antibodies covalently linked to nucleic acids (oligonucleotides type 1) of a sequence which allows interaction (hybridisation of selected regions) with other oligonucleotides (oligonucleotides type 2), their ligation, and subsequent RCA reaction. Molecules type 4 and type 5 have a capacity to interact and/or bind to molecules type 2 and type 3.

The term "ligation" means forming a covalent chemical bond between two molecules by means of a catalysed reaction. According to the present invention, ligation refers to joining the ends of oligonucleotides type 2, following a process of hybridisation of these oligonucleotides to oligonucleotides type 1, i.e. the oligonucleotides constituting components of molecules type 4 and 5 (oligonucleotides linked to antibodies), in order to generate a circular oligonucleotide which will serve as a template in RCA reactions.

"Hybridisation" (nucleic acid hybridisation, oligonucleotide-oligonucleotide hybridisation), as used in the description of this invention, refers to a process of forming hydrogen bonds (annealing) between two complementary stretches of nucleic acids (two oligonucleotides).

"Molecule type 6" is defined as molecule including, but not limited to, oligonucleotides (oligonucleotides type 3) capable of recognizing and binding to (hybridising to) products of RCA reaction formed using a selected sequence of ligated oligonucleotide type 2 as a template. Molecules type 6 carry molecular signature, for example, fluorescence emission, which is detected by appropriate instrument, for instance, fluorescence microscope, flow cytometer, laser scanning cytometer or spectrofluorimeter.

"Oligonucleotides" in the term "molecules type 6 (oligonucleotides type 3)" used in the amplification step (step 3, sub-step g, iii) after RCA reactions are intended to hybridise to a product of RCA reactions and may be selected by the persons skilled in the art on the basis of common knowledge (e.g. as in FISH - fluorescence in situ hybridisation).

"Molecule type 7" is defined as a molecule capable of recognizing and binding to products of RCA reactions formed using a selected sequence of ligated oligonucleotides type 2 as a template, and capable of serving as a binding target for other molecules that carry or can generate a molecular signature, for instance, complementary oligonucleotides linked with labels detectable by mass spectrometry, or oligonucleotides that are subsequently ligated and serve as substrates for RCA reactions leading to a detectable product.

The term "binding" used e.g. in the definitions of molecules type 1-7, means bringing various molecules into permanent or transient contact by inducing formation of a covalent bond or bonds, or by inducing weak chemical interactions, or bonds (including hydrogen, ionic, hydrophobic, van der Waals) where bringing into contact, linking or attaching said molecules is achieved by enzymatic or non-enzymatic means. An example of binding process according to present invention is contacting molecule type 1 with DNA ends or molecules type 2 and 3 with molecules type 1 in order to form a new linkage between these molecules.

The term "catalytic means", for the purposes of present invention, means methods or agents causing chemical changes in the system that do not occur spontaneously, but they are made possible by means of employing the action of a specific enzyme (protein catalyst), RNA or other factors, for example a surface (for instance platinum surface) or a low molecular weight chemical.

"RCA reactions" or "rolling circle amplification reactions" are the reactions known by the person skilled in the art, which employ rolling circle replication (RCR). RCR is rapid replication of nucleic acids that can synthesize multiple copies of circular molecules of DNA or RNA. It is derived from a natural biological system which is used by some bacteriophages and viruses. 'RCA reactions' used in this invention enable generation of detectable signals conveying information about DNA ends.

In order to understand the detection step of the method according to the invention, the term "signal" used herein means a detectable signature of the molecule or molecules, for instance, characteristic fluorescence emission, or other characteristic absorption, or emission of the energy of electromagnetic waves, or magnetic resonant properties or other spectroscopic, chemical or physical characteristic, in the RCA reaction definition as well as other definitions provided in this specification.

"Amplification of the signal" is a procedure or procedures resulting in modifying the molecule of interest or its molecular environment in a way which results in a stronger, more readily detectable signal conveying information about the presence and/or properties of this molecule of interest. According to the present invention, the amplification is made possible by RCA reactions which generate a large number of molecules that are subsequently recognized by multiple labelled molecules resulting in "enhanced" signal, e.g. fluorescence signal, which may be detectable by typical means.

"Detection", as it is obvious for the person skilled in the art, is a capacity to acquire information about the presence of an object or property in an environment where this object or property is concealed, or not readily noticeable. According to the present invention, the information may be encoded in a fluorescence or other signal derived from the sample and recorded by a dedicated instrument. Detection techniques which may be used in the present invention include but are not limited to various types of microscopy, methods of automated analysis of high cell numbers, spectroscopy, including fluorescence microscopy (wide field, confocal, multifocal, super-resolution, microscopy with catapulting, laser scanning, high throughput, high content), fluorimetry, transmitted light optical microscopy for absorption detection (histology), flow cytometry, cell sorting (FACS), mass spectrometry.

Regarding the present invention it is extremely important to understand the features of the method, the detection sensitivity is further defined as an ability to detect a low or high number of objects of interest, or a low or high level of a property of interest; high sensitivity refers to an ability to detect a low number or level of the objects or property of interest. For the purposes of the present invention, the high sensitivity in relation to an ability of detection means, that even single (one) DNA end is detectable by proper technique. What is obvious, any other higher number of single ends (many ends) is detectable by the method according to the invention. Above facts are illustrated in the examples shown in the description below.

In claims according to the present invention also other terms were introduced to describe the scope of protection of the invention.

The term "modified nucleotide" means a nucleotide which differs from the nucleotides that are building blocks of DNA or RNA by possessing of the functional group, or groups or other chemical modifications that normally do not exist in DNA or RNA of living organisms. Nonlimiting examples include bromodeoxyuridine, ethynyldeoxyuridine or biotinylated nucleotides.

The term "oligonucleotides type 1" is defined, but not limited to, oligonucleotides linked to antibodies to form molecules type 4 and molecules type 5, and capable of hybridising with oligonucleotides type 2.

The term "oligonucleotides type 2" means oligonucleotides which may (i) hybridise with oligonucleotides type 1 that are components of molecules type 4 and molecules type 5, (ii) undergo ligation, and thus (iii) form a substrate for subsequent RCA reaction.

The term "oligonucleotides type 3" means oligonucleotides labeled with a fluorophore or fluorophores, or other labels detectable by methods know in the art, capable of hybridising with resulting oligonucleotides formed by means of RCA reaction.

"A solution of nucleotides" referred to in the amplification step (step 3, sub-step g, iii) is a solution of the four nucleotides that are typical components of DNA of living organisms.

For better understanding the method of present invention, the following system in claim 1 was used. The method according to present invention comprising three main steps I-III and each step comprising further sub-steps a-g. Further, the sub-step g comprising three secondary sub-steps i-iii. The steps are obliged to be carried out in the shown order, namely step II has to follow step I and step III has to follow step II and each of those main steps have to comprise at least one sub-step. Unless indicated otherwise, other sub-steps may or may not be used, as it is indicated in claims, and a number of combinations of those may be used. The term "optionally" mans that the sub-step (e.g. secondary sub-step) may or may not be used in the method. The names of main steps I-III have no defined meaning and were introduced in order to structure the step sequence making the method comprehensible and clear.

The above-described molecules type 1-7, oligonucleotides 1-3 or other types of molecules, or solutions thereof, according to the invention, may be obtained from commercial sources and/or prepared using any convenient method. The selection of the abovementioned molecules, on the basis of common knowledge and according to the definitions of the present description, should be considered natural and obvious for person skilled in the art, unless otherwise stated.

Since the method according to the invention does not include the use of unmodified methods known in molecular diagnostic field, like standard TUNEL, or PLA method that is conventionally recommended to be used for detection of proteins, but uses only parts, elements or aspects of an approach thereof some of generally defined molecules 1-7, oligonucleotides 1-3, or other types of molecules or solutions thereof as well as media like buffers may be taken individually or in group(s) to the realisation of the invention from the commercial available kits. Therefore, it is obvious that it is not possible to provide a catalog of specific compounds designated in the present invention as molecules 1-7 and provide its specification in the examples, since the suppliers do not provide their characteristics.

For example, the molecule type 6 has to have a sequence that allows for hybridization with the RCA product and has to have a fluorescent molecule attached. In general, such sequences and fluorescent molecule types are not disclosed by their manufacturer. Another example for the above is molecule type 7 which may be another nucleotide (substrate) suitable for further RCA reaction, or may be fluorescently labeled molecule which emits specific fluorescence, or can exhibit FRET with another molecule, etc.

Examples of commercially available kits, reagents and their suppliers are APO-BRDU kit (AU:1001, Phoenix Flow Systems), HCS DNA Damage Kit (H10292, ThermoFisher,), Colorimetric TUNEL Apoptosis Assay (#8088, ScienCell), OxiSelect^{™} Comet Assay Kit (STA-350, Cell Biolabs, Inc.), NEBuffer2 (NEBiolabs, B7002S), N6-(6-Amino)hexyl-2'-deoxyadenosine-5'-triphosphate - Biotin (Biotin-7-dATP) (Jena Bioscience, NU-835-BIO-S), Biotin-16-Propargylamino-dCTP (Jena Bioscience , NU-809-BIO16), biotin-16-5-aminoallyl-dUTP (Jena Bioscience, NU-803-BIO16), dGTP (Jena Bioscience, NU-1003), DNA Polymerase I (*E.Coli*) (NEBiolabs, MO209), Duolink^{®} In Situ PLA Probe anti-mouse MINUS (Sigma, DUO92004), Duolink^{®} In Situ PLA Probe anti-rabbit PLUS (Sigma, DUO92002), Duolink^{®} In Situ Detection Reagents Green (Sigma, DUO92014), Duolink^{®} In Situ Detection Reagents Red (Sigma, DUO92008), Duolink^{®} In Situ Wash Buffers (Sigma, DUO82049).

### ADVANTAGES OF THE INVENTION

The examples of main advantages of the invention in the light of the state of the art are described below.

The method according to the invention enables direct detection and subsequent visualisation of DNA end(s) what has not been achieved using any known methods from the art. The invention enables marking the presence and position of a single DNA break. These features allow for thinking that the present invention solves the essential technical problem existing in the art, namely, it allows determining the presence, number, and location of single- and double-strand DNA breaks in the nucleus, and gives an ability to distinguish the types of the break(s). This is required in order to assess the quality of DNA of the subject, and for further study of the mechanisms of DNA damage induction, sensing and repair.

Moreover, not only the determination *per se* may be achieved by the method of the invention but also the quantitative assay of the number of DNA ends may be obtained. The above-mentioned feature may be used in evaluating the quality of the biological material, or presence of environmental hazards

As it was mentioned above and exposed in the Claims as well as shown in the Examples, the method according to the invention is highly sensitive; it is more sensitive that other methods known in the art. The present invention allows detecting directly even a single DNA break.

The invention further concerns a method which is highly specific and allows detecting specific type of DNA lesion. Knowledge obtained hereby may be used, for example, for diagnostic purposes, if it is used with proper combination of information concerning etiology of formation of DNA breaks and its roles in genetic disorders.

The method is also universal as regards the scope of invention. It involves the ability of variety of embodiments used, taking into consideration the DNA break type, type of DNA ends, kind of biological material, molecules, reagents and techniques used.

### DESCRIPTION OF THE FIGURES

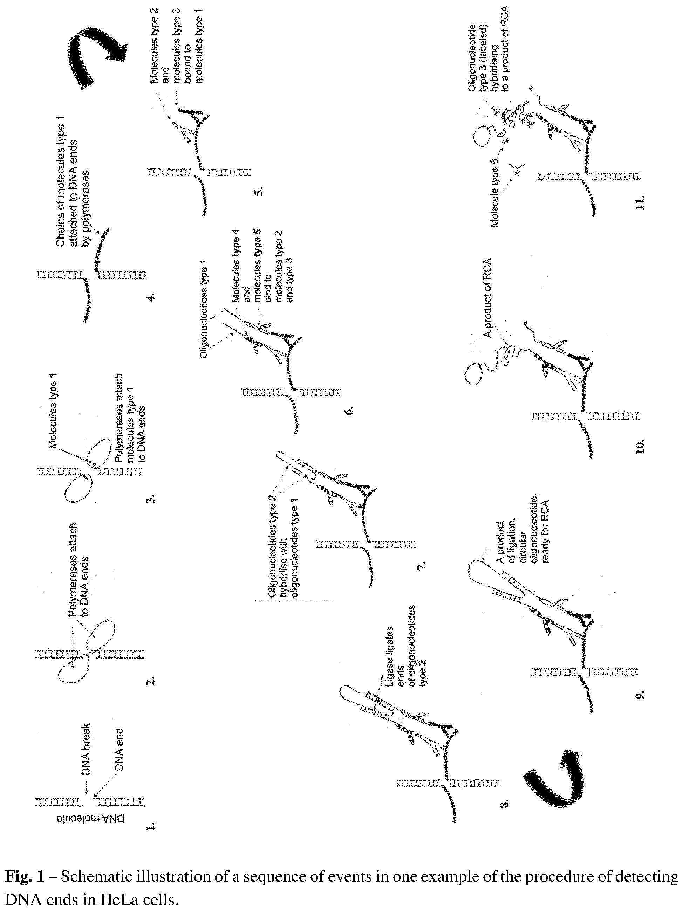

**Fig. 1****.** Schematic illustration of a sequence of events in one example of the procedure of detecting DNA ends in HeLa cells. It is a main part of exemplary variant of a method, namely the embodiment when molecules 2 and 3 are primary antibodies and molecules type 4 and 5 are secondary antibodies, leading to detection of DNA ends in a biological material and it can be described in the 11 following diagrams: 1. A DNA break is formed in a living cell. This scheme represents any type of a single- or double-strand DNA break. 2. Following fixation of the cell, an enzyme recognizes the DNA breaks. 3. The enzyme begins to connect molecules type 1 (nucleotide analogs) to DNA ends. A number of molecules type 1 are added to two DNA ends depicted in a diagram. 4. The action of an enzyme results in formation of a chain of molecules type 1 attached to each of the two recognized DNA ends. 5. Two types of molecules, type 2 and 3, recognize and attach to molecules type 1 attached to DNA ends. The number and titers of the attaching molecules 2 and 3 is sufficiently high to position many molecules type 2 and 3 close to each other on the chain of molecules type 1 (for clarity only the various molecules attaching to one of the two DNA breaks are shown). 6. The molecules type 2 and 3 already attached to a chain of molecules type 1 are recognized by molecules type 4 and 5. These molecules type 4 and 5 are conjugated with DNA oligonucleotides (oligonucleotides type 1, two classes) and bind to molecules 2 and 3. 7. Oligonucleotides (type 2, a solution of two different oligonucleotides) hybridise with oligonucleotides type 1 (that are components of molecules type 4 and 5). 8. Enzyme ligase ligates the ends of the hybridised oligonucleotides type 2 thus preparing a circular oligonucleotide which will be required in RCA reactions. 9. A product of ligation, a circular oligonucleotide hybridised to oligonucleotides type 1, ready for RCA reaction. 10. Product of RCA reaction - a long oligonucleotide attached to one of the antibodies 4 or 5. 11. Fluorescently labeled oligonucleotides type 3 hybridise with the product of RCA reaction thus marking the presence and position of DNA breaks. The Scheme does not include steps for preparation of the biological material since this is not necessary for the explanation of the interactions between molecules type 1-6, on which illustration is focused.

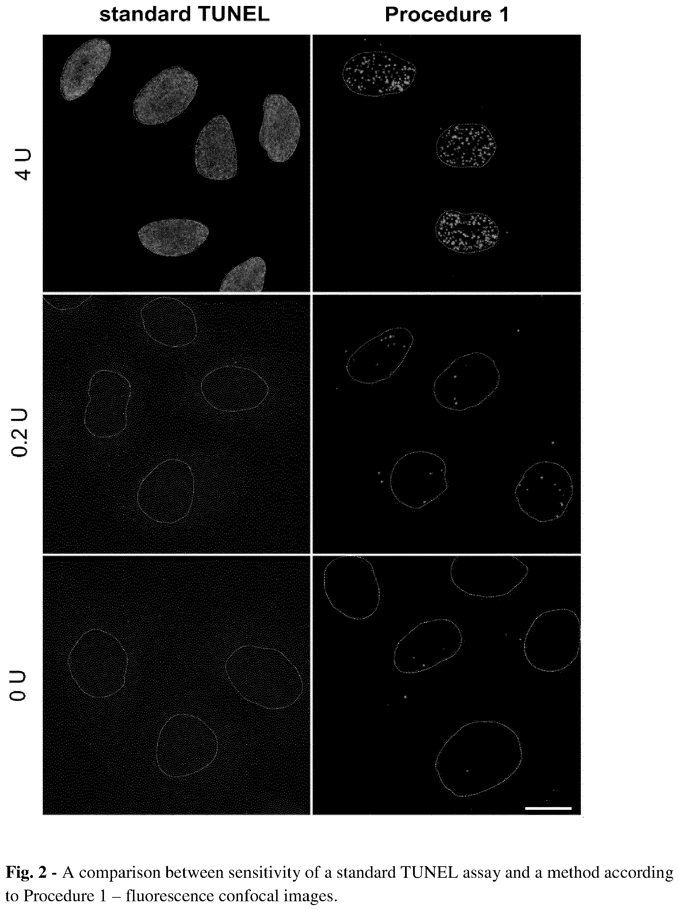

**Fig. 2****.** A comparison between sensitivity of a standard TUNEL assay and a method according to Procedure 1 - fluorescence confocal images. Representative fluorescence confocal images of untreated and DNase I treated HeLa cells in which free DNA ends were detected according to a standard TUNEL assay (APO-BRDU) or Procedure 1 are shown. In samples prepared according to Procedure 1, free DNA ends can be seen as bright and distinct fluorescent foci (right column). For both assays, treatment of cells with a high concentration of DNase I (4U, first row) resulted in a significant increase in the signal in the nuclei when compared to untreated cells (0 U, third row). However, only for samples prepared according to Procedure 1 there was a difference between cells treated with a low concentration of DNase I (second row, right image) and untreated cells (third row, right image) - the number of detected free DNA ends is higher in DNase treated cells. For the standard TUNEL assay, no significant fluorescent signal is observed in samples treated with a low concentration of DNase I (second row, left image) when compared to untreated cells (third row, left image). Scale bar - 20 µm.

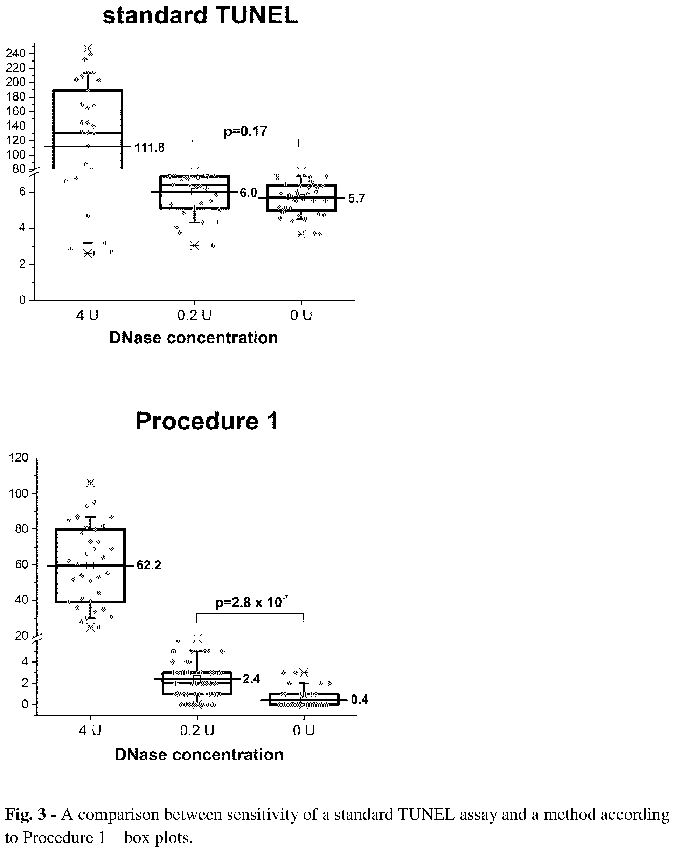

**Fig. 3****.** A comparison between sensitivity of a TUNEL assay and a method according to Procedure 1 - box plots. Box plots representing the results of analysis of microscopic images of DNA breaks. For the standard TUNEL assay (APO-BRDU), the signal was the mean grey value of pixels (fluorescence intensity) within the nucleus (note that TUNEL does not yield images of individual DNa breaks, it only results in a grainy signal throughout the nucleus). For the method according to Procedure 1, the number of foci in each nucleus was determined. The bottom of each box is the 25^{th} percentile, the top is the 75^{th} percentile, the line in the middle is the 50^{th} percentile, the whiskers are the 10^{th} and the 90^{th} percentiles. The solid horizontal line (wider than the box) represents the mean value. An independent two-sample t-test has shown that the difference in mean values between samples treated with a low concentration of DNase I (0.2 U) and untreated samples (0 U) is statistically significant only after processing cells according to Procedure 1 (right graph; p-value=2.8 × 10⁻⁷). This indicates, that the method according to Procedure 1 is significantly more sensitive than the standard TUNEL assay.

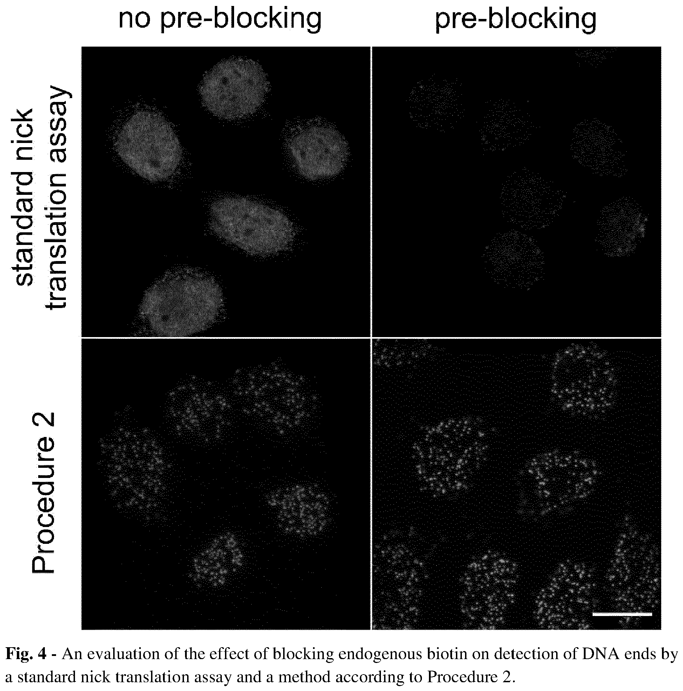

**Fig. 4****.** An evaluation of the effect of blocking endogenous biotin on detection of DNA ends by a standard nick translation assay and a method according to Procedure 2. Representative fluorescence confocal images of untreated HeLa cells in which free DNA ends were detected according to the standard nick translation assay (first row) or Procedure 2 (second row). The samples were prepared with or without pre-blocking step (step 3 in Procedure 2 - blocking endogenous biotin). For samples prepared according to the standard nick translation assay, blocking endogenous biotin results in a significant decrease in fluorescent signal in the nuclei of cells - the difference in signals is clearly visible on recorded confocal images (first row). For samples prepared according to Procedure 2, pre-blocking does not have a major impact on obtained images (second row). Scale bar - 20 µm.

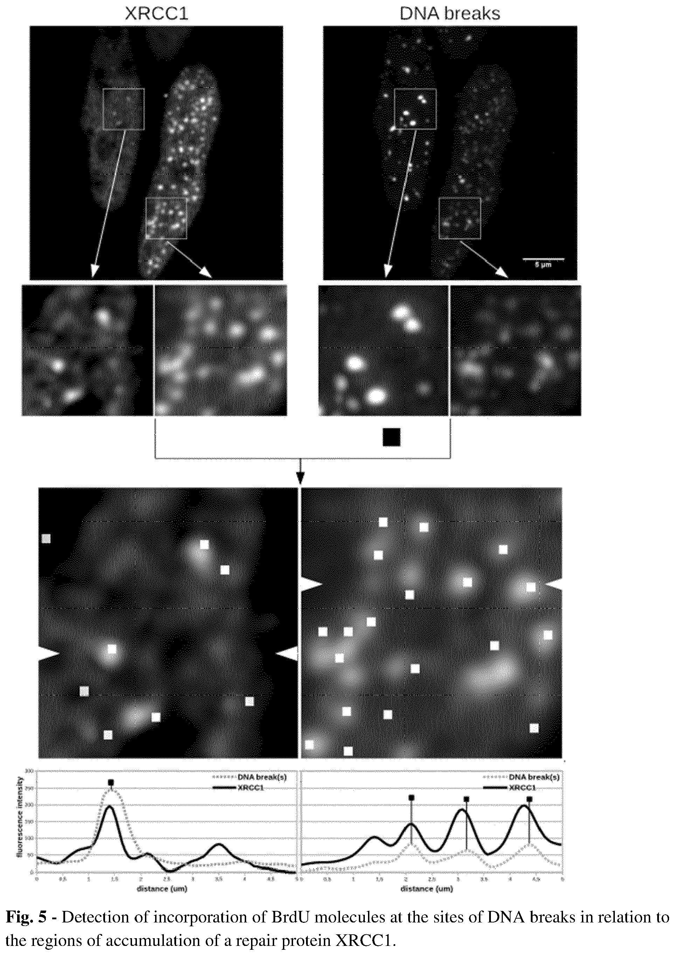

**Fig. 5****.** Detection of incorporation of BrdU molecules at the sites of DNA breaks in relation to the regions of accumulation of a repair protein XRCC1. Incorporation of BrdU molecules marks specific types of DNA 3'-OH ends recognised by TdT enzyme, which are present when double-strand blunt-end breaks or double-strand 3'-protruding breaks or single-strand gaps occur. The gaps are repaired by BER pathway. XRCC1 protein is one of the principal repair factors involved in this repair process. The sites, where BrdU is detected alongside with an adjacent XRCC1 focus, are most probably only the sites where single-strand gaps occur. ImageJ Software was used to analyze the images and to find and assign the fluorescence maxima (squares) of the BrdU foci. The maxima represent centers of mass of the BrdU foci and thus mark the localization of DNA damage. The analyses of the fluorescence profiles (at the bottom, under the panels of microscopy images) of the images representing the repair protein foci and localizing the above-mentioned maxima in the profiles shows that DNA breaks are consistently detected adjacent to or in the peripheral regions of XRCC1 repair foci. There is a distinct spatial association between XRCC1 and BrdU foci detected in the microscopy images. The images are the maximum intensity projections of 3D stack images. The profiles of fluorescence intensity were measured in the regions between the arrowheads.

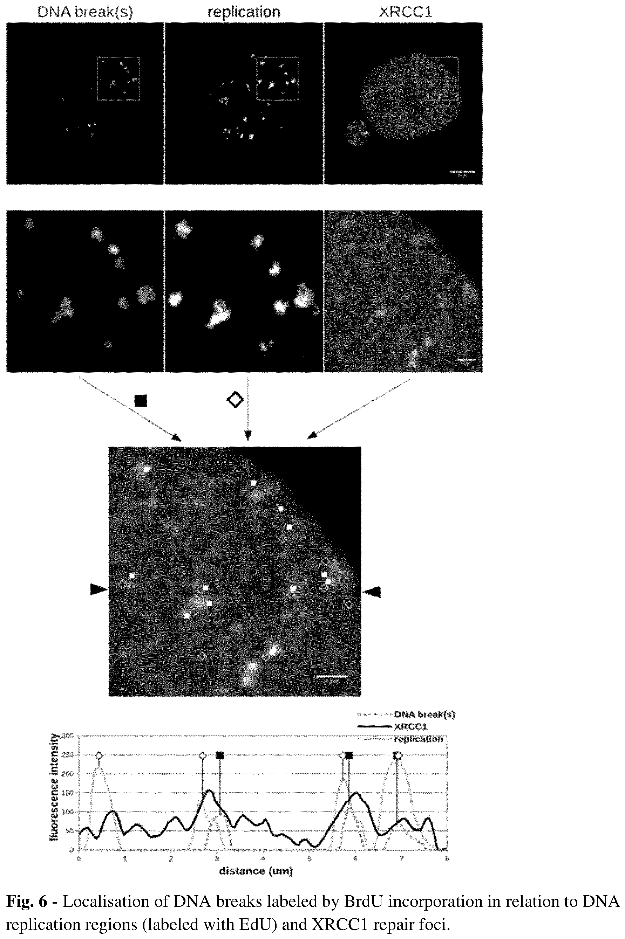

**Fig. 6****.** Localization of DNA breaks labeled by BrdU incorporation, which are most probably single-strand gaps (as described and explained in Example 4) in relation to DNA replication regions (labeled with EdU) and XRCC1 repair foci. The analysis of the fluorescence profiles (at the bottom) of the images representing the repair protein foci and localizing the fluorescence maxima in the profiles (BrdU foci - square, DNA replication regions - rhombus) confirm spatial association between the three types of subnuclear objects. ImageJ Software was used to analyze the images and to find and assign the fluorescence maxima and to generate the fluorescence intensity profiles. Not every region of DNA replication is associated with the region of BrdU detection what proves minimized cross-reactivity of the method of detection according to Procedure 1 (the method only leads to detection of BrdU without any nonspecific detection of EdU). The images are the maximum intensity projections of 3D stack images. The profiles of fluorescence intensity were measured in the regions between the arrowheads. Scale bars: 5 µm or 1 µm (in the enlarged parts of the images).

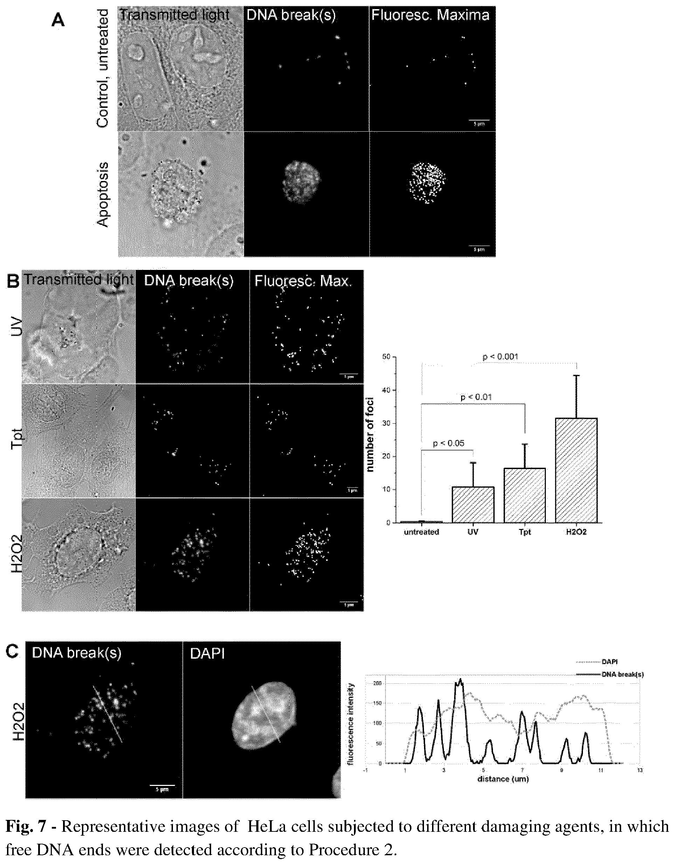

**Fig. 7****.** Examples of control, apoptotic and damaged HeLa cells. A). Examples of images of cell nuclei in which BrdU was incorporated and detected using a method according to Procedure 1. This example proves applicability of the method to detection of apoptotic cells in cell culture. The nucleus of the apoptotic cell contains significantly more DNA breaks than the nucleus of a normal, control cell. B). Examples of images of cells treated with different damaging agents (UV, topotecan (Tpt) and H₂O₂). The box plot shows that the treatment with different agents leads to the increase of the average number of detectable regions of incorporation of BrdU molecules in comparison to untreated, control cells. Regions of incorporation of BrdU represent regions of localisation of free ends of DNA (the average number in cells treated with H₂O₂: 31.57 ± 8.61, UV: 10.73 ± 4.89, Tpt: 16.39 ± 4.92). The results were tested using student's t-test (p-value). The error bars represent standard error of the mean. C). The panel presents an example of images illustrating localization of double-strand blunt-end breaks or double-strand 3'-protruding breaks or single-strand gaps in a cell nucleus treated with H₂O₂ in relation to the intra-nuclear and chromatin structure (DAPI). The profiles of fluorescence intensity were obtained using ImageJ software from the regions marked with the lines in the images. Closer look at fluorescence intensity of DAPI and fluorescence maxima representing DNA breaks (BrdU foci) can give information about the local density of chromatin in the damaged regions. In the presented images BrdU foci were visualized with fluorescence maxima detected using ImageJ Software. The images are the maximum intensity projections of 3D stack images. Scale bars: 5 µm.

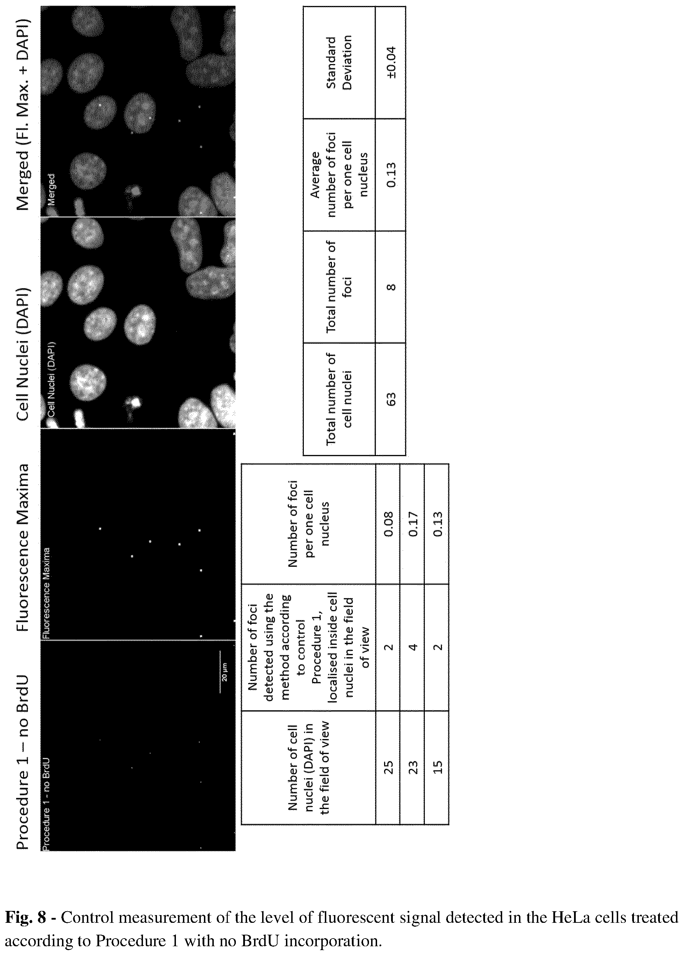

**Fig. 8****.** Control measurement of the level of fluorescent signal detected in the HeLa cells treated according to Procedure 1 with no BrdU incorporation. The number of detected foci and the number of observed nuclei shows that on average there are only 0.13 ± 0.04 (total number of nuclei N = 63) foci detected per a single cell nucleus. The foci were defined as the regions where the level of fluorescent signal was higher than 0 a.u. and their localisation was determined using a built-in feature of the ImageJ Software that localizes fluorescence maxima. The majority of them were localized outside of cell nuclei what was determined based on the overlay of fluorescence maxima with the fluorescent images of cell nuclei stained with DAPI. The presented microscopy images are the maximum intensity projections of 3D stack images.

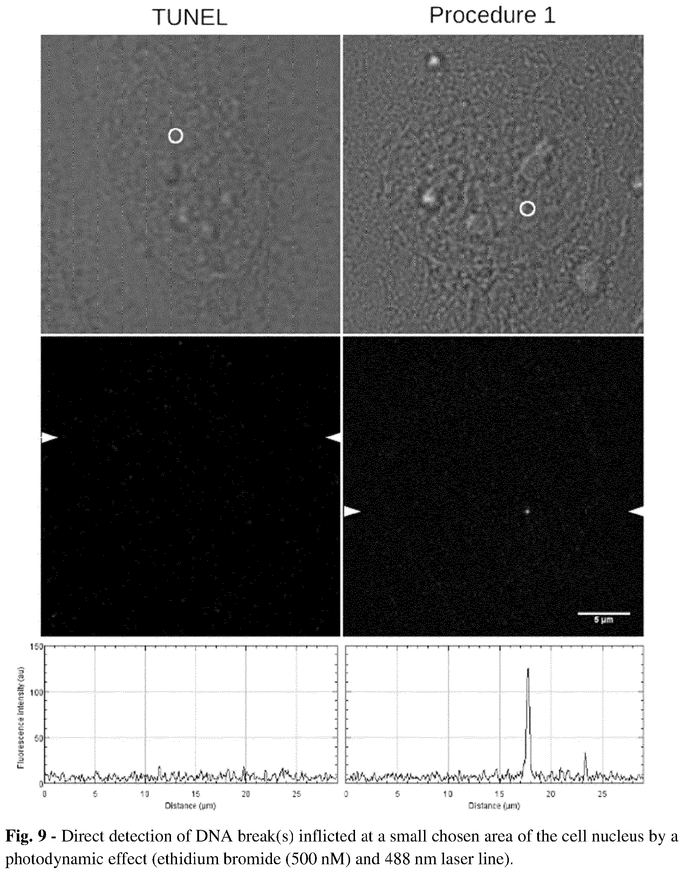

**Fig. 9****.** Direct detection of DNA break(s) inflicted at a small chosen area of the cell nucleus by a photodynamic effect (ethidium bromide (500 nM) and 488 nm laser line).
   Hela cells were incubated with ethidium bromide (500 nM) and illuminated using 488 nm laser line (9 mJ - the dose known to induce damage (Zarebski, M., Wiernasz, E. and Dobrucki, J. W. (2009), Recruitment of heterochromatin protein 1 to DNA repair sites. Cytometry, 75A: 619-625. doi:10.1002/cyto.a.20734)) at places marked by the white circle (first row). DNA breaks were detected by standard TUNEL (left column) and according to Procedure 1 (right column). DNA break(s) (focus) are visible only after detection according to Procedure 1, as shown on the fluorescence images (second row) and fluorescence intensity profiles (third row, fluorescence intensity profiles were measured between arrowheads). ImageJ Software was used to analyze the images and to find and assign the fluorescence maxima and to generate the fluorescence intensity profiles.

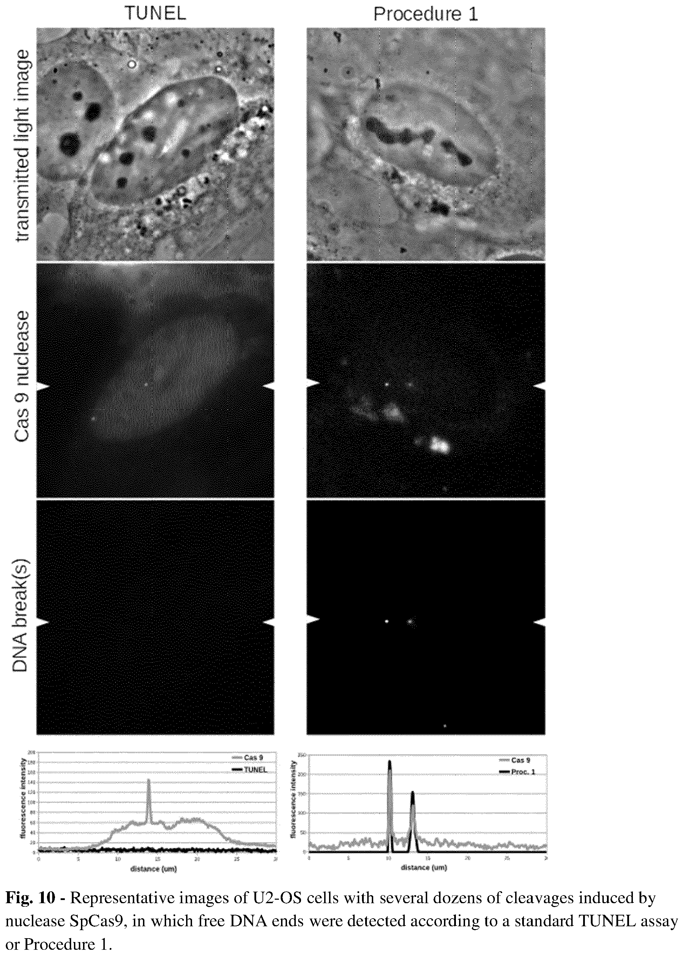

**Fig. 10****.** Comparison between a standard TUNEL method and a method according to Procedure 1 used in detection of several dozens of cleavages induced by nuclease SpCas9 in U2-OS cells (the cleavages induced in subtelomeric region of chromosome 3). The images and the profiles of fluorescence intensity show that average value of the fluorescence intensity that was measured in the regions associated with the accumulation of nuclease SpCas9 and emitted by Alexa Fluor^{®} 594 conjugated with the secondary antibodies used in standard immunofluorescent detection of BrdU (standard TUNEL assay) was approximately 0 a.u. (50 nuclei were analysed and the value was normalized based on the level of the background signal, representing non-specifically bound antibody molecules). The example of the fluorescence profiles measured for the representative example of recorded microscopy images distinctively illustrate that no BrdU foci could be detected in the microscopy images recorded using a standard fluorescence wide-field microscope when standard TUNEL assay was applied. Respectively, when the method according to Procedure 1 was applied no non-specific background signal was detected and the regions of incorporation of BrdU were represented by distinct foci visualized in the microscopy images. These foci co-localized with the regions of accumulation of SpCas9, what was additionally illustrated in the profiles of fluorescence intensity. The fluorescence intensity profiles were generated for the regions between the arrowheads. Image size: 30 × 30 µm.

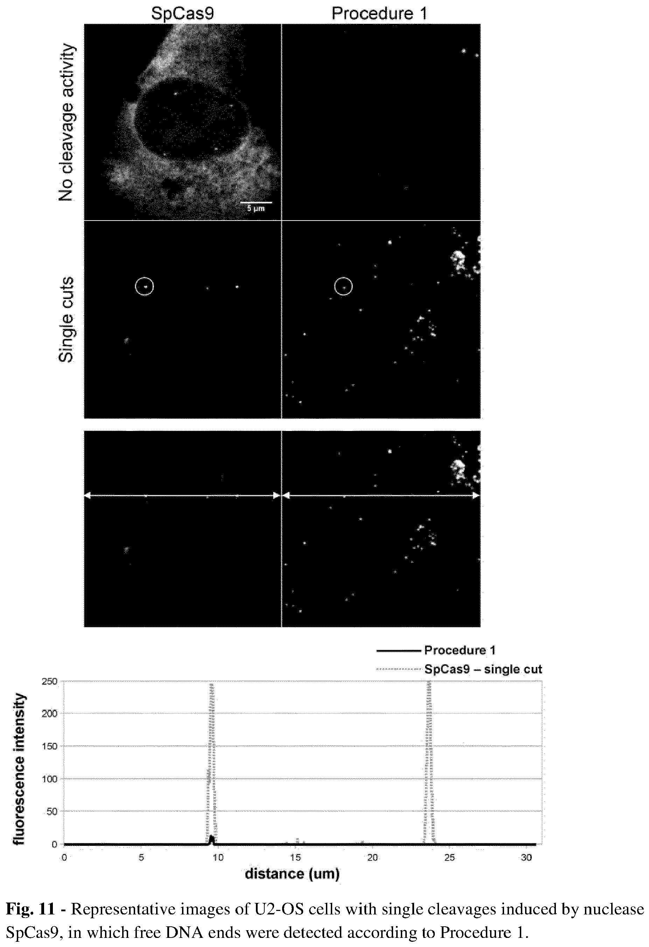

**Fig. 11****.** Application of a method according to Procedure 1 in detection of single cleavages induced by nuclease SpCas9 in human U2-OS cells. When SpCas9 does not perform any cleavage activity, no BrdU foci can be detected in the regions of the accumulation of the nuclease. In turn, induction of a single cut at the site of accumulation of SpCas9 results in detection of incorporated BrdU molecules. The distinct BrdU focus is spatially associated with the SpCas9 focus, what is additionally illustrated by the profiles of fluorescence intensity. The profiles were measured between the arrowheads. The results prove that the method according to Procedure 1 can reach very high sensitivity in recognition of DNA breaks -even only one, single double-strand DNA break can be detected. The presented microscopy images are the maximum intensity projections of 3D stack images.

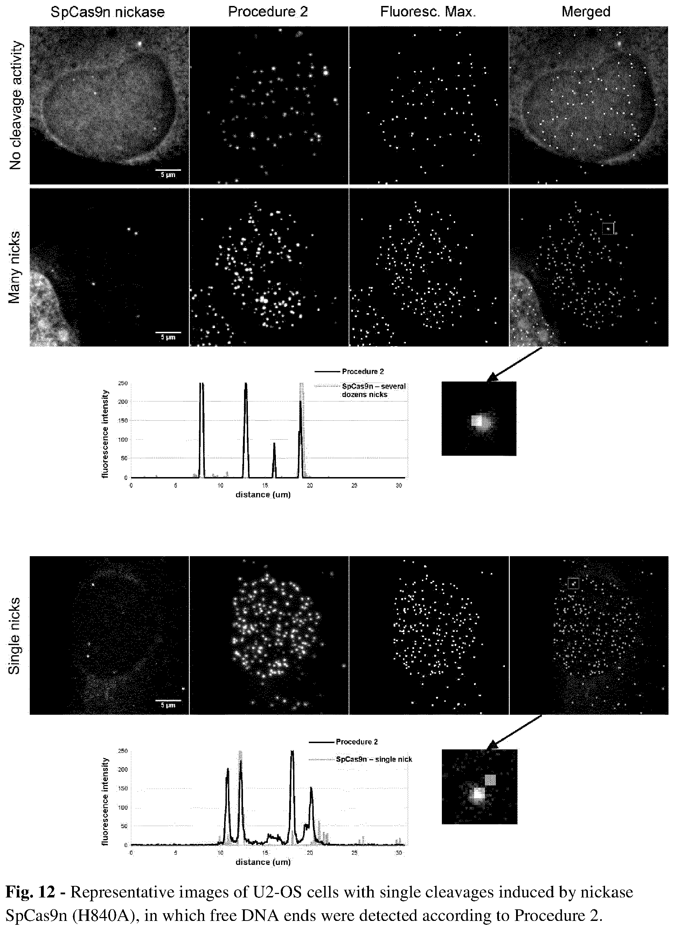

**Fig. 12****.** Application of a method according to Procedure 2 in detection of single cleavages induced by nickase SpCas9n (H840A) in human U2-OS cells. When SpCas9n does not perform any cleavage activity, no foci of biotinylated nucleotides can be detected in the regions of the accumulation of the nickase. There is no spatial association between these two subnuclear objects. In turn, induction of either a single nick or up to several dozens of nicks at the site of accumulation of SpCas9n results in detection of incorporated modified nucleotides. The foci of biotinylated nucleotides were localized and represented by fluorescence maxima in the images. When only one single-strand DNA break is induced, the site of the incorporation of nucleotides colocalizes with the SpCas9n focus. If the number of nicks is higher than one (up to several dozens) the regions of incorporation of nucleotides and SpCas9n foci also colocalize. The presented profiles of fluorescence intensity distinctively depict this association (the maxima representing the BrdU and SpCas9n foci overlap). The results prove high sensitivity of the method performed according to Procedure 2. The method can be applied in detection of single single-strand DNA breaks *in situ* in cell nuclei. The presented microscopy images are the maximum intensity projections of 3D stack images. The profiles of fluorescence intensity were obtained using ImageJ software along the horizontal lines running across the SpCas9n foci of interest.

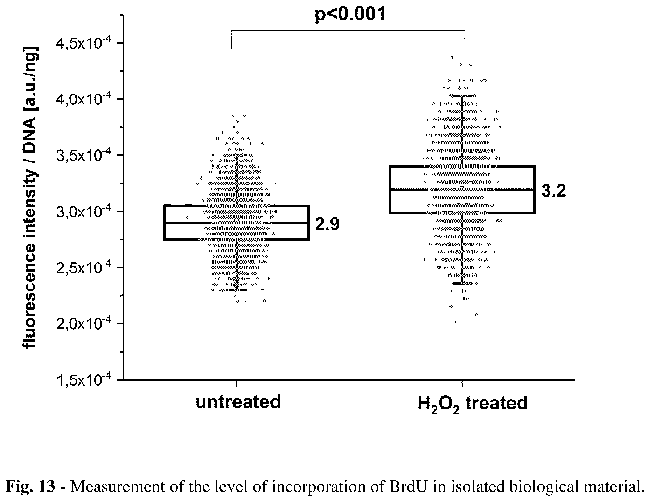

**Fig. 13****.** Measurement of the level of incorporation of BrdU in isolated biological material. The material was isolated from untreated, control HeLa cells and HeLa cells that were subjected to H₂O₂ (4 mM, 30 minutes). The number of free DNA ends increases as a result of the treatment with damaging agents, such as H₂O₂, what results in the increase of the number of incorporated BrdU molecules and the increase of total fluorescence intensity signal in the samples (BrdU was detected using the method according to Procedure 1 followed by the extraction of cellular components including chromatin and DNA). The final outcome, which is presented on the graph (the box plot), is the fluorescence intensity per 1 ng of DNA. The bottom of each box is the 25th percentile, the top is the 75th percentile, the whiskers are the 10th and the 90th percentiles and the line in the middle is the mean value. The measured fluorescence intensity per DNA unit (1 ng) in the control sample was 2.9 ± 0.2 × 10⁻⁴ a.u., whereas the fluorescence intensity measured per 1 ng of DNA in the sample treated with H₂O₂ was higher (3.2 ± 0.3 × 10⁻⁴ a.u.) (see the table and the plot). An independent two-sample t-test has shown that the difference between fluorescence intensity per 1 ng of DNA between untreated and H2O2 treated samples is statistically significant (p-value < 0.001). The concentration of DNA was measured using a multimode plate reader Infinite 200 Pro (Tecan).

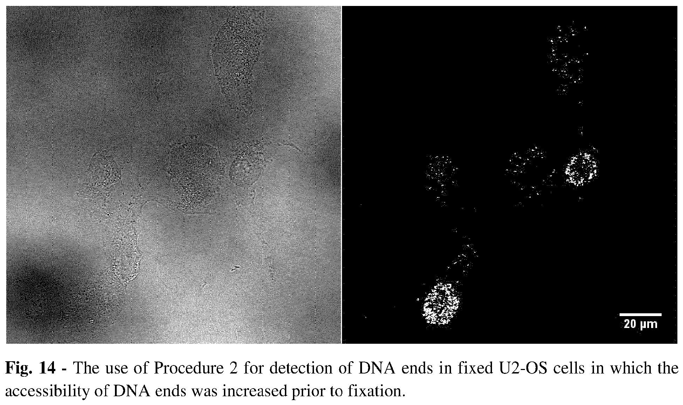

**Fig. 14****.** -_The use of Procedure 2 for detection of DNA ends in fixed U2-OS cells in which the accessibility of DNA ends was increased prior to fixation. The right-hand image presents fluorescent foci representing modified DNA ends (according to Procedure 2). They are specifically detected inside cell nuclei (the nuclei are localized in the transmitted light image on the left) and their number differs between individual cells. Regarding the localization of fluorescent foci representing single-strand DNA breaks the obtained images are reliable, what proves the applicability of the step of incubation of live cells (prior to fixation) with Tris (in this case 1 mM, incubation for 20 minutes at 37°C) to increase accessibility of chromatin and DNA ends. The images are the maximum intensity projections of 3D stack images.

### EXAMPLES

The present invention is explained more in detail with the aid of the following examples which are not intended to limit the scope of the present invention in any manner.

Examples are described on the basis of general protocols which concern main embodiments presented in the description.

For comparative purposes, the following examples use, in most cases, the standard method for detection of DNA breaks, i.e. the TUNEL assay. TUNEL assay (Terminal deoxynucleotidyl transferase dUTP nick end labeling) is a technique of detecting DNA ends in fixed cells, which employs polymerase Tdt, nucleotide analogs and anti-analog antibodies, and fluorescence detection, as disclosed in patent application WO 1997008345 A1, and a communication Darzynkiewicz Z, Galkowski D, Zhao H. Analysis of apoptosis by cytometry using TUNEL assay. Methods. 2008 Mar;44(3):250-4. doi: 10.1016/j.ymeth.2007.11.008.

### General procedures

### Procedure 1

General procedure of *in situ* DNA damage detection performed using enzyme **TdT (terminal deoxynucleotidyl transferase)** - detection of single-strand gaps, double-strand blunt-end breaks or double-strand 3'-protruding breaks.

Sample preparation: cells cultured on coverslips or on Petri dishes with glass bottom under standard conditions for the type of used cells. The density of the cell culture should reach approximately 50% - 60% of confluency at the beginning of the procedure.

Procedure:
1. Pre-preparation *(optional):*
   Incubation of live cells with 0.5 - 10 mM Tris (5 min - 1 h at room temperature (RT)), cells should be monitored for morphological abnormalities.
2. Fixation and permeabilization:
   a. Incubation with 1% - 4% formaldehyde solution, 10-15 min, 4°C. *(optional)*
   b. Incubation with an ionic detergent (e.g. Triton X-100 (0.25 - 3%) 10 min - 1 h, RT. *(optional)*
   c. Incubation in 70% ethanol in water, 1 h minimum but preferred 12-18 h, -20°C, sample should be stable for several months. *(optional but most preferred)*
3. Pre-blocking *(optional):*
   a. Wash (PBS 1 × 5 min)
   b. Streptavidin solution - incubation performed on a droplet, 30 min, RT
   c. Wash (PBS 3 × 5 min)
   d. Biotin solution - incubation performed on a droplet, 30 min, RT
   e. Wash (PBS 3 × 5 min)
4. Increasing accessibility of DNA ends:
   Incubation with 0.5-10 mM EDTA in water, 10 min - 1 h, RT.
5. Incorporation procedure:
   Incorporation of unmodified and modified nucleotides (e.g. BrdU) using terminal deoxynucleotidyl transferase:
   a. incubation is performed on a droplet in a humid chamber
   b. samples are rinsed in wash buffers
6. Blocking:
   Incubation with 1- 5% BSA solution in PBS (from 1 h to overnight in *4°C, RT).*
7. Incubation with primary antibodies - performed on a droplet in a humid chamber:
   a. primary antibodies type 1 (e.g. mouse monoclonal against BrdU), preferred dilution 1:100 in 1-5% BSA, 1 h, RT
   b. wash (PBS 3 × 5 min)
   c. primary antibodies type 2 (e.g. rabbit polyclonal against BrdU), preferred dilution 1:100 in 1-5% BSA, 1 h, RT
   d. wash (PBS 3 × 5 min)
8. PLA procedure:
   a. incubation with PLA Probes diluted in 1% BSA (37 °C, 60 min, reaction on a droplet, in a humid chamber) in 40 µl of the reaction mixture per one coverslip:
      - 8 µl of PLA Probe MINUS stock (e.g. anti-mouse)
      - 8 µl of PLA Probe PLUS stock (e.g. anti-rabbit)
      - 24 µl of 1% BSA
   b. wash samples with Wash Buffer A (2 × 5 min)
   c. ligation - incubation (37°C, 30 min, reaction on a droplet, in a humid chamber) in 40 µl of the reaction mixture per one coverslip:
      - 8 µl of 5x Ligation stock (Sigma, Duolink, DUO82009),
      - 31 µl of ultrapure, RNAse/DNAse free, distilled water,
      - 1 µl of Ligase (1 U/µl) (Sigma, Duolink, DUO82027 or DUO82029)
   d. wash samples with Wash Buffer A (2 × 2 min)
   e. amplification - incubation (37°C, 100 min, reaction performed on a droplet, in a humid chamber) in 40 µl of the reaction mixture per one coverslip:
      - 8 µl of 5x Amplification stock (Sigma, Duolink, DUO82060 or DUO82011),
      - 31.5 µl of ultrapure, RNAse/DNAse free, distilled water,
      - 0.5 µl of Polymerase (10 U/µl) (Sigma, Duolink, DUO82028 or DUO82030)
9. Imaging of samples in PBS using a fluorescence microscope.

### Procedure 2

General procedure of DNA damage detection performed using enzyme **DNA polymerase I** - detection of single-strand nicks, single-strand gaps and double-strand 3' recessed ends.

Sample preparation: cells cultured on coverslips or on Petri dishes with glass bottom under standard conditions for the type of used cells. The density of the cell culture should reach approximately 50% - 60% of confluency at the beginning of the procedure.

Procedure:
1. Pre-preparation *(optional):*
   a. Incubation of live cells with 0.5 - 10 mM Tris (5 min - 1 h at room temperature (RT)), cells should be monitored for morphological abnormalities.
2. Fixation and permeabilization:
   a. Incubation with 1% - 4% formaldehyde solution, 10-15 min, 4°C. *(optional)*
   b. Incubation with an ionic detergent (e.g. Triton X-100 (0.25 - 3%) 10 min - 1 h, RT. *(optional)*
   c. Incubation in 70% ethanol in water, 1 h minimum but preferred 12-18 h, -20°C, sample should be stable for several months. *(optional but most preferred)*
3. Pre-blocking *(optional):*
   a. Wash (PBS 1 × 5 min)
   b. Streptavidin solution - incubation performed on a droplet, 30 min, RT
   c. Wash (PBS 3 × 5 min)
   d. Biotin solution - incubation performed on a droplet, 30 min, RT
   e. Wash (PBS 3 × 5 min)
4. Increasing accessibility of DNA ends:
   Incubation with 0.5-10 mM EDTA in water, 10 min - 1 h, RT.
5. Incorporation of unmodified and modified (e.g. biotin-conjugated) nucleotides using DNA polymerase I:
   a. Rinse the samples with distilled water
   b. Incubation (5 - 60 min, RT on a droplet, in a humid chamber) in reaction mixture consisting of:
      - reaction buffer
      - dNTPs (the ratio of modified to unmodified nucleotides varies from 3:1 to 1:3)
      - DNA polymerase I
      - ultrapure, RNAse/DNAse free, distilled water
   c. Reaction termination:
      - incubation with 50 mM TrisHCl (pH 7.48), 2 × 10 min, RT
      - incubation with 0.5 mM TrisHCl , 1 × 1 min RT
6. Blocking:
   1- 5% BSA solution in PBS (from 1 h to overnight in *4°C, RT).*
7. Incubation with primary antibodies - performed on a droplet in a humid chamber:
   a. primary antibodies type 1 (e.g. mouse monoclonal against biotin), preferred dilution 1:100 in 1-5% BSA, 1 h, RT
   b. wash (PBS 3 × 5 min)
   c. primary antibodies type 2 (e.g. rabbit polyclonal against biotin), preferred dilution 1:100 in 1-5% BSA, 1 h, RT
   d. wash (PBS 3 × 5 min)
8. PLA procedure:
   a. incubation with PLA Probes diluted in 1% BSA (37 *°C,* 60 min, reaction on a droplet, in a humid chamber) in 40 µl of the reaction mixture per one coverslip:
      - 8 µl of PLA Probe MINUS stock (e.g. anti-mouse)
      - 8 µl of PLA Probe PLUS stock (e.g. anti-rabbit)
      - 24 µl of 1% BSA
   b. wash samples with Wash Buffer A (2 × 5 min)
   c. ligation - incubation (37°C, 30 min, reaction on a droplet, in a humid chamber) in 40 µl of the reaction mixture per one coverslip:
      - 8 µl of 5x Ligation stock (Sigma, Duolink, DUO82009),
      - 31 µl of ultrapure, RNAse/DNAse free, distilled water,
      - 1 µl of Ligase (1 U/µl)(Sigma, Duolink, DUO82027 or DUO82029)
   d. wash samples with Wash Buffer A (2 × 2 min)
   e. amplification - incubation (37°C, 100 min, reaction performed on a droplet, in a humid chamber) in 40 µl of the reaction mixture per one coverslip:
      - 8 µl of 5x Amplification stock (Sigma, Duolink, DUO82060 or DUO82011),
      - 31.5 µl of ultrapure, RNAse/DNAse free, distilled water,
      - 0.5 µl of Polymerase (10 U/µl) (Sigma, Duolink, DUO82028 or DUO82030)
9. Imaging of samples in PBS using a fluorescence microscope.

### Example 1

### Detection of free DNA ends in untreated and DNase I treated fixed HeLa cells using the method according to Procedure 1.

In this experiment HeLa 21-4 cells (obtained from P.R. Cook, University of Oxford) were used. Cells were seeded on 18-mm coverslips (number of cells: 0.2 × 10⁶ per 1 coverslip) and cultured for 3 days in DMEM supplemented with 10% FBS. Subsequently, cells were incubated in 70% ethanol in water for 12 hours at -20°C. Then, cells were incubated in 0.5 mM EDTA in water for 30 minutes at room temperature. Before processing of DNA ends, some samples were treated with DNase I in order to induce DNA breaks. The reaction was performed in a droplet for 30 minutes at room temperature in a reaction mixture consisting of: 0.2 or 4 units of DNase I (Thermo Fisher Scientific, AM2222), 1x DNase I buffer (Thermo Fisher Scientific, AM8170G) and water. Subsequently, BrdU was linked to free ends of DNA using TdT enzyme using APO-BRDU kit (Phoenix Flow Systems, AU: 1001). The cells were then incubated with 1% BSA solution in PBS overnight at 4°C. The incubations with primary antibodies type 1 and 2 according to step 7 of Procedure 1 were performed using mouse monoclonal anti-BrdU (dilution: 1:100 in 1% BSA) (Abcam, ab8039) and rabbit polyclonal anti-BrdU (dilution: 1:100 in 1% BSA) (Abcam, ab152095), respectively. After last washing, cells were treated according to step 8 (PLA procedure) using Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002), Detection Reagents Green (Sigma, DUO92014), Duolink In Situ Wash Buffers (Sigma, DUO82049).PLA Probes were diluted in 1% BSA. After treatment the samples were imaged and analysed using a Leica TCS SP5 confocal microscope (excitation: 488 nm, emission: 500 - 600 nm).

### Results:

Most of the untreated HeLa cells subjected to Procedure 1 displayed no fluorescent foci, representing free DNA ends, within the cell nucleus. However, up to 3 foci were detected in the nuclei of a subset of cells (Fig. 2.; third row, right image). In cells treated with DNase I more foci were detected than in untreated cells. For the lower and higher DNase I concentration the maximum number of detected foci were 12 and 156, respectively. The average number of foci detected in untreated and DNase I treated (low and high concentration) cells was: 0.4 ± 0.1 (N=49), 2.4 ± 0.3 (N=78) and 62.2 ± 4.6 (N=36) (Fig. 3.). The difference between the average number of foci in untreated and DNase I treated (low concentration) cells is statistically significant, with a p-value = 2.3 × 10⁻⁷ (independent two sample t-test).

### Comparative example 1

### Detection of free DNA ends in untreated and DNase I treated fixed HeLa cells using the standard TUNEL assay.

HeLa 21-4 cells (obtained from P.R Cook, University of Oxford) were seeded on 18-mm coverslips (number of cells: 0.2 × 10⁶ per 1 coverslip), cultured for 3 days in DMEM supplemented with 10% FBS and then processed according to the APO-BRDU (TUNEL assay) kit instructions. After fixation in ethanol, some samples were treated with DNase I in order to induce DNA breaks. The reaction was performed in a droplet for 30 minutes at room temperature in a reaction mixture consisting of 0.2 or 4 units of DNase I (Thermo Fisher Scientific, AM2222), 1x DNase I buffer (Thermo Fisher Scientific, AM8170G) and water. The antibodies used for immunofluorescent staining of BrdU were: mouse monoclonal anti-BrdU (dilution: 1:100 in 1% BSA) (Abcam, ab8039) and goat anti-mouse IgG (H+L) Highly Cross-Adsorbed Secondary Antibody, Alexa Fluor 488 (Invitrogen, Thermo Fisher Scientific, A-11029). After treatment, samples were imaged using a Leica TCS SP5 confocal microscope (excitation: 488 nm, emission: 510-600 nm).

Comparative Example 1 has been compared with the results of Example 1.

### Results:

Microscopic images were analysed using ImageJ software. At least 30 nuclei were analysed for each sample. For the standard TUNEL assay (APO-BRDU), the signal was the mean grey value of pixels within the nucleus (fluorescence intensity). For the method according to Procedure 1, the number of foci in each nucleus was determined. For both assays (the standard TUNEL and the method according to Procedure 1), in samples treated with a high concentration of DNase I (4 U), a significant increase in signal was observed when compared to untreated cells (standard TUNEL, fluorescence intensity: 111.8 ± 14.6 a.u. (N=34) in DNase treated vs 5.7 ± 0.1 a.u. (N=40) in untreated, Procedure 1: 62.2 ± 4.6 foci (N=36) in DNase treated vs 0.4 ± 0.1 foci (N=49) in untreated) (Fig.3) However, an independent two-sample t-test has shown that the difference in signals between samples treated with a low concentration of DNase I (0.2 U) and untreated samples was statistically significant only after processing cells according to Procedure 1 (standard TUNEL, fluorescence intensity 6.0 ± 0.2 a.u. (N=34) in DNase treated vs 5.7 ± 0.1 a.u. (N=40) in untreated, p-value = 0.2; Procedure 1: 2.4 ± 0.3 foci (N=78) in DNase treated vs 0.4 ± 0.1 foci (N=49) in untreated, p-value = 2.3 × 10⁻⁷.). Representative images and quantitative analysis are presented in Figure 2 and Figure 3.

### Conclusion:

The method according to Procedure 1 is significantly more sensitive than the standard TUNEL assay.

### Example 2

### Detection of free DNA ends in untreated fixed HeLa cells using the method according to Procedure 2

In this experiment HeLa 21-4 cells (obtained from P.R. Cook, University of Oxford) were used. The cells were seeded on 18-mm coverslips (number of cells: 0.2 × 10⁶ per 1 coverslip), cultured for 3 days in DMEM supplemented with 10% FBS and then incubated in 70% ethanol in water for 12 hours at -20°C. After that the samples were treated according to step 3 of Procedure 2 (blocking endogenous biotin) (Molecular Probes, Endogenous Biotin Blocking Kit, E-21390). Increasing of accessibility of DNA ends (step 4) was performed with 0.5 mM EDTA in water for 30 min at RT. To incorporate unmodified and modified biotin-conjugated nucleotides, cells were dipped in UltraPure Distilled Water (Invitrogen, Thermo Fisher Scientific, 10977-035) and then incubated (1 hour incubation at 37°C in a humid chamber) with reaction mixture consisting of 1x NEBuffer2 (NEBiolabs, B7002S), 30 µM of each dNTP (Jena Bioscience, dATP: NU-1001, dCTP: NU-1002, dGTP: NU-1003, biotin-16-5-aminoallyl-dUTP: NU-803-BIO16), 3 units of DNA polymerase I (E.Coli) (NEBiolabs, MO209) per coverslip and UltraPure Distilled Water. To terminate the reaction, cells were next incubated as described in step 5(c) of Procedure 2 followed by blocking step performed with 1% BSA solution in PBS (overnight at 4°C ). The incubation with primary antibodies type 1 and 2 (step 7 of Procedure 2) were performed using mouse monoclonal anti-biotin (Abcam, ab201341) (1:100 in 1% BSA) and rabbit polyclonal anti-BrdU (Abcam, ab53494) (1:100 in 1% BSA), respectively. After last washing, cells were treated according to step 8 (PLA procedure) using Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002), Detection Reagents Green (Sigma, DUO92014), Duolink In Situ Wash Buffers (Sigma, DUO82049). After treatment, samples were imaged using a Leica TCS SP5 confocal microscope (excitation: 488 nm, emission: 510-600 nm).

### Results

Free DNA ends, represented as bright fluorescent foci, were readily detected in untreated HeLa cells processed according to Procedure 2 (with step 3 - blocking endogenous biotin) (Fig. 4., second row, right image). The average number of foci detected per one nucleus was 116 ± 6 (N=23).

### Example 3

### Detection of free DNA ends in fixed HeLa cells using the method according to Procedure 2 - evaluation of the effect of blocking endogenous biotin.

HeLa 21-4 cells were treated as in Example 2 but without step 3 (pre-blocking) from Procedure 2.

### Results:

Free DNA ends, represented as bright fluorescent foci, were readily detected in untreated HeLa cells processed according to Procedure 2 (without step 3 - blocking endogenous biotin) (Fig. 4., second row, left image). The average number of foci detected per one nucleus was 128 ± 6 (N=24).

### Conclusion (example 2 and 3):

Slightly more free DNA ends were detected in samples processed according to Procedure 2 without blocking endogenous biotin (step 3) than in samples in which this step was present (128 ± 6 vs 116 ± 6). However, an independent two-sample t-test yielded a p-value = 0.15, indicating that the difference between these two samples is not statistically significant. Thus, one can conclude that blocking endogenous biotin, while still recommended, is not crucial for the results obtained by processing samples according to Procedure 2.

To determine the difference between standard techniques and the method of the invention, the following comparative example was performed.

### Comparative example 2

### Detection of free DNA ends in fixed HeLa cells using DNA Polymerase I and the standard nick translation assay.

HeLa cells were treated as in Example 2 or 3, but steps 7c-8 from Procedure 2 were omitted. Instead, step 7b was followed by incubation with a secondary antibody. The secondary antibody used was: goat anti-mouse IgG (H+L) Highly Cross-Adsorbed Secondary Antibody, Alexa Fluor 488 (Invitrogen, Thermo Fisher Scientific, A-11029). After treatment, samples were imaged using a Leica TCS SP5 confocal microscope (excitation: 488 nm, emission: 510-600 nm).

### Results:

Both protocols (standard and method of the present invention) allowed to detect free DNA ends in untreated HeLa cells (Fig. 4). The effect of blocking endogenous biotin was readily visible in samples treated according to the standard nick translation assay - pre-blocking resulted in a significant decrease in fluorescence signal when compared to samples without endogenous biotin blocking step (fluorescence signal: 11 a.u. in samples with step 3 vs 92 a.u. in samples without step 3) (Fig. 4., first row). In samples prepared according to Procedure 2 with or without step 3, the difference between the average number of foci was not statistically significant (116 ± 6 vs 128 ± 6 foci, independent two-sample t-test: p-value = 0.15) (Fig. 4., second row).

### Conclusion:

While both assays utilizing DNA Polymerase I are able to detect free DNA ends in untreated cells, the one according to Procedure 2 is advantageous due the possibility of quantification of DNA breaks. This stems from the fact that in samples prepared according to this procedure, free DNA ends are represented as bright fluorescent foci easily detectable on a near-zero background. Also, the comparison of the effect of blocking endogenous biotin on the results obtained using these methods shows that the method according to Procedure 2 is more specific, i.e. leads to less false positives, than the standard assay. This conclusion is strongly supported by the fact that blocking endogenous biotin does not lead to a decrease in the average number of foci detected by the method according to Procedure 2.

### Example 4

### Detection of free DNA ends in fixed HeLa cells according to Procedure 1 to determine the localisation of endogenous, spontaneous DNA breaks in relation to the localisation of the repair foci formed by accumulated molecules of the repair protein X-ray Repair Cross Complementing Protein 1 CXRCC1).

To confirm that the method according to Procedure 1 of the present invention can detect specific types of breaks, that is single-strand gaps, the spontaneous DNA breaks were detected inside cell nuclei using the method described in Procedure 1. They were localised in relation to the localisation of the repair foci formed by accumulated molecules of the repair protein X-ray Repair Cross Complementing Protein 1 (XRCC1). This protein is known to be involved in single-stranded DNA damage repair pathways (single-strand break repair, abbreviated SSBR, or base excision repair, abbreviated BER).

In this experiment HeLa 21-4 cell line (obtained from prof. P.R. Cook, Oxford University) was used. Cells were seeded on 18-mm coverslips (number of cells: 3.5 × 10⁴ per 1 coverslip) and cultured for 24 hours in DMEM supplemented with 10% FBS and then transfected with plasmid pmRFP-C1-XRCC1 (transfection agent: FuGene (Promega, E2311), transfection mix prepared in OptiMEM (Gibco, Thermo Fisher Scientific, 31985070), cells cultured in OptiMEM supplemented with 10% FBS). Cells were cultured for 36 hours after transfection. Subsequently, cells were incubated with 4% PFA (Electron Microscopy Sciences, 15710-S) for 15 minutes at room temperature and permeabilised with 0,25% Triton X-100 (Sigma, T8787) for 1 hour at room temperature. BrdU was linked to free ends of DNA using TdT enzyme using APO-BRDU kit (Phoenix Flow Systems, AU: 1001). The cells were then incubated with 5% BSA solution in PBS overnight in 4°C. The incubation with primary antibodies type 1 and 2 according to step 7 of Procedure 1 were performed using mouse monoclonal antibody against BrdU (dilution: 1:100 in 5% BSA) (Abcam, ab8039) and rabbit polyclonal antibody against BrdU (dilution: 1:100 in 5% BSA) (Abcam, ab152095), respectively. After the last step of washing, cells were treated according to step 8 (PLA procedure) using Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 5% BSA. Samples were washed using Duolink^{®} In Situ Wash Buffers (Sigma, DUO82049).The fluorescence signal was generated using Duolink^{®} In Situ Detection Reagents Green (Sigma, DUO92014). Enzymatic reactions were prepared in ultrapure, RNAse/DNAse free, distilled water (Invitrogen (Thermo Fisher Scientific), 10977-035). After the treatment the samples were imaged and analysed using a Leica TCS SP5 confocal microscope (for detection of BrdU bound to the free ends of DNA: excitation: 488 nm, emission: 498 - 540 nm; for detection of XRCC1: excitation: 561 nm, emission: 600 - 700 nm). 3D stack images were deconvolved using SVI 3D Huygens Deconvolution & Analysis Software (Scientific Volume Imaging B.V., Hilversum, Netherlands).

### Results:

Incorporation of BrdU molecules marks specific types of DNA 3'-OH ends recognised by TdT enzyme, which are present when double-strand blunt-end breaks or double-strand 3'-protruding breaks or single-strand gaps occur. The gaps occur during the BER pathway. Therefore, the regions, where BrdU is detected alongside with an adjacent XRCC1 focus, are most probably only the sites where single-strand gaps occur. ImageJ Software (Abràmoff M.D., Magalhães P.J., Ram S.J.: Image processing with imageJ. (Biophotonics Int., 2004; 11: 36-41) was used to analyse the images and to find and assign the fluorescence maxima of the BrdU foci. The analyses of the fluorescence profiles of the images representing the repair protein foci and localising the above-mentioned maxima in the profiles shows that DNA breaks are consistently detected adjacent to or in the peripheral regions of XRCC1 repair foci (Fig. 5).

### Conclusion:

The results confirm the specificity of the method according to Procedure 1 showing that the signal is obtained only if a DNA break occurs what is confirmed by the accumulation of the repair factor. XRCC1, according to the literature (Caldecott K.W.: XRCC1 and DNA strand break repair. DNA Repair (Amst)., 2003; 2: 955-969; Hanssen-Bauer A., Solvang-Garten K., Akbari M., Otterlei M.: X-ray Repair Cross Complementing protein 1 in base excision repair. Int. J. Mol. Sci., 2012; 13: 17210-17229, Abbotts R., Wilson D.M. 3rd: Coordination of DNA single strand break repair. Free Radic. Biol. Med., 2017; 107: 228-244), specifically accumulates at the sites of DNA damage, in response to the occurrence of single-strand DNA breaks, including those that require the activity of the proteins involved in base excision repair, when single-strand gaps occur. Simultaneous detection of accumulation of this repair protein at the sites where BrdU molecules are incorporated provides confirmation that the signal detected from BrdU is not an artefact and the method according to Procedure 1 results in recognition of free DNA ends. Moreover, the results of this experiment show that the method according to Procedure 1 is applicable in the analysis of the DNA breaks localisation *in situ* in cell nuclei.

### Example 5

### Detection of free DNA ends in fixed HeLa cells according to Procedure 1 to determine the localisation of endogenous, spontaneous DNA breaks in relation to the localisation of the repair foci formed by accumulated molecules of the repair protein X-ray Repair Cross Complementing Protein 1 (XRCC1) and in relation to the localisation of the regions of DNA replication.

To confirm that the method according to Procedure 1 of the present invention can be used in detection of specific types of breaks that occur within DNA replication regions, with minimised unspecific detection signal, endogenous, spontaneous DNA breaks were localised in relation to the localisation of the repair foci formed by accumulated molecules of the repair protein X-ray Repair Cross Complementing Protein 1 (XRCC1) and in relation to the localisation of the regions of DNA replication.

The experiment was performed as in Example 4, using the same HeLa cell line. In order to label replication forks the live cells were incubated with the precursor EdU (Click-iT EdU Alexa Fluor 488 Imaging Kit, Invitrogen, Thermo Fisher Scientific, C10337) for 30 minutes at 37°C according to the manufacturer's instructions prior to the fixation with 4% PFA. After detection of PLA probes regions of incorporation of EdU were detected using a "click" reaction (Click-iT EdU Alexa Fluor 488 Imaging Kit, Invitrogen, Thermo Fisher Scientific, C10337) according to the manufacturer's instructions, except for Alexa Fluor 488 azide reagent provided in the kit but replaced with Atto 390 azide (Sigma, 68321). After the treatment the samples were imaged and analysed using a Leica TCS SP5 confocal microscope (for detection of EdU incorporated in the regions of replicating DNA: excitation: 405 nm, emission: 420 - 480 nm; for detection of BrdU bound to the free ends of DNA: excitation: 488 nm, emission: 498 - 540 nm; for detection of XRCC1: excitation: 561 nm, emission: 600 - 700 nm). 3D stack images were deconvolved using SVI 3D Huygens Deconvolution & Analysis Software (Scientific Volume Imaging B.V., Hilversum, Netherlands).

### Results:

DNA breaks that were modified by incorporation of BrdU molecules, which are most probably single-strand gaps (as described and explained in Example 4), are positioned within replication sites and at the same time adjacent to or in the peripheral regions of XRCC1 repair foci (Fig. 6). The analysis of colocalisation and the analysis of fluorescence profiles and localisation of fluorescence maxima of BrdU foci and regions of DNA replication (the regions of incorporation of EdU) confirm spatial association between the three types of subnuclear objects. Furthermore, it should be highlighted that not every region of DNA replication is associated with the region of BrdU detection.

### Conclusion:

The results show that the method according to Procedure 1 is applicable in the field of research related to replication stress. This subject is relevant in the context of cellular senescence and the processes of ageing. Moreover, the fact that there is no distinct association between localisation of the regions of incorporation of EdU and the regions of incorporation of BrdU indicates that there is no cross-reactivity of detection in the method according to Procedure 1 that would lead to non-specific recognition of another nucleoside analogue used in this experiment, which is EdU. This proves the specificity of the recognition of modified DNA ends.

### Example 6

### DNA ends detection in fixed HeLa cells subjected to different damaging agents (UV-induced DNA damage, H₂O₂-induced DNA damage, DNA breaks induced with a camptothecin derivative - topotecan (Tpt)) using the method according to Procedure 1.

The aim of the experiment was to image and compare the fluorescent signal obtained after incorporation of BrdU to free DNA ends using the method according to Procedure 1 in untreated HeLa cells and in cells in which DNA was subjected to different damaging agents (UV-induced DNA damage, H₂O₂-induced DNA damage, DNA breaks induced with a camptothecin derivative - topotecan (Tpt)).

HeLa 21-4 cells (obtained from prof. P.R. Cook, Oxford University) were seeded on 18-mm coverslips (number of cells: 0.12 × 10⁶ per 1 coverslip) and cultured for 24 hours in DMEM supplemented with 10% FBS.

Subsequently, HeLa cells were subjected to different damaging agents (UV-induced DNA damage, H₂O₂-induced DNA damage, DNA breaks induced with a camptothecin derivative - topotecan (Tpt)), in the following manner:

### UV:

DMEM supplemented with 10% FBS was replaced with pre-warmed PBS (37°C) supplemented with Mg²⁺ and Ca²⁺ ions, the samples were placed under Philips TUV PL-S 5 W/2P lamp, emitting at 254 nm, placed in a standard cell culture incubator (without CO₂ control). The lamp delivered 10 W/m²s, measured 20 cm from the lamp. Cells were exposed to UV-C for 1 minute. Then the samples were placed on ice and immediately fixed with 4% PFA.

H₂O₂:

H₂O₂ (final concentration was 4 mM) was added directly to the growth medium (DMEM supplemented with 10% FBS). The cells were incubated at 37°C for 30 minutes in the standard cell culture incubator with CO₂ level control. After incubation the samples were instantly fixed in 4% PFA.

### Tpt:

The Tpt (Sigma-Aldrich, T2705) was added directly to the growth medium at the final concentration of 20 µM. The cells were incubated at 37°C for 30 minutes in the standard cell culture incubator with CO₂ level control. After incubation the samples were instantly fixed in 4% PFA.

Further part of the experiment was performed as in Example 4, using the same HeLa cell line, except the fact that cells were not transfected with any plasmid. Prior to the imaging DNA in cells was stained with DAPI (4',6-diamidino-2-phenylindole, 1 µM in PBS, 30 min, RT) (Sigma-Aldrich, D9542). After the treatment the samples were imaged and analysed using a Leica TCS SP5 confocal microscope (for detection of DAPI: excitation: 405 nm, emission: 420 - 480 nm; for detection of BrdU bound to the free ends of DNA: excitation: 488 nm, emission: 498 - 540 nm).

### Results:

The examples of recorded images of nuclei of HeLa cells presented in Fig. 7 show that there is significantly more signal detected in cells in which DNA damage was induced using exogenous agents than in control, normal, untreated cells. The average number of detected endogenous BrdU foci is 0.4 ± 0.1 (according to the Example 1). The treatment with different agents leads to the increase of the average number of detectable regions of incorporation of BrdU molecules, in other words of free ends of DNA (the average number in cells treated with H₂O₂: 31.57 ± 8.61, UV: 10.73 ± 4.89, Tpt: 16.39 ± 4.92) (Fig. 7). Additionally, an image of an example of an apoptotic cell in normal, untreated cell culture, shows that in such cells there are many more detectable regions of incorporation of BrdU. Their number (more than 100) reflects the degree of DNA fragmentation that occurs during apoptosis. Wild type, untreated HeLa cells that show signs of heavy DNA damage (apoptotic cells) normally constitute less than 5% of the entire cell population. In the population of cells treated with different damaging agents their percentage increased up to approximately 30% among cells treated with H₂O₂, approximately 20% among cells treated with UV light and approximately 18% among cells treated with Tpt.

### Conclusion:

The method according to Procedure 1 is applicable in detection of exogenously induced DNA damage *in situ* in cells. It enables localisation of DNA breaks what is presented by further application of image analysis technique. The localisation of fluorescence maxima and the analysis of fluorescence profiles can provide the information regarding localisation of double-strand blunt-end breaks or double-strand 3'-protruding breaks or single-strand gaps in fixed cell nuclei in relation to the intra-nuclear and chromatin structure (stained using DAPI) (An example of such analyses shown in Fig. 7C). Moreover, the image presented in Fig. 7A proves that the method according to Procedure 1 can also be used for detection of apoptosis. The presented cell shows signs of morphological features characteristic for apoptotic cells (see the transmitted light image). Besides, the presented images prove that the fluorescent signal obtained as a result of the application of this method is the signal specifically representing DNA ends that occur as a result of DNA breaks induction. The comparison between the number of foci detectable in untreated cells and the number of foci detected in damaged cells proves that the obtained signal is not an artefactual effect of the technical shortcomings of the labelling technique. Moreover, the results prove that the method according to Procedure 1 can be followed by thorough quantitative analysis of the obtained microscopy images and can give a lot of information about the condition and the quality of the analysed biological material. Furthermore, such analysis can deliver information about the environmental conditions to which the biological material is subjected. The data can be used in comparative analysis.

### Example 7

### Detection of the signal in untreated fixed HeLa cells treated according to Procedure 1 in which the entire step 5 of Procedure 1 (incorporation of BrdU with TdT enzyme) was omitted.

The experiment was performed as in Example 6, using the same HeLa cell line, except cells were not subjected to any damaging factors and the entire step 5 of Procedure 1 was omitted. After the treatment the control samples were imaged and analysed using a Leica TCS SP5 confocal microscope (excitation: 488 nm, emission: 498 - 540 nm).

### Results:

Very few foci were detected on the entire coverslip. The foci were defined as the regions where the level of fluorescent signal was more than 0 a.u. and their localisation was determined using a built-in feature of the ImageJ Software that localises fluorescence maxima. The majority of the foci were localised outside of cell nuclei what was determined based on the overlay of fluorescence maxima with the fluorescent images of cell nuclei stained with DAPI (Fig. 8). The analysis of the number of detected foci and the number of observed nuclei shows that on average there are only 0.13 ± 0.04 (total number of nuclei N = 63) foci detected per a single cell nucleus. In the case of untreated HeLa cells, in which free ends of DNA were modified by incorporation of BrdU, the average number of foci detected per a single nucleus was 0.4 ± 0.1 (N=49), as described in Example 1.

### Conclusion and interpretation:

There is very little non-specific antibody recognition in the method according to Procedure 1. The very high specificity of the method according to Procedure 1 results from the fact that the signal can only be obtained if the monoclonal and polyclonal antibodies are bound in close proximity to each other. In other words: if they are bound to the same BrdU molecule or if they are bound selectively to BrdU molecules that form chains attached to DNA free 3'-OH ends. The use of two different types of primary antibodies increases the specificity. With no presence of BrdU, very little signal is detectable, what supports the notion that there is very little cross-reactivity of the applied method of detection.

### Example 8

### Detection of DNA breaks in fixed HeLa cells induced by a photodynamic effect using the method according to Procedure 1.

In this experiment the DNA breaks were induced by treating live cells (HeLa 21-4 cells, obtained from prof. P.R. Cook, Oxford University) with ethidium bromide (500 µM) for 10 min and then parking the laser beam (488 nm) at a chosen place inside the nucleus (dose: 9 mJ) 2 minutes after illumination step 2 of Procedure 1 was performed. The aim of the experiment was to directly detect DNA break (s) inflicted at a specific area of the cell nucleus by a photodynamic effect.

HeLa 21-4 cells were first seeded on 18-mm coverslips and cultured for 3 days in DMEM supplemented with 10% FBS. After culturing, DNA damage was induced in chosen cells, as described previously, followed by incubation in 70% ethanol in water, 15 h, -20°C. After that, increasing of accessibility of DNA ends (step 4) was performed with 10 mM EDTA in water, 1 h, RT. Incorporation of modified (BrdUTP) nucleotides (Phoenix Flow Systems) by terminal deoxynucleotidyl transferase was performed according to the kit supplier's instructions (APO-BRDU kit, Phoenix Flow Systems). The cells were then incubated with 1% BSA solution in PBS (overnight in 4°C). The incubation with primary antibodies type 1 and 2 according to step 7 of Procedure 2 were performed using mouse monoclonal anti-BrdU (Abcam, ab8039) and rabbit polyclonal anti-BrdU (Abcam, ab152095) (1:100 in 1% BSA), respectively. After last washing, cells were treated according to step 8 (PLA procedure) using Duolink In Situ PLA PROBE anti-rabbit MINUS (Sigma, DUO92006), Duolink In Situ PLA PROBE anti-mouse PLUS (Sigma, DUO92001), Detection reagents green (Sigma, DUO92014), Duolink In Situ Wash Buffers, DUO82049. After treatment, samples were imaged using a Leica TCS SP5 confocal microscope (excitation: 488 nm, emission: 510-600 nm).

### Results :

Microscopic images were analysed using ImageJ software (Abràmoff M.D., Magalhães P.J., Ram S.J.: Image processing with imageJ. Biophotonics Int., 2004; 11: 36-41). The fluorescence focus is clearly visible exactly at the illuminated place or in its direct proximity in 90% of damaged nuclei (example: Fig. 10, right column).

### Conclusions:

Small number of DNA breaks induced by interaction of light and a DNA intercalator (photosensitizer) is possible to detect by the method according to Procedure 1.

### Comparative example 3

### Detection of DNA breaks in fixed HeLa cells induced by a photodynamic effect using the standard TUNEL assay.

HeLa 21-4 cells were seeded, cultured and treated as in Example 6. For further treatment, the standard TUNEL assay (APO BrdU Phoenix Flow Systems) was performed according to the manufacturer's instruction. After the treatment the samples were imaged and analysed using a Leica TCS SP5 confocal microscope (excitation: 488 nm, emission: 510-600 nm).

The comparative example 3 has been compared with the results of example 9.

### Results:

Microscopic images were analysed using ImageJ software (Abràmoff M.D., Magalhães P.J., Ram S.J.: Image processing with imageJ. Biophotonics Int., 2004; 11: 36-41). For standard TUNEL assay, at the illuminated place, no focus was visible. For the method according to Procedure 1, the focus was clearly visible exactly at the illuminated place or in its direct proximity (Fig. 9).

### Conclusion:

The method according to Procedure 1 is more sensitive than standard TUNEL assay and allows detection of a low number of DNA double strand breaks induced by interaction between light and a photosensitizer. Similar number of damage is not detected by standard TUNEL assay.

### Example 9

### The use of Procedure 1 and standard TUNEL assay for detection of DNA ends produced as a result of cleavage activity of nuclease SpCas9 in fixed U2-OS cells

To determine applicability of Procedure 1 for detection of DNA ends occurring as a result of the cleavage activity of nuclease SpCas9, CRISPR/Cas9 technique was used. The nuclease was targeted against the subtelomeric region located on chromosome 3 that is characterised by the presence of repetitive sequences. SpCas9 was used to induce more than a few (up to several dozens) cleavages.

In this Example, before the detection, U2-OS cells (HTB-96^{™}, ATCC) were seeded on glass bottom 35 mm petri dishes with 14 mm microwells (No. 1.0 coverglass, thickness: 0.13 - 0.16 mm) (MatTek Corporation, P35G-1.0-14-C) (3.5 × 10⁴ cells per 1 petri dish) and cultured for 24 hours in DMEM supplemented with 10% FBS. After 24 hours, to obtain the conditions suitable to image Cas9 foci at the specific sites inside a cell nucleus (CRISPR/Cas9 technique), cells were transfected with plasmids encoding fluorescently labeled (3X mCherry) nuclease SpCas9 and suitable combinations of sgRNA (transfection agent: FuGene (Promega, E2311), transfection mix prepared with OptiMEM (Gibco, Thermo Fisher Scientific, 31985070), cells cultured in OptiMEM supplemented with 10% FBS) and cells were cultured in OptiMEM supplemented with 10% FBS). After transfection cells were cultured for 48 hours. For cells presented in the Fig. 10, treated according to Procedure 1, there was no pre-preparation with Tris (step 1). Next, Step 2 comprised incubation with 1% PFA (Electron Microscopy Sciences, 15710-S) for 15 minutes at room temperature and permeabilisation with 0,25% Triton X-100 (Sigma, T8787) for 1 hour at room temperature. After permeabilisation, BrdU was linked to free ends of DNA with TdT enzyme using APO-BRDU kit (Phoenix Flow Systems, AU: 1001). Incubation with the enzyme was performed on a droplet in a humid chamber. After the enzymatic reaction (performed according to the supplier's instruction), samples were quickly rinsed twice with Rinse Buffer (Phoenix Flow Systems, AU: 1001). Afterwards, samples were incubated in 5% BSA solution in PBS overnight in 4°C and subsequently the incubation with primary antibodies was performed [mouse monoclonal anti-BrdU (dilution: 1:100 in 5% BSA) (Abcam, ab8039), rabbit polyclonal anti-BrdU (dilution: 1:100 in 5% BSA) (Abcam, ab152095)]. For further treatment, following reagents were used: PLA Probes: Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 5% BSA; PLA Probes were diluted in 5% BSA.. PLA Detection: samples were washed using Duolink^{®} In Situ Wash Buffers (Sigma, DUO82049).The fluorescence signal was generated using Duolink^{®} In Situ Detection Reagents Red (Sigma, DUO92008). Enzymatic reactions were prepared in ultrapure, RNAse/DNAse free, distilled water (Invitrogen (Thermo Fisher Scientific), 10977-035).

For TUNEL assay cells were seeded and transfected as mentioned-above. After transfection cells presented in Fig. 10 were also cultured for 48 hours and ssubsequently fixed with 1% PFA (Electron Microscopy Sciences, 15710-S) for 15 minutes at room temperature and permeabilised with 0,25% Triton X-100 (Sigma, T8787) for 1 hour at room temperature. BrdU linking to free ends of DNA was also carried out in the same manner. Afterwards, cells were incubated with a primary antibody [mouse monoclonal anti-BrdU (dilution: 1:100 in 5% BSA) (Abcam, ab8039)] for 1 hour, at room temperature, on a droplet (50 µl) in a humid chamber. After the incubation with the primary antibody, the samples were washed in PBS (the buffer was replaced four times, the samples were kept in PBS for 1 hour) and the incubation with a secondary antibody was performed [goat anti-Mouse IgG1 secondary antibody, Alexa Fluor^{®} 594 conjugate (dilution: 1:400 in 5% BSA) (Invitrogen, Thermo Fisher Scientific, A21125)] - 1 hour, at room temperature, on a droplet (50 µl) in a humid chamber. Next, samples were washed in PBS (3 × 5 min.) and left in PBS overnight.

After the treatment, for all samples, phase-contrast and fluorescence microscopy imaging was performed with a Leica DM-IRB inverted microscope equipped with a mercury arc lamp, a 10-position filter wheel (Sutter Instrument), CFP/YFP/HcRed filter set, GFP/DsRed filter set (Semrock), a CCD camera (Photometries), and MetaMorph acquisition software (Molecular Devices). Red labels (Alexa Fluor^{®} 594 and labels used in Duolink^{®} In Situ Detection Reagents Red (Sigma, DUO92008)) were excited at 556/20 nm (wavelength/bandwidth), and its emission was collected in a 630/91-nm channel. GFP was excited at 470/28 nm, and its emission was collected in a 512/23-nm channel Imaging data were acquired and analyzed with MetaMorph acquisition software (Molecular Devices) and with ImageJ Software (Abràmoff M.D., Magalhães P.J., Ram S.J.: Image processing with ImageJ . (Biophotonics Int., 2004; 11: 36-41). Thresholds were set based on the ratios of nuclear focal signals to background nucleoplasmic fluorescence.

### Results:

The images and the profiles of fluorescence intensity presented in Fig. 10 show that no signal was detected when the standard TUNEL assay was applied in detection of free DNA ends generated using CRISPR/Cas9 technique, where several dozens of cleavages were induced at one site in the human genome. The average value of the fluorescence intensity that was measured in the regions associated with the accumulation of nuclease SpCas9 and emitted by Alexa Fluor^{®} 594 conjugated with the secondary antibodies used in standard immunofluorescent detection of BrdU was approximately 0 (50 nuclei were analysed and the value was normalised based on the level of the background signal, representing non-specifically bound antibody molecules). The example of the fluorescence profiles measured for the representative example of recorded microscopy images distinctively illustrate that no BrdU foci could be detected. By contrast, when the method according to Procedure 1 was applied no non-specific background signal was detected and the regions of incorporation of BrdU were represented by distinct foci visualised in the microscopy images (Fig. 10). These foci colocalised with the regions of accumulation of SpCas9. These observations are additionally confirmed by the representative fluorescence intensity profiles obtained using ImageJ software (Fig. 10).

### Conclusion:

The results confirm that the method according to Procedure 1 is capable of detecting DNA cleavages induced by SpCas9 in human cells, unlike the standard TUNEL assay that is not even sensitive enough to recognise several dozens of double-strand DNA breaks located in close proximity to each other at the specific site in human genome. The results of the image analysis provide another proof for specificity and sensitivity of the method according to Procedure 1 and accentuate its advantage over the existing methods that have been used in detection of DNA breaks so far.

### Example 10

### The use of Procedure 1 for highly sensitive detection of DNA ends produced as a result of cleavage activity of nuclease SpCas9 in fixed U2-OS cells

To determine applicability of Procedure 1 for detection of DNA ends resulting from the cleavage activity of nuclease SpCas9, CRISPR/Cas9 technique was used. The nuclease was targeted against the subtelomeric region located on chromosome 3 that is characterised by the presence of repetitive sequences. For this purpose, U2-OS cells (HTB-96^{™}, ATCC) were prepared in two different manners to obtain the following:
- the cells in which SpCas9 did not induce any cuts, despite the fact that nuclease molecules bound in the targeted region
- the cells in which SpCas9 induced only one, single double-strand cut (break) at the site located in the genome sequence localised in the close proximity to the region of repetitive sequences.

In this Example, before the detection, U2-OS cells were seeded on 18-mm coverslips (number of cells: 3.5 × 10⁴ per 1 coverslip) and cultured for 24 hours in DMEM supplemented with 10% FBS. After 24 hours, to generate the above-mentioned "cell types" and to create the conditions suitable to image SpCas9 foci at the specific sites inside a cell nucleus (CRISPR/Cas9 technique), cells were transfected with plasmids encoding fluorescently labeled (3X mCherry) SpCas9 and suitable combinations of sgRNA (transfection agent: FuGene (Promega, E2311), transfection mix prepared with OptiMEM (Gibco, Thermo Fisher Scientific, 31985070), cells cultured in OptiMEM supplemented with 10%FBS). After transfection cells were cultured for 48 hours and subsequently treated according to Procedure 1. For cells presented in Fig. 11, there was no pre-preparation with Tris (step 1). Step 2 comprised only fixation with 70% Et(OH) (overnight; -20°C). After the incubation of the samples in 10 mM EDTA solution in water for 30 minutes at room temperature, the samples were only quickly rinsed twice with Wash Buffer (Phoenix Flow Systems, AU: 1001). BrdU was linked to free ends of DNA with TdT enzyme using APO-BRDU kit (Phoenix Flow Systems, AU: 1001). Incubation with the enzyme was performed on a droplet in a humid chamber. After the enzymatic reaction (performed according to the supplier's instruction), samples were quickly rinsed twice with Rinse Buffer (Phoenix Flow Systems, AU: 1001). Afterwards, samples were incubated in 1% BSA solution in PBS overnight in 4°C and subsequently the incubation with primary antibodies was performed [mouse monoclonal antibody against BrdU (dilution: 1:100 in 1% BSA) (Abcam, ab8039), rabbit polyclonal antibody against- BrdU (dilution: 1:100 in 1% BSA) (Abcam, ab 152095)]. For further treatment, the following reagents were used: PLA Probes: Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 1% BSA. PLA Detection: samples were washed using Duolink^{®} In Situ Wash Buffers (Sigma, DUO82049). The fluorescent signal was generated using Duolink^{®} In Situ Detection Reagents Green (Sigma, DUO92014). Enzymatic reactions were prepared in ultrapure, RNAse/DNAse free, distilled water (Invitrogen, Thermo Fisher Scientific, 10977-035). After the treatment the samples were imaged and analysed using a Leica TCS SP5 confocal microscope (for detection of BrdU bound to the free ends of DNA: excitation: 488 nm, emission: 498 - 540 nm; for detection of SpCas9: excitation: 594 nm, emission: 605 - 700 nm).

### Results:

The images presented in Fig. 11 show that if even just one DNA double-strand break occurs as a result of SpCas9 cleavage activity, it can be specifically recognised by the method according to Procedure 1. It is reflected by the occurrence of associated fluorescent signals characteristic for accumulated SpCas9 molecules and incorporated BrdU molecules. When SpCas9 does not perform any cleavage activity, no incorporation of BrdU can be detected in the area of accumulation of the nuclease (Fig. 11). The images in Fig. 11. show that when only one, single double-strand DNA break is induced, the localisation of BrdU detection region is in the area colocalising with the SpCas9 focus (Fig. 11). In some cases the BrdU foci can be detected in the regions adjacent to the SpCas9 foci (data not shown). Most probably it is caused by the fact, that the treatment with EDTA that causes the chromatin loosening leads to the transposition of two complimentary (3' and 5') DNA ends that occur and are freed when DNA is cleaved. The two non-stabilised DNA fragments that are created at the site of the lesion are separated and moved away from each other.

### Conclusion:

The results confirm that the method according to Procedure 1 is capable of detecting DNA cleavages induced by SpCas9 in human cells. Furthermore, it can be used as a highly specific marker in detection of SpCas9-incuced DSBs, regardless of the number of cleavages since it has such high sensitivity that it is able to detect single lesions. The fact that there is no detectable signal in the case where no cleavages are induced confirms high specificity of the method. The results also confirm very high sensitivity of the technique: it gives possibility to detect one double-strand DNA break.

### Example 11

### The use of Procedure 2 for detection of DNA ends produced as a result of nicking activity of nickase SpCas9n (H840A) in fixed U2-OS cells

To determine applicability of Procedure 2 for detection of DNA ends resulting from the nicking activity of nickase SpCas9n (H840A), CRISPR/Cas9 technique was used. The nickase was targeted against the subtelomeric region located on chromosome 3 that is characterised by the presence of repetitive sequences. For this purpose, U2-OS cells (HTB-96^{™}, ATCC) were prepared in three different manners to obtain the following:
- the cells in which SpCas9n induced more than a few (up to several dozens) single-strand nicks (the nicks were induced in the same DNA strand),
- the cells in which SpCas9n did not induce any nicks, despite the fact that SpCas9n molecules bound in the targeted region,
- the cells in which SpCas9n induced only one, single single-strand nick at the site located in the genome sequence localised in the close proximity to the region of repetitive sequences.

In this Experiment, before the detection, U2-OS cells were seeded on 18-mm coverslips (number of cells: 3.5 × 10⁴ per 1 coverslip) and cultured for 24 hours in DMEM supplemented with 10% FBS. After 24 hours, to generate the above-mentioned "cell types" and to create the conditions suitable to image SpCas9n foci at the specific sites inside a cell nucleus (CRISPR/Cas9 technique), cells were transfected with plasmids encoding fluorescently labeled (3X GFP) SpCas9n (H840A) and suitable combinations of sgRNA (transfection agent: FuGene (Promega, E2311), transfection mix prepared with OptiMEM (Gibco, Thermo Fisher Scientific, 31985070), cells cultured in OptiMEM supplemented with 10%FBS). Then, cells were cultured for 48 hours after transfection. Subsequently, cells were treated according to Procedure 2. In the case of cells presented in the images in Fig. 12, there was no treatment with Tris. Next stepcomprised fixation with 70% Et(OH) (overnight incubation; -20°C). Thereafter, the samples were pre-blocked using Endogenous Biotin-Blocking Kit (Invitrogen, Thermo Fisher Scientific, E21390) according to the description provided in Procedure 2. After the incubation of the samples in 10 mM EDTA solution in water for 30 minutes at room temperature, the samples were quickly rinsed with distilled water and incubated with the nick translation assay reaction mixture [1x NEBuffer2 (NEBiolabs, B7002S), 30 uM of each dNTP (Jena Bioscience, N6-(6-Amino)hexyl-2'-deoxyadenosine-5'-triphosphate - Biotin (Biotin-7-dATP): NU-835-BIO-S, Biotin-16-Propargylamino-dCTP: NU-809-BIO16, biotin-16-5-aminoallyl-dUTP: NU-803-BIO16, dGTP: NU-1003) 3 units of DNA Polymerase I (*E.Coli*) (NEBiolabs, MO209) per coverslip, Ultra Pure Distilled Water (Invitrogen, Thermo Fisher Scientific, 10977-035)] for 1 h incubation in 37C in a humid chamber. Afterwards, samples were incubated in 1% BSA solution in PBS overnight in 4°C. Next, samples were incubated in 1% BSA solution in PBS overnight in 4°C and subsequently the incubation with primary antibodies was performed [mouse monoclonal antibiotin [Hyb-8] (dilution: 1:100 in 1% BSA) (Abcam, ab201341), rabbit polyclonal anti-biotin (dilution: 1:100 in 1% BSA) (Abcam, ab53494)]. For further treatment, the following reagents were used: PLA Probes: Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 5% BSA. PLA Detection: samples were washed using Duolink^{®} In Situ Wash Buffers (Sigma, DUO82049). The fluorescence signal was generated using Duolink^{®} In Situ Detection Reagents Red (Sigma, DUO92008). Enzymatic reactions were prepared in ultrapure, RNAse/DNAse free, distilled water (Invitrogen (Thermo Fisher Scientific), 10977-035). After the treatment the samples were imaged and analysed using a Leica TCS SP5 confocal microscope (for detection of SpCas9n: excitation: 488 nm, emission: 498 - 540 nm; for detection of biotinylated or unmodified nucleotides bound to the free ends of DNA: excitation: 594 nm, emission: 605 - 700 nm).

### Results:

The images presented in Fig. 12 show that if one or more single-strand DNA breaks occur as a result of SpCas9n (H840A) nicking activity, they are specifically recognised by DNA Polymerase I and the method according to Procedure 2. It is reflected by the occurrence of associated fluorescent signals characteristic for accumulated fluorescently labeled SpCas9n molecules and incorporated biotinylated nucleotides. When SpCas9n does not perform any cleavage activity, no incorporation of modified nucleotides can be detected in the area collocalising or partially colocalising with the region of accumulation of the nickase (Fig. 12). The images in Fig. 12 show that when only one single-strand DNA break is induced, the localisation of the incorporation of nucleotides colocalises with the SpCas9n focus (Fig. 12). If the number of nicks is higher than one (up to several dozens) the regions of incorporation of nucleotides and SpCas9n foci also colocalise (Fig. 12).

### Conclusion:

The results confirm that the method according to Procedure 2 is capable of detecting DNA nicks induced by SpCas9n in human cells. Furthermore, it can be used as a highly specific marker in detection of SpCas9n-incuced single-strand breaks (SSBs), regardless of the number of breaks. The fact that there is no detectable signal in the case where no SSBs are induced confirms high specificity of the method. The results also confirm very high sensitivity of super TUNEL technique: it provides a possibility of detection of up to only one single-strand DNA break.

### Example 12

### The use of Procedure 1 for detection of DNA breaks in cells in suspension with subsequent isolation of chromatin.

In this Example, HeLa 21-4 cells (obtained from P.R. Cook, University of Oxford) were seeded on 6-well plates and cultured for 48 hours in DMEM supplemented with 10% FBS until the culture reached 100% confluency (number of cells: 1.2 × 10⁶ per 1 well). Next, cells were treated with the DNA damaging agent H₂O₂ which was added directly to the growth medium (final concentration: 4 mM). The cells were incubated at 37°C for 30 minutes in the standard cell culture incubator with CO₂ level control. The control, untreated cells and the damaged cells (H₂O₂) were then harvested using 0.5 ml trypsin and transferred to 1.5 ml eppendorf tubes. Afterwards, trypsin was inactivated by adding 0.5 ml DMEM supplemented with 10% FBS. Subsequently, cells were spun down in the table top centrifuge (Eppendorf centrifuge, 5417C, rotor: F45-30-11) (1700 rpm, 5 minutes, room temperature). The cell pellet was resuspended in PBS and again spun down (this washing step was repeated twice). When the supernatant PBS was discarded, cells were resuspended in the remaining droplet of PBS. Subsequently, ice-cold 70% Et(OH) was added dropwise, and cells were incubated in Et(OH) for 30 minutes in -20°C. Then, the cells were treated according to Procedure 1, wherein steps 1, 3, 4, 9 were omitted, and every washing step and every incubation were followed by centrifugation. BrdU was linked to free ends of DNA using TdT enzyme using APO-BRDU kit (Phoenix Flow Systems, AU: 1001) according to the supplier's instructions. Next, the cells were blocked for 30 minutes in 1% BSA solution in PBS and subsequently the incubation with primary antibodies was performed [mouse monoclonal antibody against BrdU (dilution: 1:100 in 1% BSA) (Abcam, ab8039), rabbit polyclonal antibody against BrdU (dilution: 1:100 in 1% BSA) (Abcam, ab152095)]. For further treatment, the following reagents were used: PLA Probes: Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 5% BSA PLA Detection: samples were washed using Duolink^{®} In Situ Wash Buffers (Sigma, DUO82049). The fluorescence signal was generated using Duolink^{®} In Situ Detection Reagents Green (Sigma, DUO92014). Enzymatic reactions were prepared in ultrapure, RNAse/DNAse free, distilled water (Invitrogen (Thermo Fisher Scientific), 10977-035). After the labelling the cell pellet was resuspended in 50 µl 10 mM Tris (pH 8.8) and spun down in the table top centrifuge (1 minute, 14000 rpm, room temperature). The cells were resuspended in 10 µl of TE Buffer (10mM Tris-HCl containing 1mM EDTA•Na₂) and boiled for 15 minutes (99°C). The samples were cooled on ice and spun down in the table top centrifuge (1 minute, full speed: 14000 rpm, room temperature). The entire supernatant was collected, diluted in distilled water to the total volume of 40 µl and used in further measurements.

The level of fluorescence intensity of the cell extract obtained from untreated control cells and the H₂O₂ damaged cells was measured using a fluorescence spectrophotometer (Hitachi F-7000 supplied with Xe lamp). The fluorescence intensity was measured for 30 s (excitation 480 nm, emission 520 nm).

### Results:

Incorporation of BrdU marks specific types of DNA 3'-OH ends recognised by TdT enzyme, which are present when double-strand blunt-end breaks or double-strand 3'-protruding breaks or single-strand gaps occur. The number of free DNA ends increases as a result of the treatment with damaging agents, such as H₂O₂, what results in the increase of the number of incorporated BrdU molecules and the increase of total fluorescence intensity signal in the samples treated according to Procedure 1 (Fig. 13). The measured fluorescence intensity per DNA unit (1 ng) in the control sample was 2.9 × 10⁻⁴ a.u., whereas the fluorescence intensity measured per 1 ng of DNA in the sample treated with H₂O₂ was higher - 3.19 × 10⁻⁴ a.u. (Table 1).

### Conclusion:

The method according to Procedure 1 is applicable to quantitative detection and comparative analysis of DNA damage in cells in suspension followed by extraction of chromatin.

**Table 1**

| | Untreated | H₂O₂ treated |
|---|---|---|
| DNA concentration [ng/µl] | 5.0 | 3.6 |
| volume [µl] | 40 | 40 |
| fluorescence signal [a.u.] | 0.058 ± 0.005 | 0.046 ± 0.005 |
| **flurescence signal per 1 ng of DNA [a.u./ng]** | **2.9 ± 0.2 × 10⁻⁴** | **3.2 ± 0.3 × 10⁻⁴** |

### Example 13

### Use of a combination of Procedures 1 and 2 for detection of DNA breaks in isolated chromatin.

In this Example, HeLa 21-4 cells (obtained from P.R. Cook, University of Oxford) were seeded on 6-well plates and cultured for 48 hours in DMEM supplemented with 10% FBS until the culture reached 100% confluency (number of cells: 1.2 × 10⁶ per 1 well). Next, cells were treated with the DNA damaging agent H₂O₂ which was added directly to the growth medium (final concentration: 4 mM). The cells were incubated at 37°C for 30 minutes in the standard cell culture incubator with CO₂ level control. The control, untreated cells and the damaged cells (H₂O₂) were then harvested using 0.5 ml trypsin and transferred to 1.5 ml Eppendorf tubes. Afterwards, trypsin was inactivated by adding 0.5 ml DMEM supplemented with 10% FBS. Subsequently, cells were spun down in the table top centrifuge (Eppendorf centrifuge, 5417C, rotor: F45-30-11) (1700 rpm, 5 minutes, room temperature). The cell pellet was resuspended in 10 mM Tris (pH 8.8), a small aliquot of the cells in suspension (50 µl) was transferred to a new 1.5 ml Eppendorf tube and spun down in the table top centrifuge (1 minute, 14000 rpm, room temperature). The supernatant was discarded and the washing step in the same volume (50 µl) of 10 mM Tris (pH 8.8) was repeated (centrifugation in the table top centrifuge (1 minute, 14000 rpm, room temperature)). The cells were resuspended in 10 µl of TE Buffer (10mM Tris-HCl containing 1mM EDTA•Na₂) and boiled for 15 minutes (99°C). The samples were cooled on ice and spun down in the table top centrifuge (1 minute, full speed: 14000 rpm, room temperature).

The entire supernatant (containing chromatin extracted from cells) was collected. DNA in the supernatant was precipitated by adding 8 µl of isopropanol, followed by the centrifugation in the table top centrifuge (1 minute, full speed: 14000 rpm, room temperature). DNA was resuspended in 5 µl of 70% Et(OH) and spun down in the table top centrifuge (1 minute, full speed: 14000 rpm, room temperature). The supernatant was discarded. The sample was treated according to Procedure 1, wherein step 1 was a part of the abovementioned steps leading to isolation of the biological material, thus leading to the increase of accessibility of DNA ends. Steps 2, 3 and 4 were omitted. In step 5 the DNA ends were modified by resuspending isolated DNA in a modified reaction mixture containing: DNA Polymerase I (*E.Coli*) (NEBiolabs, MO209), 1x NEBuffer2 (NEBiolabs, B7002S), TdT (Phoenix Flow Systems, AU: 1001) with the reaction buffer (Phoenix Flow Systems, AU: 1001), mixture of dNTPs (Jena Bioscience, N6-(6-Amino)hexyl-2'-deoxyadenosine-5'-triphosphate - Biotin (Biotin-7-dATP): NU-835-BIO-S, Biotin-16-Propargylamino-dCTP: NU-809-BIO16, dGTP: NU-1003) and BrdU (Phoenix Flow Systems, AU: 1001), Ultra Pure Distilled Water (Invitrogen, Thermo Fisher Scientific, 10977-035). The suspension was incubated for 30 minutes in 37°C. The polymerase reaction was terminated using Tris-HCl as described in step 5c of Procedure 2. The suspension was transferred onto pre-incubated with streptavidin, biotin-covered cover glass (Bio-02, MicroSurfaces, Inc.) and incubated for 30 minutes at room temperature in order to immobilize isolated, modified DNA (with incorporated BrdU and biotinylated nucleotides) on the glass coverslip. Next, the immobilized DNA was blocked for 30 minutes in 1% BSA solution in PBS and subsequently the incubation with primary antibodies was performed [mouse monoclonal antibody against BrdU (dilution: 1:100 in 1% BSA) (Abcam, ab8039), rabbit polyclonal antibody against BrdU (dilution: 1:100 in 1% BSA) (Abcam, ab152095)]. For further treatment, the following reagents were used: PLA Probes: Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 5% BSA PLA Detection: samples were washed using Duolink^{®} In Situ Wash Buffers (Sigma, DUO82049). The fluorescence signal was generated using Duolink^{®} In Situ Detection Reagents Green (Sigma, DUO92014). Enzymatic reactions were prepared in ultrapure, RNAse/DNAse free, distilled water (Invitrogen (Thermo Fisher Scientific), 10977-035). After the treatment the level of fluorescence intensity of the cell extract obtained from untreated control cells and the H₂O₂ damaged cells was measured using a Leica TCS SP5 confocal microscope by imaging the fluorescence images of immobilized, modified DNA, immobilized on the cover glass (for detection of BrdU bound to the free ends of DNA: excitation: 488 nm, emission: 498 - 540 nm).

### Results:

The number of free DNA ends increases as a result of the treatment with damaging agents, such as H₂O₂. This results in the increase of the number of incorporated BrdU molecules and the increase of total, integrated fluorescence intensity signal in the samples treated according to the combination of Procedures 1 and 2. For each sample, the mean grey value (fluorescence intensity) was determined using ImageJ software. The measured fluorescence intensity in the control sample was 6.0 ± 0.1 a.u., whereas the fluorescence intensity measured in the sample treated with H₂O₂ was 8.9 ± 0.1 a.u. An unpaired two-sample t-test has shown that the difference in means between control and H₂O₂ treated samples is statistically significant (p-value < 0.001).

### Conclusion:

The method according to the combination of Procedures 1 and 2 is applicable to quantitative detection and comparative analysis of DNA damage in isolated chromatin.

### Example 14

### Use of Procedure 2 for detection of DNA ends in fixed U2-OS cells in which the accessibility of DNA ends was increased prior to fixation.

In this Experiment, before the detection, U2-OS cells were seeded on 18-mm coverslips (number of cells: 3.5 × 10⁴ per 1 coverslip) and cultured in DMEM supplemented with 10% FBS. The cells were cultured for 72 hours. Subsequently, cells were treated according to Procedure 2. Prior to fixation, the cells were incubated with 1 mM Tris for 20 minutes at 37°C. During the incubation the morphology of the cells was monitored. Next step comprised fixation with 70% Et(OH) (overnight incubation; -20°C). Thereafter, the samples were pre-blocked using Endogenous Biotin-Blocking Kit (Invitrogen, Thermo Fisher Scientific, E21390) according to the description provided in Procedure 2. After the incubation of the samples in 10 mM EDTA solution in water for 30 minutes at room temperature, the samples were quickly rinsed with distilled water and incubated with the nick translation assay reaction mixture [1x NEBuffer2 (NEBiolabs, B7002S), 30 uM of each dNTP (Jena Bioscience, N6-(6-Amino)hexyl-2'-deoxyadenosine-5'-triphosphate - Biotin (Biotin-7-dATP): NU-835-BIO-S, Biotin-16-Propargylamino-dCTP: NU-809-BIO16, biotin-16-5-aminoallyl-dUTP: NU-803-BIO16, dGTP: NU-1003) 3 units of DNA Polymerase I (*E.Coli*) (NEBiolabs, MO209) per coverslip, Ultra Pure Distilled Water (Invitrogen, Thermo Fisher Scientific, 10977-035)] for 1 h incubation in 37C in a humid chamber. Afterwards, samples were incubated in 1% BSA solution in PBS, overnight in 4°C. Next, samples were incubated in 1% BSA solution in PBS, overnight in 4°C, and subsequently the incubation with primary antibodies was performed [mouse monoclonal anti-biotin [Hyb-8] (dilution: 1:100 in 1% BSA) (Abcam, ab201341), rabbit polyclonal anti-biotin (dilution: 1:100 in 1% BSA) (Abcam, ab53494)]. For further treatment, the following reagents were used: PLA Probes: Duolink^{®} In Situ PLA^{®} Probe Anti-Mouse MINUS, Affinity purified Donkey anti-Mouse IgG (H+L) (Sigma, DUO82004, kit: DUO92004) and Duolink^{®} In Situ PLA^{®} Probe Anti-Rabbit PLUS, Affinity purified Donkey anti-Rabbit IgG (H+L) (Sigma, DUO82002, kit: DUO92002); PLA Probes were diluted in 5% BSA. PLA Detection: samples were washed using Duolink^{®} In Situ Wash Buffers (Sigma, DUO82049). The fluorescence signal was generated using Duolink^{®} In Situ Detection Reagents Red (Sigma, DUO92008). Enzymatic reactions were prepared in ultrapure, RNAse/DNAse free, distilled water (Invitrogen (Thermo Fisher Scientific), 10977-035). After the treatment the samples were imaged and analysed using a Leica TCS SP5 confocal microscope (excitation: 594 nm, emission: 605 - 700 nm).

### Results:

In the obtained images (Fig. 14) different numbers of fluorescent foci could be detected. The foci were specifically localized inside cell nuclei.

### Conclusion:

The way to increase accessibility of chromatin in live cells can be obtained by incubation of live cells with Tris solution. This step in the procedures could significantly improve the efficiency of the DNA damage detection in cells by significant chromatin loosening. The results confirm that adding this step to Procedure 2 results in reliable data what proves that the treatment of live cells with Tris prior to fixation can be an alternative way to increase the accessibility of DNA ends in a sample.

The examples described above disclose the inventive method. As in another aspect the subject invention also provides the use of rolling circle replication for marking the presence and position of single DNA end(s) as well as the use of the method for detection of DNA end(s) in a biological material, it should be clear for a person skilled in the art that the abovementioned examples are equally representative for the use-related aspect of the invention and they are not going to be described again for brevity of the description.

A person skilled in the art will be also aware that the subject invention is susceptible to numerous modifications, alterations, changes, substitutions and that the features presented in the examples may be combined suitably to particular needs, all of which will be encompassed by the scope of protection as defined in the enclosed claims.

The invention is further defined in the following numbered clauses:
1. Method of detection of DNA end(s) in a biological material, comprising the following steps I - III and at least one of sub-steps a-h of each of steps I - III:
   *I. PREPARATION OF THE MATERIAL*
      a. fixation and/or permeabilization and/or lysis and/or isolation and/or fractionation and/or immobilization of the biological material,
      b. increasing accessibility of DNA end(s),
      c. blocking nonspecific binding site(s) for molecules type 2-6, in the biological material,
   *II. PROCESSING OF DNA END(S)*
      d. modification of DNA end(s) by chemical or physical processing followed by binding molecules type 1 to the DNA end(s) by catalytic or noncatalytic means,
      e. blocking nonspecific binding site(s) for molecules type 2-6 in the biological material,
   *III. RECOGNITION AND DETECTION OF THE MODIFIED DNA END(S)*
      f. incubation of the biological material from step II with at least two molecules type 2 and 3 which bind to the molecules type 1 in a manner that allows steps leading to rolling circle amplification (RCA) reactions,
      g. detection of DNA end(s) by:
         i. optionally contacting suitable molecules type 4 and/or 5 with molecules type 2 and 3, wherein the molecules type 4 and/or 5 are conjugated with the oligonucleotides type 1,
         ii. adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2,
         iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
      h. detection of molecules type 6,
      wherein when more than one sub-step a - c of step I is performed then they may occur in any order.
2. Method according to clause 1, wherein DNA end(s) are the result of any DNA break(s) including single-strand break or double-strand break.
3. Method according to clause 2, wherein DNA break(s) are selected from the group comprising single-strand nick, single-strand gap, single-strand break of single phosphodiester bond, double-strand blunt-end break, double-strand 3'-protruding break, double-strand 5'-protruding break, types of DNA strand breaks including these where 3'-OH or 5'-phosphate DNA ends are not present.
4. Method according to clause 1, wherein detection of DNA end(s) is performed *in situ.*
5. Method according to clause 1, wherein the biological material is live.
6. Method according to clause 1, wherein the biological material is fixed.
7. Method according to clause 5 or 6, wherein the biological material is selected from the group comprising animal, plant, protozoan, bacterial cells, viruses, tissues and fragments and/or components thereof.
8. Method according to clause 5 or 6, wherein the biological material is cell or tissue or fragments thereof surrounded by a membrane.
9. Method according to clause 8, wherein sub-step c is performed in step I.
10. Method according to clause 5, wherein before step I sub-step a, an additional step of increasing accessibility of DNA end(s) is performed.
11. Method according to clause 1, wherein the biological material is present in a solution or on a porous surface or solid support.
12. Method according to clause 11, wherein solid supports or types of a porous surface are selected from the group comprising glass, plastic, water gels, aero gels metals and ceramics.
13. Method according to clause 1, wherein molecule type 1 is selected from the group comprising halogenated nucleotide or nucleoside molecules such as BrdU, IdU, CldU, or DNA precursor analogs such as EdU (5-Ethynyl-2'-deoxyuridine), F-ara-EdU, 5-Ethynyl-2'-deoxycytidine, or biotinylated nucleotide molecules, ADP-ribose molecules, or protein molecules, nucleotide, or nucleoside molecules labeled with labels selected from a group comprising fluorescent molecules, or chemiluminescent molecules, or radioisotopes, or enzyme substrates, or biotin molecules, molecule type 1 is also selected from the group comprising any other molecules capable of binding to DNA or RNA end(s) and serving as a target for binding of molecules type 2, or type 3, or any other substrates for enzymes, or any of their analogs, or oligomers, or polymers, or combinations thereof.
14. Method according to clause 1, wherein molecule type 2 and molecule type 3 independently are selected from the group comprising antibody or fragments thereof, streptavidin, avidin, biotin molecules or analogs thereof, ligands, proteins, peptides, nucleic acids, antigens, reactive molecules like azides, oligomers, polymers, and any analogs of the abovementioned or combinations thereof.
15. Method according to clause 1, wherein molecule 4 and molecule 5 independently are selected from the group comprising antibodies covalently linked to nucleic acids (oligonucleotides type 1) of a sequence which allows interaction (hybridisation of selected regions) with other oligonucleotides (oligonucleotides type 2), their ligation, and subsequent RCA reaction.
16. Method according to clause 1, wherein molecule 4 and molecule 5 independently are selected from the group comprising antibody streptavidin, avidin, biotin, streptavidin analog, biotin analog, ligands, proteins, peptides, nucleic acids, antibody fragments, antigens, reactive molecules like azides, oligomers, polymers, analogs of the abovementioned or combinations thereof.
17. Method according to clause 1, wherein molecule type 6 is a labeled oligonucleotide (oligonucleotides type 3) capable of recognizing and binding to products of RCA reaction formed using a selected sequence of ligated oligonucleotide type 2 as a template.
18. Method according to clause 1, wherein molecule type 7 is a molecule capable of recognizing and binding to products of RCA reaction formed using a selected sequence of ligated oligonucleotide type 2 as a template, and capable of serving as a binding target for other molecules that carry or can generate a molecular signature.
19. Method according to clause 1, wherein amplification of sub-step g in step III is improved by addition of the molecules type 7 which bind to molecules type 6.
20. Method according to clause 19, wherein molecule type 7 is labeled with labels selected from a group comprising fluorescent chemiluminescent molecules, radioisotopes, enzyme substrates, biotin, nanoparticles.
21. Method according to clause 1, wherein catalytic mean comprises non-enzymatic or enzymatic mean.
22. Method according to clause 21, wherein the enzymatic mean is selected from the group comprising DNA polimerase I, TdT, Klenow fragment, Phu polymerase, Taq polymerase, T4 DNA Polymerase, T7 DNA Polymerase, T4 Polynucleotide Kinase, RNA polymerases.
23. Method according to clause 21, wherein the non-enzymatic mean is selected from the group comprising chemical factors with catalytic properties.
24. Method according to clause 1, wherein noncatalytic mean comprises physical or biochemical factors.
25. Method according to clause 1, wherein the detection of sub-step h is performed by techniques selected from the group comprising microscopy, methods of automated analysis of high cell numbers, spectroscopy, including fluorescence microscopy (wide field, confocal, multifocal, super-resolution, microscopy with catapulting, laser scanning, high throughput, high content), fluorimetry, transmitted light optical microscopy for absorption detection, flow cytometry, cell sorting (FACS), mass spectrometry.
26. Method according to clause 1, wherein molecules types 2 and 3 are not conjugated with oligonucleotides type 1.
27. Method according to clause 26, wherein contacting suitable molecules type 4 and type 5 with molecule type 2 and 3 is performed in step III sub-step g (i).
28. Method according to clause 1, wherein increasing accessibility of DNA end(s) does not induce DNA damage.
29. Method according to clause 1, wherein detection of single DNA end(s) is obtained.
30. Method according to clause 1, wherein marking the presence and position of single DNA end(s) is acquired.
31. Method according to clause 1, wherein detection of DNA end(s) in a biological material being cells or tissues, comprises the following steps:
   *I. PREPARATION OF THE MATERIAL*
      a. fixation and/or permeabilization and/or isolation and/or immobilization of the biological material,
      b. increasing accessibility of DNA end(s),
   *II. PROCESSING OF DNA END(S)*
      d. modification of DNA end(s) by chemical processing followed by binding molecules type 1 being nucleotides or analogs thereof, to the DNA end(s) by catalytic means,
      e. blocking nonspecific binding site(s) for molecules type 2-6 in the biological material,
   *III. RECOGNITION AND DETECTION OF THE MODIFIED DNA END(S)*
      f. incubation of the biological material from step II with at least two molecules type 2 and 3 being primary antibodies, which bind to the different types of binding sites of molecules type 1 in a manner that allows steps leading to rolling circle amplification (RCA) reactions,
      g. detection of DNA end(s) by:
         i. contacting suitable molecules type 4 and 5 being secondary antibodies, with molecules type 2 and 3, wherein the molecules type 4 and 5 are conjugated with the oligonucleotides type 1,
         ii. adding oligonucleotides type 2 and enzyme ligase to allow hybridization of said added oligonucleotides type 2 to the oligonucleotides type 1 already linked to molecules type 4 and 5 and subsequently performing DNA ligation of oligonucleotides type 2,
         iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
      h. detection of molecules type 6.
32. Method according to clause 1, wherein detection of DNA end(s) in a biological material being cells or tissues, comprises the following steps:
   *I. PREPARATION OF THE MATERIAL*
      a. fixation and/or permeabilization and/or isolation and/or immobilization of the biological material,
      b. increasing accessibility of DNA end(s),
      c. blocking nonspecific binding site(s) for molecules type 2-6 in the biological material,
   *II. PROCESSING OF DNA END(S)*
      d. modification of DNA end(s) by chemical processing followed by binding molecules type 1 being unmodified nucleotides or biotinylated nucleotides, to the DNA end(s) by catalytic means,
      e. blocking nonspecific binding site(s) for molecules type 2-6 in the biological material,
   *III. RECOGNITION AND DETECTION OF THE MODIFIED DNA END(S)*
      f. incubation of the biological material from step II with at least two molecules type 2 and 3 being primary antibodies which bind to the different types of binding sites of molecules type 1 in a manner that allows steps leading to rolling circle amplification (RCA) reactions,
      g. detection of DNA end(s) by:
         i. contacting suitable molecules type 4 and 5 being secondary antibodies, with molecules type 2 and 3, wherein molecules type 4 and 5 are conjugated with the oligonucleotides type 1,
         ii. adding oligonucleotides type 2 and enzyme ligase to allow hybridization of said added oligonucleotides type 2 to the oligonucleotides type 1 already linked to molecules type 4 and 5 and subsequently performing DNA ligation of oligonucleotides type 2
         iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
      h. detection of molecules type 6.
33. Method according to clause 1, wherein detection of DNA end(s) in a biological material being cells or tissues, comprising the following steps:
   *I. PREPARATION OF THE MATERIAL*
      a. fixation and/or permeabilization and/or isolation and/or immobilization of the biological material,
      b. increasing accessibility of DNA end(s),
      c. blocking nonspecific binding site(s) for molecules type 2-4 and 6 in the biological material,
   *II. PROCESSING OF DNA END(S)*
      d. modification of DNA end(s) by chemical processing followed by binding molecules type 1 being unmodified nucleotides or biotinylated nucleotides, to the DNA end(s) by catalytic means,
      e. blocking nonspecific binding site(s) for molecules type 2-4 and 6 in the biological material,
   *III. RECOGNITION AND DETECTION OF THE MODIFIED DNA END(S)*
      f. incubation of the biological material from step II with at least two molecules: molecule type 2 being primary antibody and molecule type 3 being streptavidin conjugated with oligonucleotides type 1, which bind to the molecules type 1 in a manner that allows steps leading to rolling circle amplification (RCA) reactions,
      g. detection of DNA end(s) by:
         i. optionally contacting suitable molecule type 4 being secondary antibody with molecule type 2, wherein the molecule type 4 is conjugated with the oligonucleotides type 1,
         ii. adding oligonucleotides type 2 and enzyme ligase to allow hybridization of said added oligonucleotides type 2 to the oligonucleotides type 1 already linked to molecules type 2 and 3, or to 3 and 4 if oligonucleotide type 1 is not conjugated with the molecule 2, and subsequently performing DNA ligation of oligonucleotides type 2,
         iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
      h. detection of molecules type 6.
34. Use of rolling circle replication for marking the presence and position of single DNA end(s).
35. Use of the following steps:
   - contacting suitable molecules type 4 and/or 5 with molecules type 2 and 3, wherein the molecules type 4 and/or 5 are conjugated with the oligonucleotides type 1,
   - adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2,
   - performing amplification by adding enzyme polymerase and a solution of nucleotides to allow rolling circle amplification (RCA) reactions, and molecules type 6 to allow subsequent hybridization of molecules type 6 to thus obtained product of RCA reactions,
   for detection of DNA end(s) in properly prepared biological material.
36. Use according to clause 35, wherein molecules types 2 and 3 are not conjugated with oligonucleotides type 1.
37. Use according to clause 36, wherein contacting suitable molecules type 4 and type 5 with molecule type 2 and 3 is performed.
38. Use according to clause 35, wherein the biological material is prepared by the following steps:
   - optionally, fixation and/or permeabilization and/or isolation and/or immobilization of the biological material,
   - increasing accessibility of DNA end(s),
   - optionally blocking nonspecific binding site(s) for molecules type 2-6 in the biological material
39. Use of a method comprising the following steps I - III and at least one of sub-steps a-h of each of steps I - III:
   *I. PREPARATION OF THE MATERIAL*
      a. fixation and/or permeabilization and/or lysis and/or isolation and/or fractionation and/or immobilization of the biological material,
      b. increasing accessibility of DNA end(s),
      c. blocking nonspecific binding site(s) for molecules type 2-6, in the biological material,
   *II. PROCESSING OF DNA END(S)*
      d. modification of DNA end(s) by chemical or physical processing followed by binding molecules type 1 to the DNA end(s) by catalytic or noncatalytic means,
      e. blocking nonspecific binding site(s) for molecules type 2-6 in the biological material,
   *III. RECOGNITION AND DETECTION OF THE MODIFIED DNA END(S)*
      f. incubation of the biological material from step II with at least two molecules type 2 and 3 which bind to the molecules type 1 in a manner that allows steps leading to rolling circle amplification (RCA) reactions,
      g. detection of DNA end(s) by:
         i. optionally contacting suitable molecules type 4 and/or 5 with molecules type 2 and 3, wherein the molecules type 4 and/or 5 are conjugated with the oligonucleotides type 1,
         ii. adding oligonucleotides type 2 and enzyme ligase to allow hybridization of said added oligonucleotides type 2 to the oligonucleotides type 1 already linked to molecules type 4 and/or 5, or to molecules type 2 and 3 if they are linked to oligonucleotides type 1, and subsequently performing DNA ligation of oligonucleotides type 2,
         iii. performing amplification by adding enzyme polymerase and a solution of nucleotides to allow rolling circle amplification (RCA) reactions, and molecules type 6 to allow subsequent hybridization of molecules type 6 to thus obtained product of RCA reactions,
      h. detection of molecules type 6,
         wherein when more than one sub-step a - c from step I is performed then they may occur in any order,
         for detection of DNA end(s) in a biological material.
40. Use according to clause 39, wherein DNA end(s) are the result of any DNA break(s) including single strand break or double strand break.
41. Use according to clause 40, wherein DNA break(s) are selected from the group comprising single-strand nick, single-strand gap, single-strand break of single phosphodiester bond, double-strand blunt-end break, double-strand 3'-protruding break, double-strand 5'-protruding break, types of DNA strand breaks including these where 3'-OH or 5'-phosphate DNA ends are not present.
42. Use according to clause 39, wherein detection of DNA end(s) is performed *in situ.*
43. Use according to clause 39, wherein the biological material is live.
44. Use according to clause 39, wherein the biological material is fixed.
45. Use according to clause 43 or 44, wherein the biological material is selected from the group comprising animal, plant, protozoan, bacterial cells, viruses, tissues and fragments and/or components thereof.
46. Use according to clause 43 or 44, wherein the biological material is cell or tissue or fragments thereof surrounded by membrane.
47. Use according to clause 46, wherein sub-step c is performed in step I.
48. Use according to clause 43, wherein before step I sub-step a, an additional step of increasing accessibility of DNA end(s) is performed.
49. Use according to clause 39, wherein the biological material is present in a solution or on a porous surface or solid support.
50. Use according to clause 39, wherein solid supports or types of porous surface are selected from the group comprising glass, plastic, water gels, aero gels metals and ceramics.
51. Use according to clause 39, wherein molecule type 1 is selected from the group comprising halogenated nucleotide or nucleoside molecules such as BrdU, IdU, CIdU, or DNA precursor analogs such as EdU (5-Ethynyl-2'-deoxyuridine), F-ara-EdU, 5-Ethynyl-2'-deoxycytidine, or biotinylated nucleotide molecules, ADP-ribose molecules, or protein molecules, nucleotide, or nucleoside molecules labeled with labels selected from a group comprising fluorescent molecules, or chemiluminescent molecules, or radioisotopes, or enzyme substrates, or biotin molecules, molecule type 1 is also selected from the group comprising any other molecules capable of binding to DNA or RNA end(s) and serving as a target for binding of molecules type 2, or type 3, or any other substrates for enzymes, or any of their analogs, or oligomers, or polymers, or combinations thereof.
52. Use according to clause 39, wherein molecule type 2 and molecule type 3 independently are selected from the group comprising antibody or fragments thereof, streptavidin, avidin, biotin molecules or analogs thereof, ligands, proteins, peptides, nucleic acids, antigens, reactive molecules like azides, oligomers, polymers, and any analogs of the abovementioned or combinations thereof.
53. Use according to clause 39 wherein molecule 4 and molecule 5 independently are selected from the group comprising antibodies covalently linked to nucleic acids (oligonucleotides type 1) of a sequence which allows interaction (hybridisation of selected regions) with other oligonucleotides (oligonucleotides type 2), their ligation, and subsequent RCA reaction.
54. Use according to clause 39 or 53, wherein molecule 4 and molecule 5 independently are selected from the group comprising antibody streptavidin, avidin, biotin, streptavidin analog, biotin analog, ligands, proteins, peptides, nucleic acids, antibody fragments, antigens, reactive molecules like azides, oligomers, polymers, analogs of the abovementioned or combinations thereof.
55. Use according to clause 39, wherein molecule type 6 is a labeled oligonucleotide (oligonucleotides type 3) capable of recognizing and binding to products of RCA reaction formed using a selected sequence of ligated oligonucleotide type 2 as a template.
56. Use according to clause 39, wherein molecule type 7 is a molecule capable of recognizing and binding to products of RCA reaction formed using a selected sequence of ligated oligonucleotide type 2 as a template, and capable of serving as a binding target for other molecules that carry or can generate a molecular signature.
57. Use according to clause 39, wherein amplification of sub-step g in step III is improved by addition of the molecules type 7 which bind to molecules type 6.
58. Use according to clause 57, wherein molecule type 7 is labeled with labels selected from a group comprising fluorescent chemiluminescent molecules, radioisotopes, enzyme substrates, biotin, nanoparticles.
59. Use according to clause 39, wherein catalytic mean comprises non-enzymatic or enzymatic mean.
60. Use according to clause 59, wherein the enzymatic mean is selected from the group comprising DNA polimerase I, TdT, Klenow fragment, Phu polymerase, Taq polymerase, T4 DNA Polymerase, T7 DNA Polymerase, T4 Polynucleotide Kinase, RNA polymerases.
61. Use according to clause 59, wherein the non-enzymatic mean is selected from the group comprising chemical factors with catalytic properties.
62. Use according to clause 39, wherein noncatalytic mean comprises physical or biochemical factors.
63. Use according to clause 39, wherein the detection of sub-step h is performed by techniques selected from the group comprising microscopy, methods of automated analysis of high cell numbers, spectroscopy, including fluorescence microscopy (wide field, confocal, multifocal, super-resolution, microscopy with catapulting, laser scanning, high throughput, high content), fluorimetry, transmitted light optical microscopy for absorption detection, flow cytometry, cell sorting (FACS), mass spectrometry.
64. Use according to clause 39, wherein molecules types 2 and 3 are not conjugated with oligonucleotides type 1.
65. Use according to clause 64, wherein contacting suitable molecules type 4 and type 5 with molecule type 2 and 3 is performed in step III sub-step g (i).
66. Use according to clause 39, wherein increasing accessibility of DNA end(s) does not induce DNA damage.
67. Use according to clause 39, wherein detection of single DNA end(s) is obtained.
68. Use according to clause 39, wherein marking the presence and position of single DNA end(s) is acquired.

## Claims

1. A method of detection of DNA end(s) in a biological material, comprising the steps:
i. preparation of the material;
ii. processing of DNA end(s) by binding of molecules to the DNA end(s) by catalytic or noncatalytic means *[molecules type 1];*
iii. addition of binding molecules *[molecules type 2*/*3]* that bind to the molecules bound to the DNA end(s) *[molecules type 1]* and which are linked to oligonucleotides *[oligonucleotides type 1];*
iv. addition of oligonucleotides *[oligonucleotides type 2]* that hybridise with the linked oligonucleotides *[oligonucleotide type 1]* to create ligatable ends that are ligated to form a circular oligonucleotide hybridised to the linked oligonucleotides;
v. performing amplification; and
vi. detection of amplification products to detect the DNA end(s).

2. The method of claim 1, wherein the binding molecules *[molecules type 2*/*3]* that bind to the molecules bound to the DNA end(s) are linked to the oligonucleotides *[oligonucleotide type 1]* via a binding interaction with further binding molecules *[molecules type 4*/*5]* that are conjugated to the oligonucleotides *[oligonucleotides type 1].*

3. The method of claim 1 or claim 2, wherein the amplification comprises rolling circle amplification.

4. The method of any one of claims 1-3, wherein the preparation of the material comprises:
(a) fixation and/or permeabilization and/or lysis and/or isolation and/or fractionation and/or immobilization of the biological material; and/or
(b) increasing accessibility of the DNA end(s); and/or
(c) blocking nonspecific binding site(s) for binding molecules.

5. The method of any one of claims 1-4, wherein the processing of the DNA end(s) is followed by blocking nonspecific binding site(s) for binding molecules.

6. The method of any one of claims 1-5, wherein the detection of the amplification products comprises detection of labelled oligonucleotides *[molecules type 6]* that hybridise with the amplification products.

7. The method of any one of claims 1-6, wherein DNA end(s) are:
(a) the result of any DNA break(s) including single-strand break or double-strand break; and/or
(b) selected from the group comprising single-strand nick, single-strand gap, single-strand break of single phosphodiester bond, double-strand blunt-end break, double-strand 3'-protruding break, double-strand 5'-protruding break, types of DNA strand breaks including these where 3'-OH or 5'-phosphate DNA ends are not present; and/or
(c) detected *in situ.*

8. The method of any one of claims 1-7, wherein the biological material is:
(a) live or fixed; and/or
(b) selected from the group comprising animal, plant, protozoan, bacterial cells, viruses, tissues and fragments and/or components thereof; and/or
(c) cell or tissue or fragments thereof surrounded by a membrane; and/or
(d) present in a solution or on a porous surface or solid support, optionally wherein solid supports or types of a porous surface are selected from the group comprising glass, plastic, water gels, aerogels, metals and ceramics.

9. The method of any one of claims 1-8, wherein the molecules bound to the DNA end(s) *[molecule type 1]* are selected from the group comprising halogenated nucleotide or nucleoside molecules such as BrdU, IdU, CIdU, or DNA precursor analogs such as EdU (5-Ethynyl-2'-deoxyuridine), F-ara-EdU, 5-Ethynyl-2'-deoxycytidine, or biotinylated nucleotide molecules, ADP-ribose molecules, or protein molecules, nucleotide, or nucleoside molecules labeled with labels selected from a group comprising fluorescent molecules, or chemiluminescent molecules, or radioisotopes, or enzyme substrates, or biotin molecules, or the molecules bound to the DNA end(s) *[molecule type 1]* are also selected from the group comprising any other molecules capable of binding to DNA or RNA end(s) and serving as a target for binding of the binding molecules *[molecules type 2*/*3],* or any other substrates for enzymes, or any of their analogs, or oligomers, or polymers, or combinations thereof.

10. The method of any one of claims 1-9, wherein the binding molecules *[molecules type 2*/*3]* are independently selected from the group comprising antibody or fragments thereof, streptavidin, avidin, biotin molecules or analogs thereof, ligands, proteins, peptides, nucleic acids, antigens, reactive molecules like azides, oligomers, polymers, and any analogs of the abovementioned or combinations thereof.

11. The method of any one of claims 2-10, wherein the further binding molecules *[molecules type 4*/*5]* are independently selected from the group comprising antibody streptavidin, avidin, biotin, streptavidin analog, biotin analog, ligands, proteins, peptides, nucleic acids, antibody fragments, antigens, reactive molecules like azides, oligomers, polymers, analogs of the abovementioned or combinations thereof.

12. The method of any one of claim 1-11, wherein a single DNA end is detected or the presence and position of a single DNA end is determined.

13. A set of reagents for detection of DNA end(s) in a biological material according to claim 1 comprising:
i. preparation reagents for preparing the biological material
ii. molecules that can be bound to DNA end(s) by catalytic or non-catalytic means *[molecules type 1];*
iii. binding molecules *[molecules type 2*/*3]* that bind to the molecules bound to the DNA end(s) *[molecules type 1]* and which are linked to oligonucleotides *[oligonucleotides type 1];*
iv. oligonucleotides *[oligonucleotides type 2]* that hybridise with the linked oligonucleotides *[oligonucleotide type 1]* to create ligatable ends that are ligated to form a circular oligonucleotide hybridised to the linked oligonucleotides; and
v. amplification reagents.

14. A method of detection of DNA end(s) in a biological material according to claim 1, comprising the steps:
i. increasing accessibility of the DNA end(s);
ii. processing of the DNA end(s) by binding of molecules comprising a nucleotide or nucleoside, or a precursor thereto, to the DNA end(s) *[molecules type 1];*
iii. addition of two different binding molecules *[molecules type 2*/*3]* that bind to the molecules comprising the nucleotide or nucleoside bound to the DNA end(s) *[molecules type 1],* wherein the two different binding molecules have different affinity or recognise different binding sites in the molecules bound to the DNA end(s);
iv. addition of two different further binding molecules *[molecules type 4*/*5]* that each bind to one of the two different binding molecules and are conjugated to oligonucleotides *[oligonucleotides type 1];*
v. addition of oligonucleotides *[oligonucleotides type 2]* that hybridise with the conjugated oligonucleotides *[oligonucleotide type 1]* to create ligatable ends that are ligated to form a circular oligonucleotide hybridised to the linked oligonucleotides;
vi. performing amplification; and
vii. detection of amplification products to detect the DNA end(s).

15. A set of reagents for detection of DNA end(s) in a biological material according to claim 13 comprising:
i. reagents for increasing accessibility of the DNA end(s);
ii. molecules comprising a nucleotide or nucleoside that can be bound to DNA end(s) *[molecules type 1];*
iii. two different binding molecules *[molecules type 2*/*3]* that bind to the molecules comprising the nucleotide or nucleoside bound to the DNA end(s) *[molecules type 1],* wherein the two different binding molecules have different affinity or recognise different binding sites in the molecules bound to the DNA end(s);
iv. two different further binding molecules *[molecules type 4*/*5]* that each bind to one of the two different binding molecules and are conjugated to oligonucleotides *[oligonucleotides type 1];*
v. oligonucleotides *[oligonucleotides type 2]* that hybridise with the conjugated oligonucleotides *[oligonucleotide type 1]* to create ligatable ends that are ligated to form a circular oligonucleotide hybridised to the linked oligonucleotides; and
vi. amplification reagents.
